# EUROPEAN PATENT APPLICATION

(11) **EP 0 275 480 A2**
(43) Date of publication of application: **27.07.1988**
(21) Application number: 87118523.7
(22) Date of filing: 14.12.1987
(51) Int. Cl.: C07K 5/02, C07C 157/12, C07C 157/14, C07C 129/12, C07C 127/15, C07C 103/50, C07D 207/327, C07D 233/64, C07D 235/16, C07D 295/18, C07D 303/48

(54) **Renin inhibitors IV**

(30) Priority: 23.12.1986 US 945582; 02.11.1987 US 113278
(71) Applicant: WARNER-LAMBERT COMPANY, Morris Plains New Jersey 07950 (US)
(72) Inventor: Hudspeth, James Perry, Malibu California 90265 (US); Kaltenbronn, James Stanley, Ann Arbor Michigan 48105 (US); Repine, Joseph Thomas, Ann Arbor Michigan 48103 (US); Roark, William Howard, Ann Arbor Michigan 48104 (US); Tinney, Francis John, Ann Arbor Michigan 48105 (US)
(74) Representative: Mansmann, Ivo

(57) **Abstract**

The invention concerns novel renin-inhibitory peptides which are useful for treating renin-associated hypertension, hyperaldosteronism, and congestive heart failure. Processes for preparing the peptides, compositions containing them and methods of using them are included. Also included is a diagnostic method which uses the compounds to determine the presence of renin-associated hypertension or hyperaldosteronism.

## Description

Renin is a natural enzyme which is released into the blood from the kidney. It cleaves its natural substrate, angiotensinogen, releasing decapeptide, angiotensin I. This is in turn cleaved by converting enzyme in the lung, kidney and other tissues to an octapeptide, angiotensin II. Angiotensin II raises blood pressure both directly by causing arteriolar constriction and indirectly by stimulating release of the sodium- retaining hormone aldosterone from the adrenal gland causing a rise in extracellular fluid volume. Inhibitors of renins have been sought as an agent for control of hypertension and hyperaldosteronism.

The present invention concerns novel peptides which inhibit renin. It also concerns pharmaceutical compositions containing these novel peptides, methods of treating renin-associated hypertension, congestive heart failure, and hyperaidosteronism, as well as the use of the peptides as diagnostic tools, and the methods for preparing the peptides.

United States 4,479,941 covers certain renin-inhibitory peptides of the formula

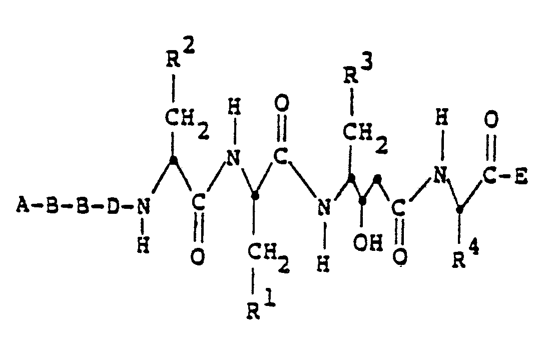

European Application No. 85/308759 covers certain renin-inhibitory dipeptides of the formula 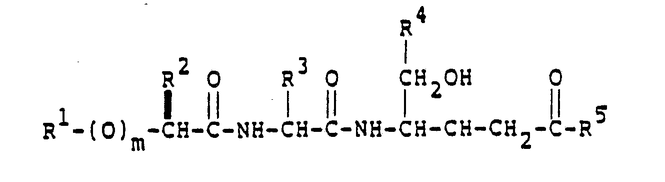 wherein m is 0 or 1 and R¹-R⁵ are a variety of organic groups.

European Application No. 184,855 covers renin-inhibitory peptides of the formula 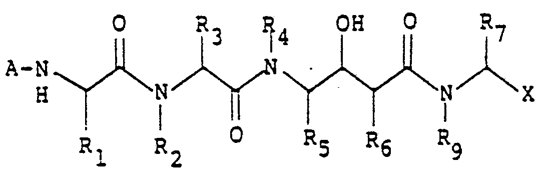 wherein A is an N-protecting group; Ri, R₃, R₅ and R₇ are lower alkyl or lipophilic or aromatic amino acid side chains and may be the same or different; R₂, R₄ and R₆ are hydrogen or lower alkyl and may be the same or different; X is hydrogen, lower alkyl or -CH₂-OR₈, wherein R₈ is hydrogen, lower alkyl or alkaryl; and Rₛ is lower alkyl, hydroxy, hydroxyalkyl, alkoxy, allyl, alkaryloxy or thioalkyl and pharmaceutically acceptable salts thereof.

The present invention relates to novel peptides of the formula 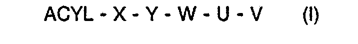 and the pharmaceutically acceptable acid addition salts thereof wherein ACYL, X, Y, W, U, and V are as defined herein below.

The invention also includes pharmaceutical compositions comprising an effective amount of the above peptide of formula I in admixture with a pharmaceutically acceptable carrier or excipient and a method for treating renin-associated hypertension in a patient suffering therefrom comprising administering to said patient the above pharmaceutical composition in unit dosage form.

Further the invention includes a pharmaceutical composition comprising an effective amount of a peptide of formula I above in admixture with a pharmaceutically acceptable carrier or excipient, and a method for treating hyperaldosteronism in a patient suffering therefrom comprising administering to said patient the above pharmaceutical composition in unit dosage form.

The invention also includes a pharmaceutical composition comprising an effective amount of a peptide of formula I above in admixture with a pharmaceutically acceptable carrier or excipient, and a method for treating congestive heart failure in a patient suffering therefrom comprising administering to said patient the above pharmaceutical composition in unit dosage form.

The present invention also includes the use of peptides of formula I above as diagnostic tools for the identification of cases of hypertension due to renin excess.

The invention further includes methods for preparing peptides of formula I above.

The following table provides a dictionary of the terms used in the description of the invention.

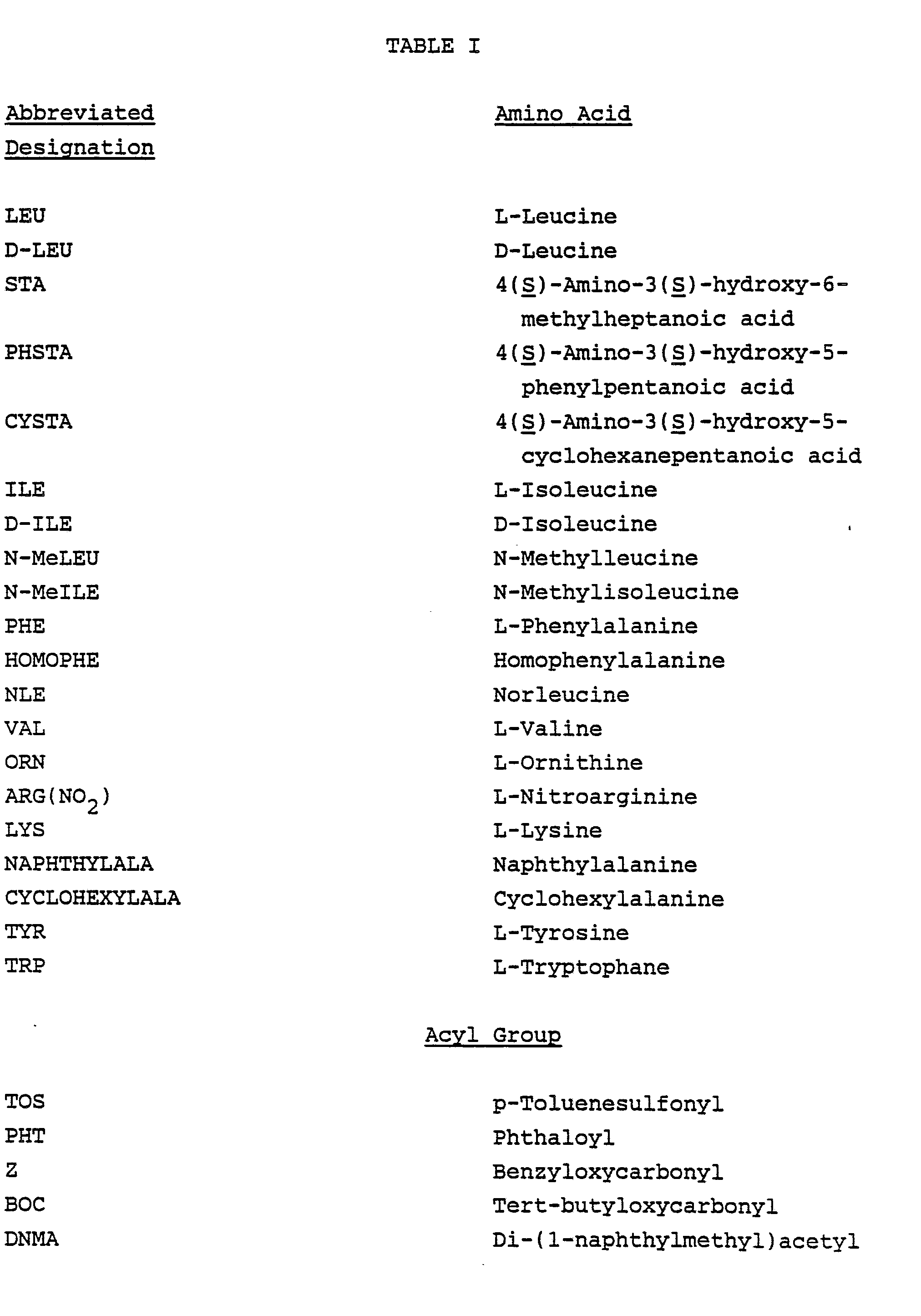

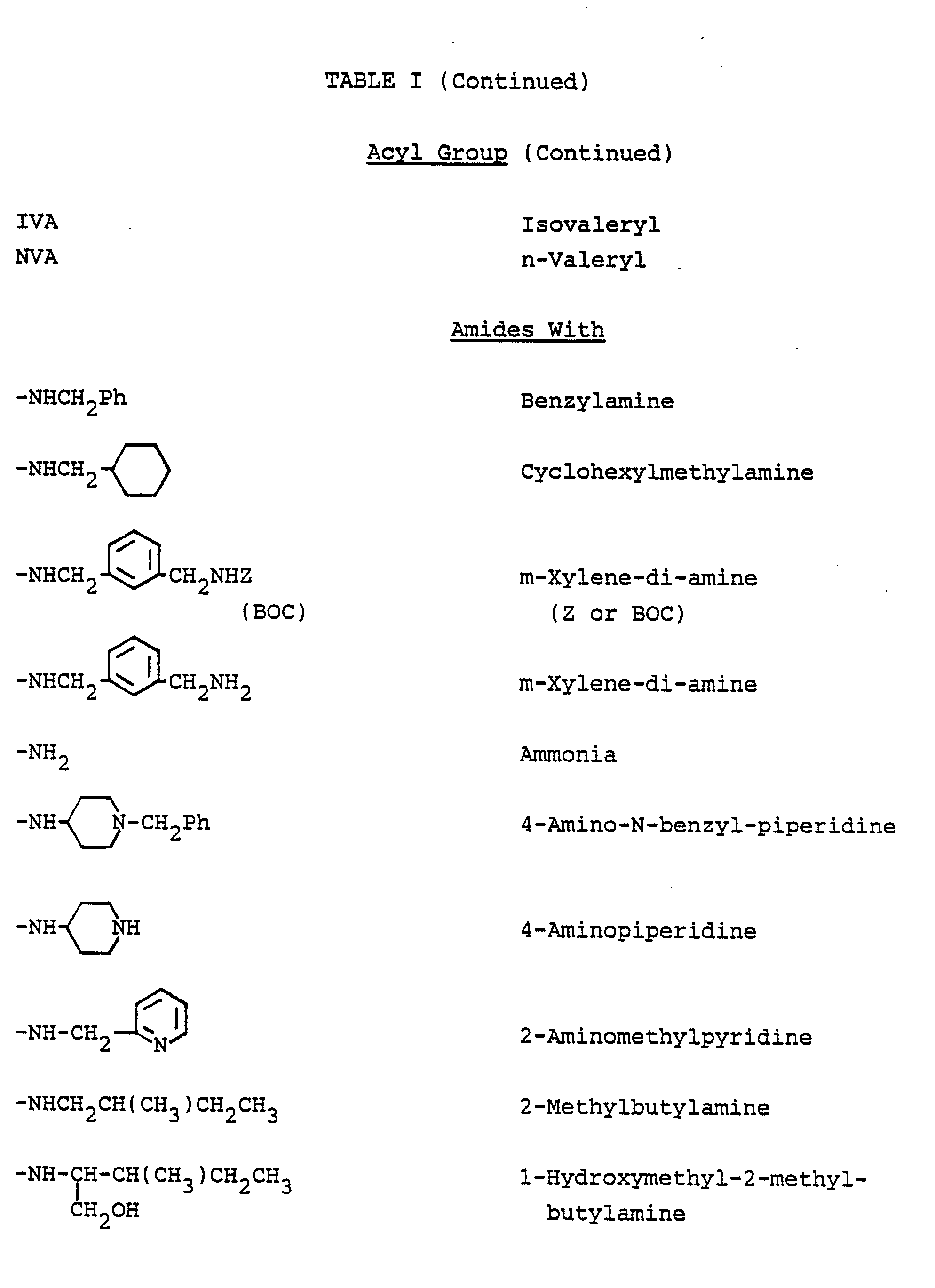

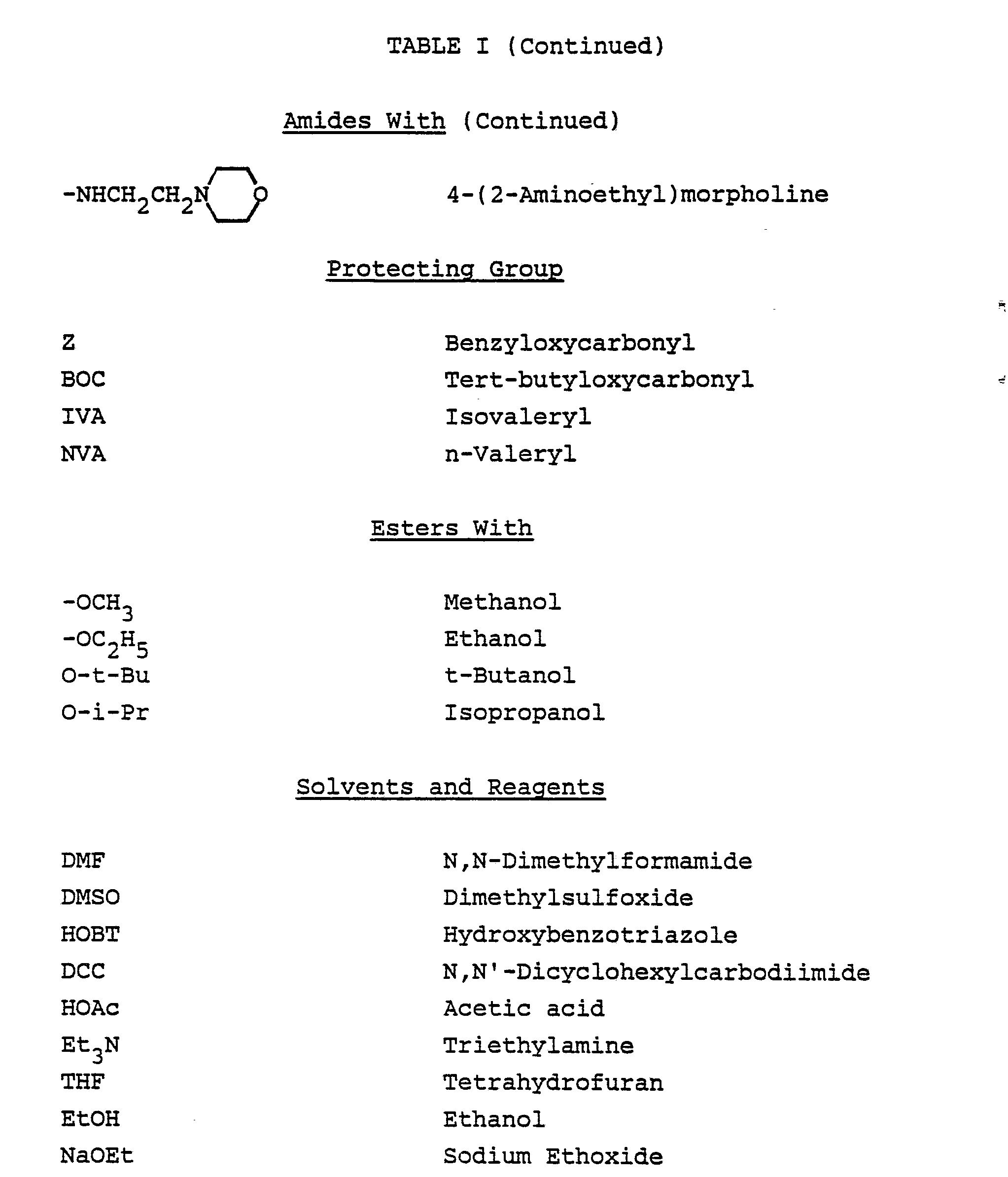

The peptides of the present invention are represented by the formula 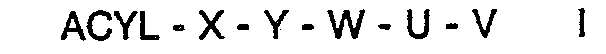 or a pharmaceutically acceptable salt thereof, wherein

ACYL is BOC, IVA, NVA, DNMA, Z, or 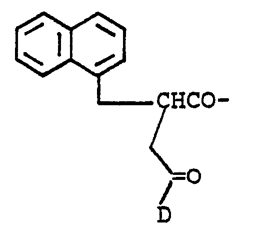 wherein D is 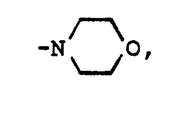 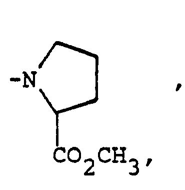 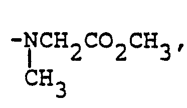 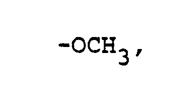 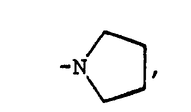 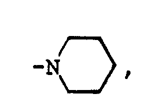 or 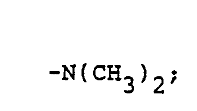 X is absent, PHE, HOMOPHE, NAPHTHYLALA, CYCLOHEXYLALA, TYR, or TRP, with the proviso that when ACYL is DNMA or 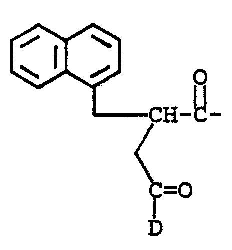 wherein D is as above, X is absent; Y is 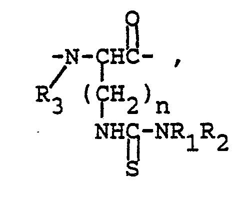 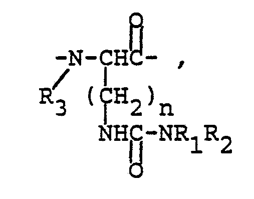 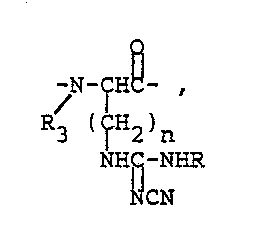 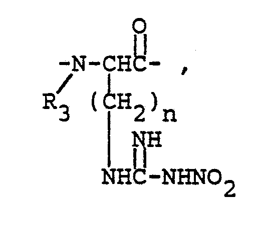 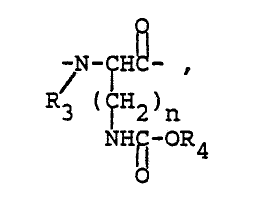 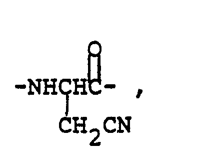 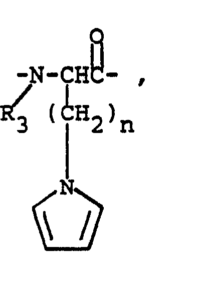 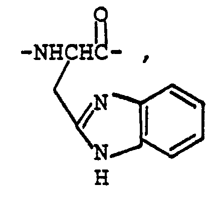 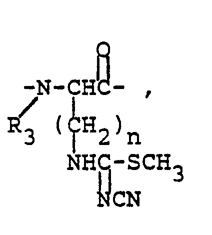 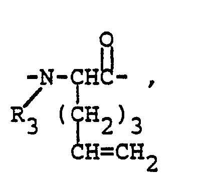 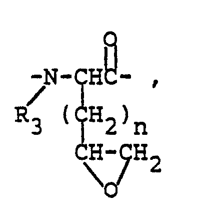 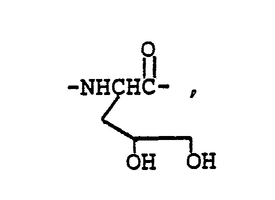 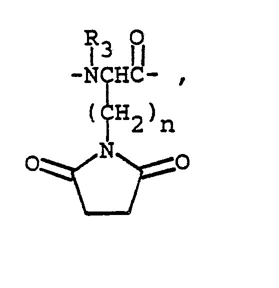 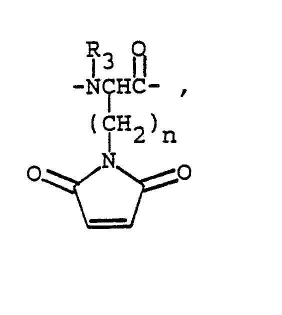 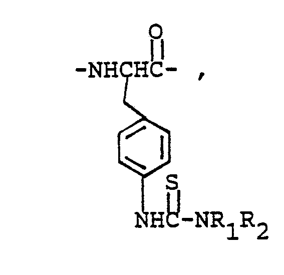 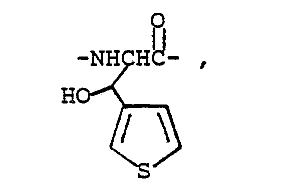 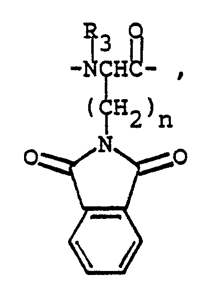 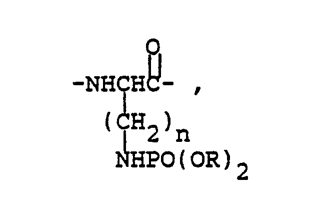 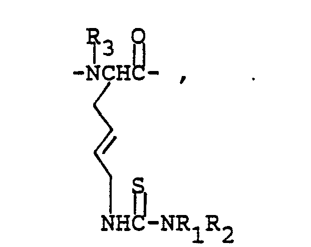 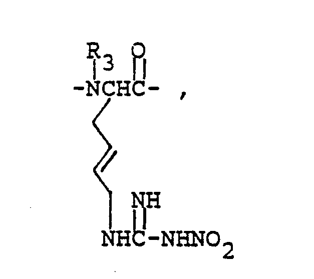 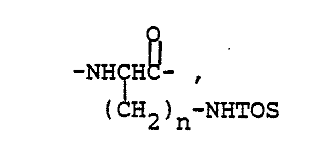 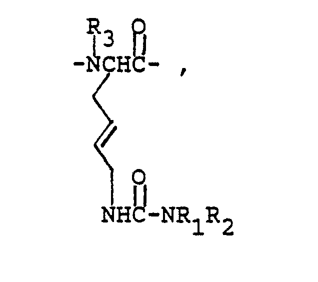 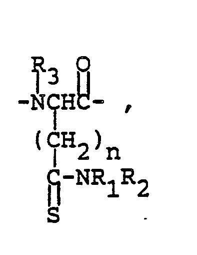 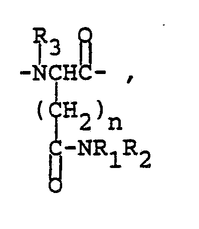 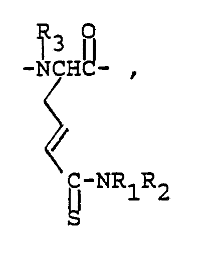 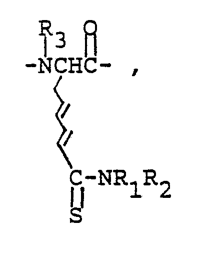 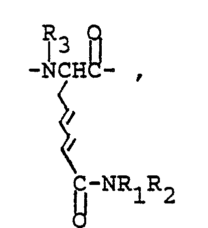 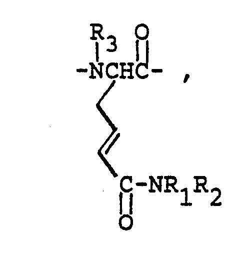 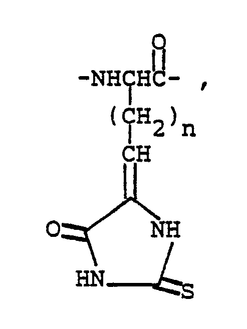 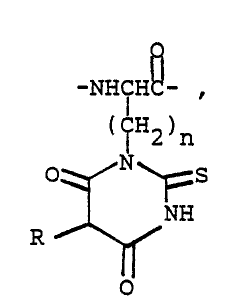 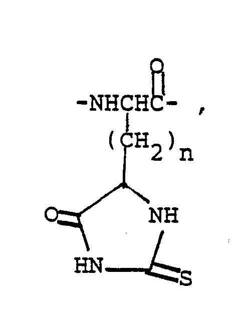 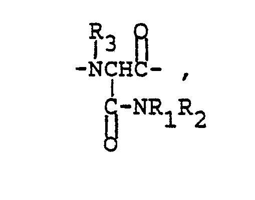 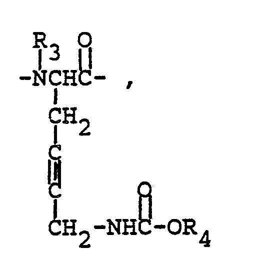 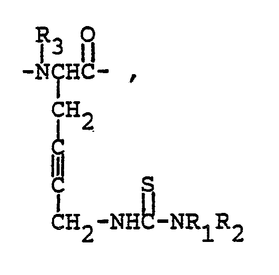 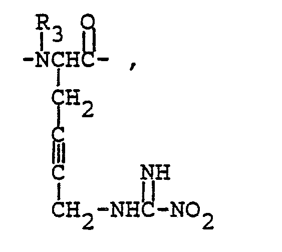 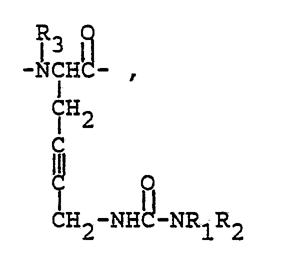 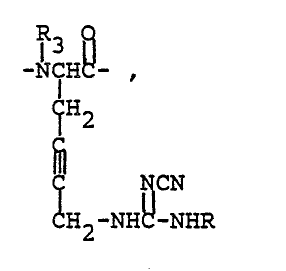 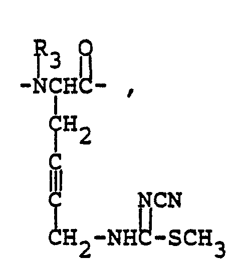 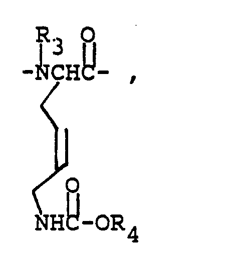 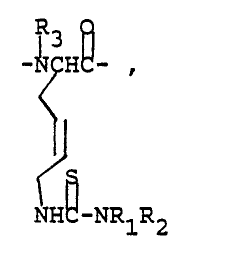 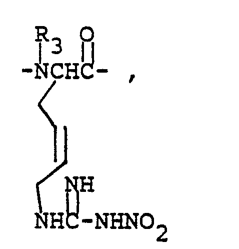 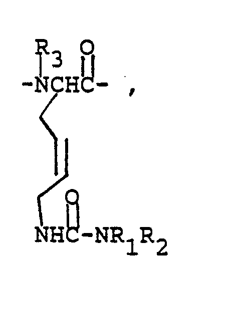 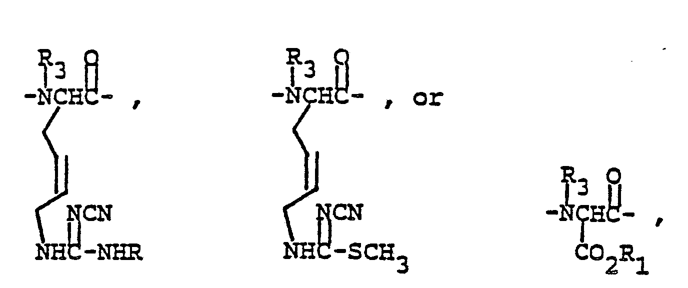 wherein n is an integer from 2 to 8, R is hydrogen or an alkyl from 1 to 3 carbon atoms,
R₁ and R₂ are each independently hydrogen, lower alkyl of from 1 to 6 carbon atoms, aryl or aralkyl, where aryl has the meaning of a mono or bicyclic aromatic ring, especially phenyl or naphthyl, which may be unsubstituted or substituted by halogen, trifluoromethyl, hydroxy, alkoxy or alkyl wherein alkyl is as defined above, R₃ is hydrogen or methyl, and R₄ is lower alkyl of from 1 to 6 carbon atoms or benzyl. W is STA, PHSTA, or CYSTA;
U is absent, LEU, ILE, VAL, N-MeLEU, N-MeILE; and v is 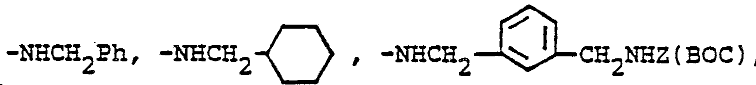 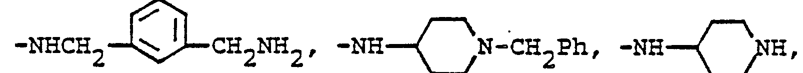 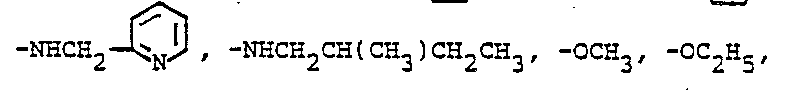 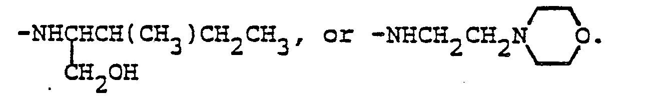
Preferred compounds of the present invention are represented by formula I wherein ACYL is BOC, IVA, DNMA, or

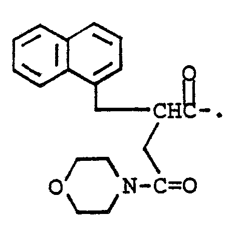

Other preferred compounds are according to formula I wherein V is 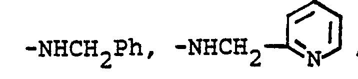 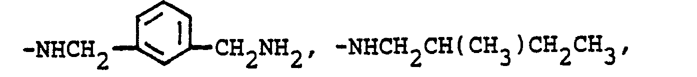 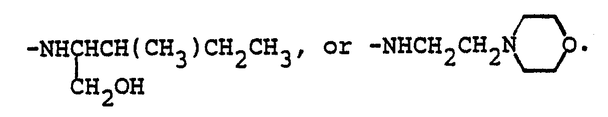 Particularly valuable compounds falling within the scope of the invention include the following:

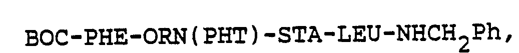

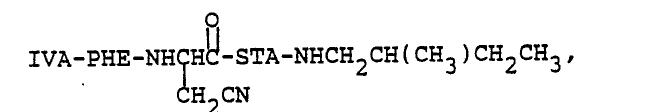

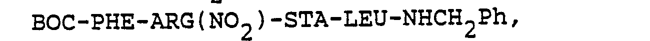

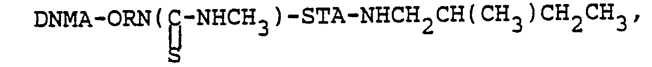

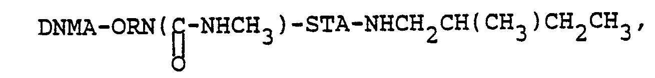

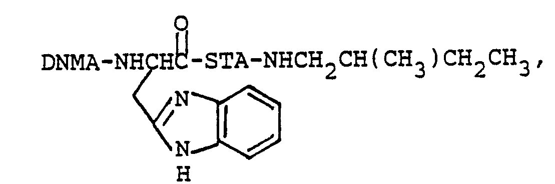

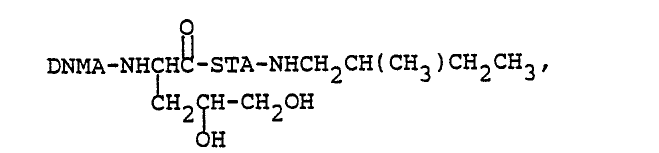

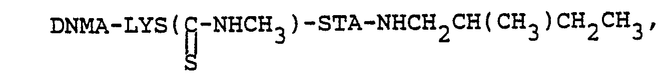

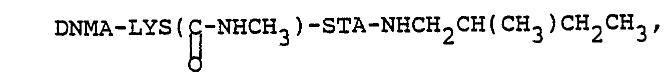

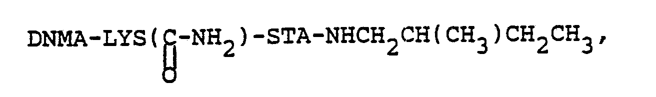

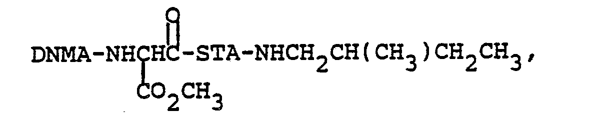

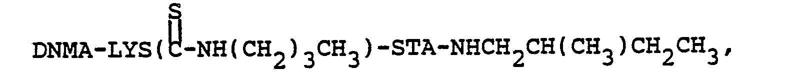

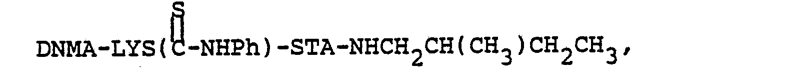

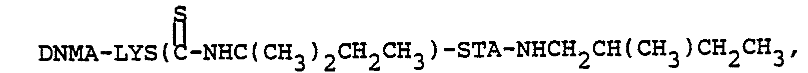

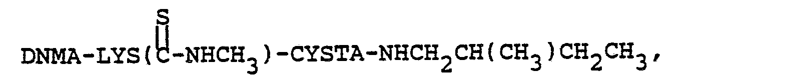

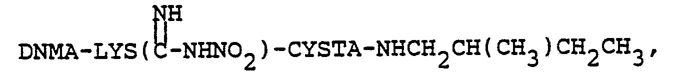

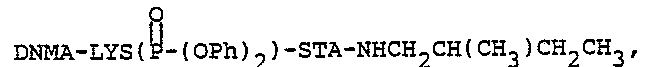

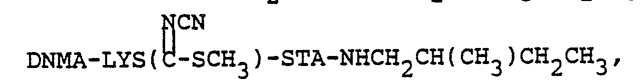

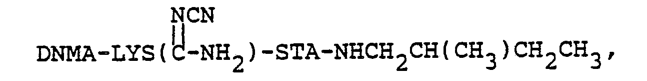

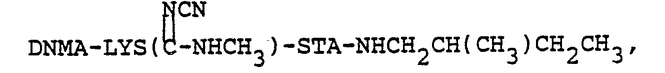

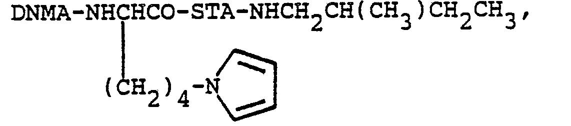

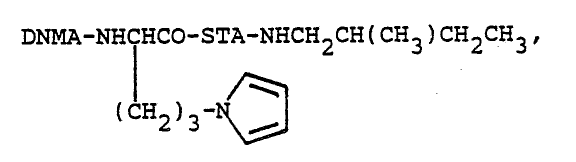

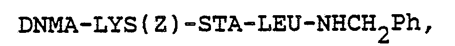

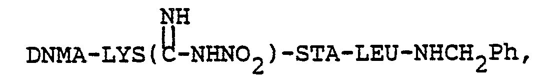

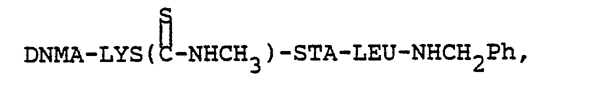

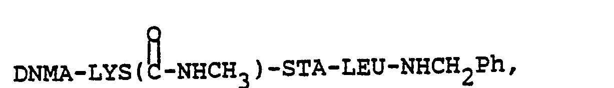

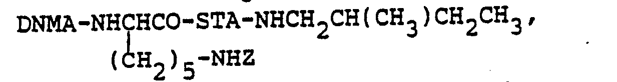

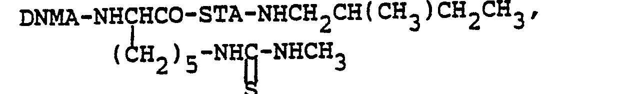

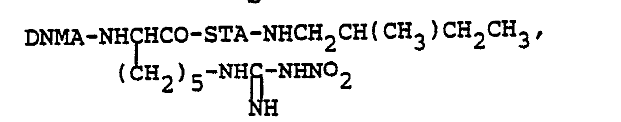

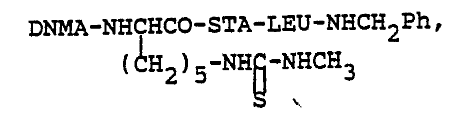

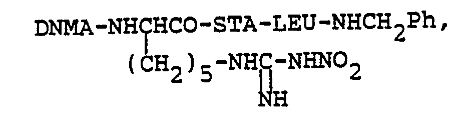

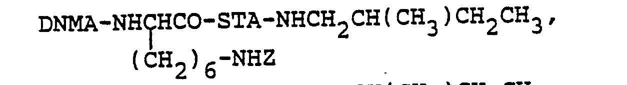

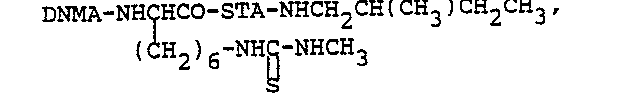

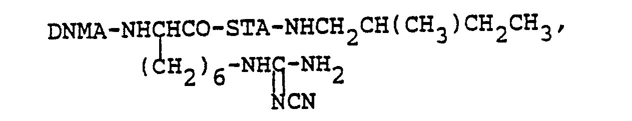

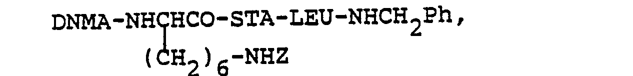

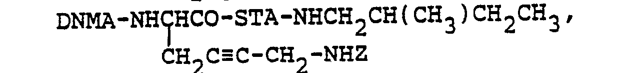

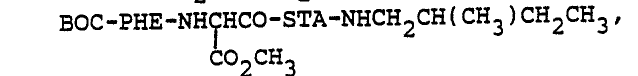

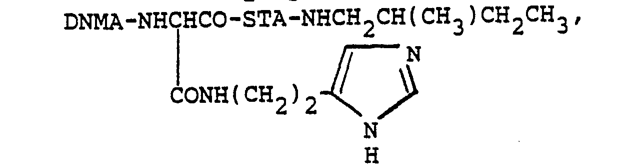

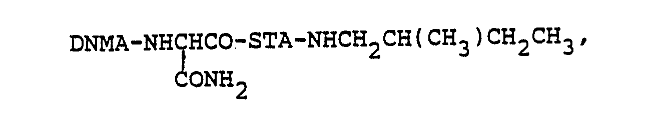

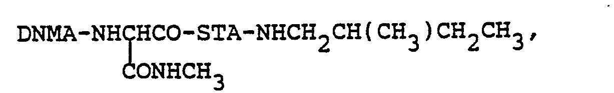

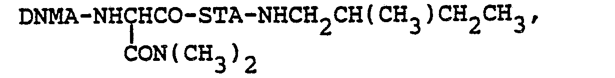

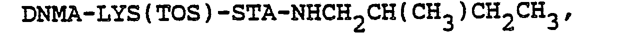

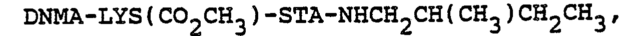

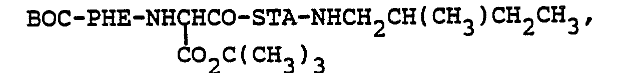

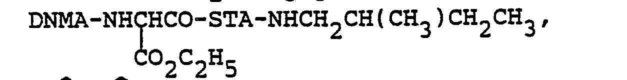

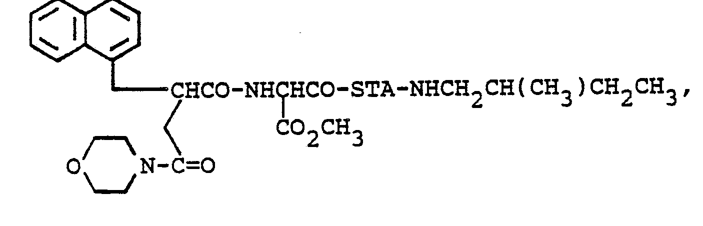

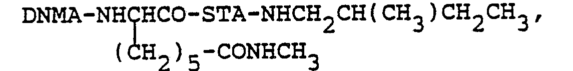

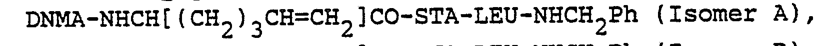

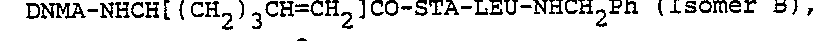

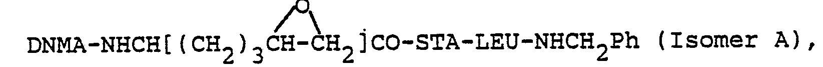

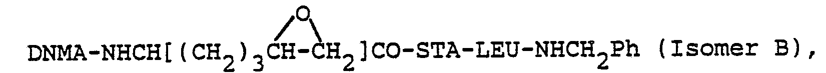

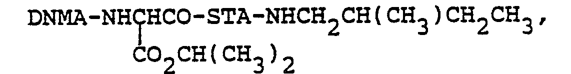

Other valuable compounds falling within the scope of the invention are: 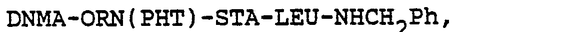 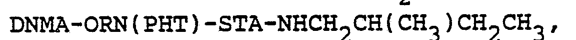 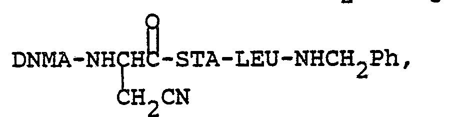 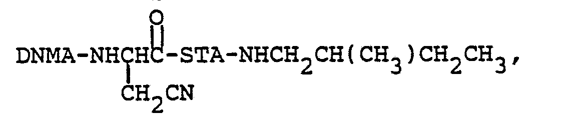 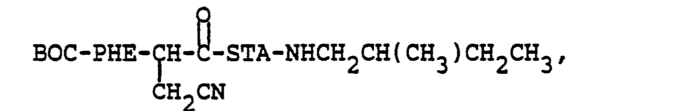 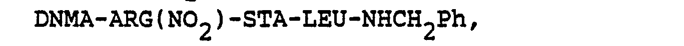 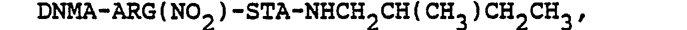 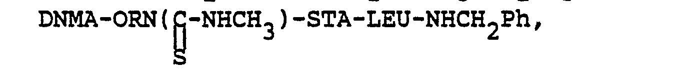 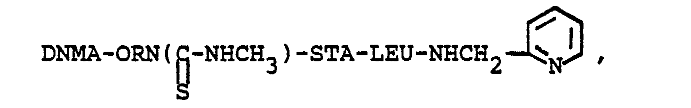 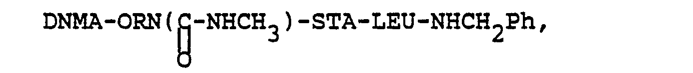 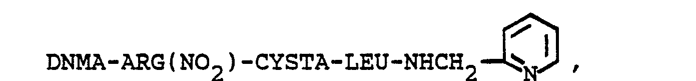 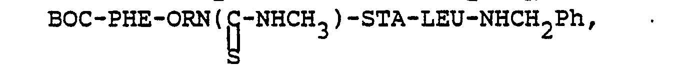 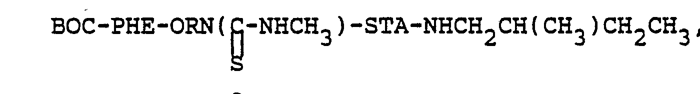 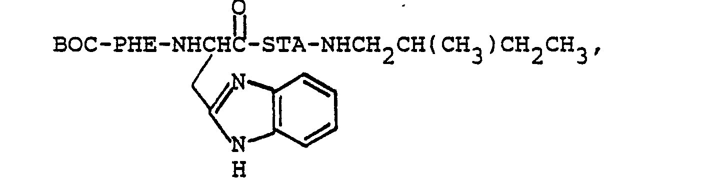 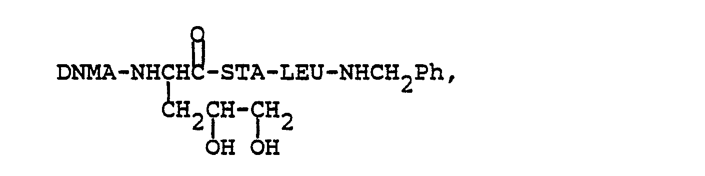 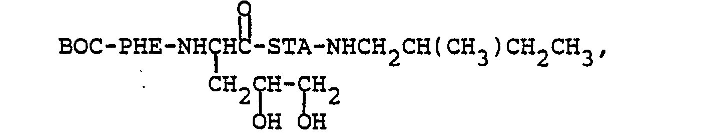 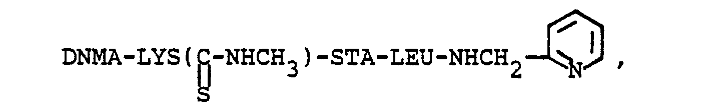 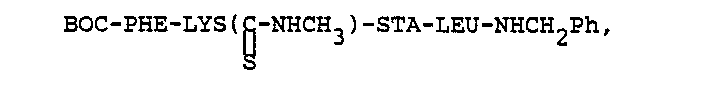 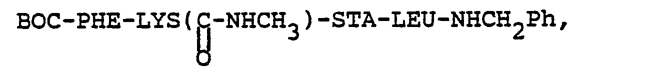 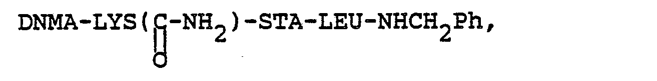 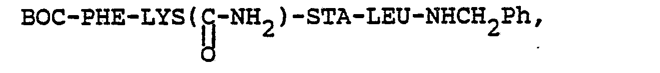 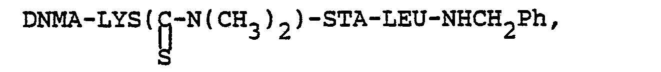 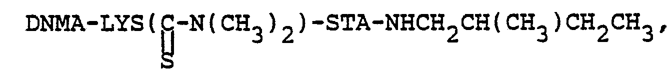 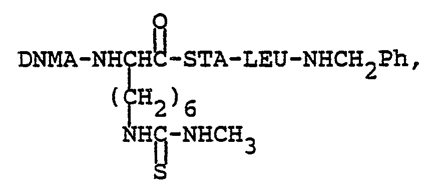 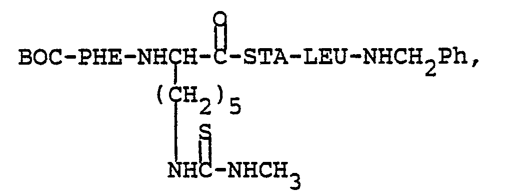 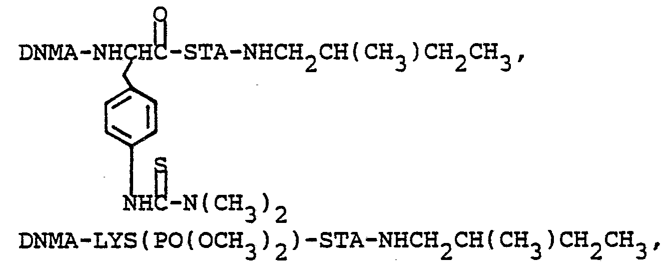 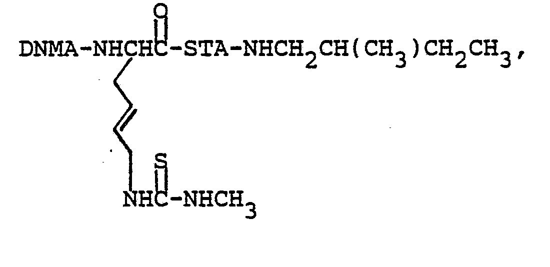 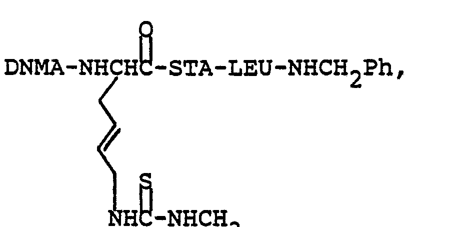 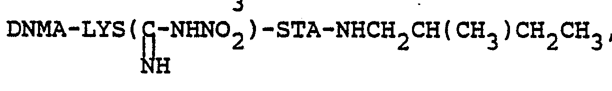 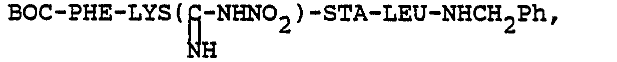 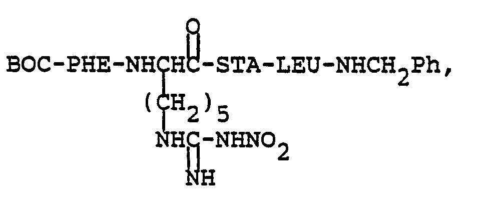 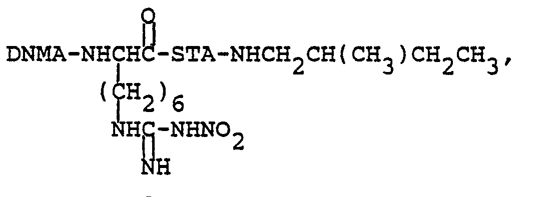 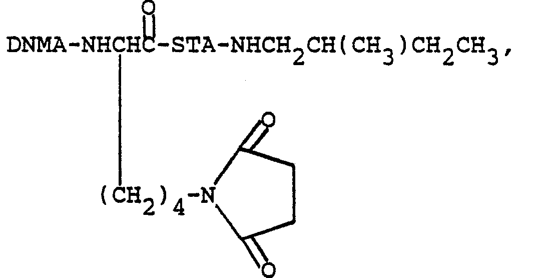 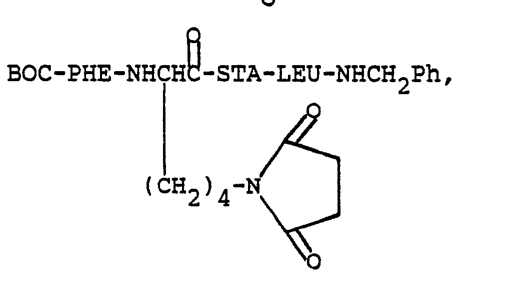 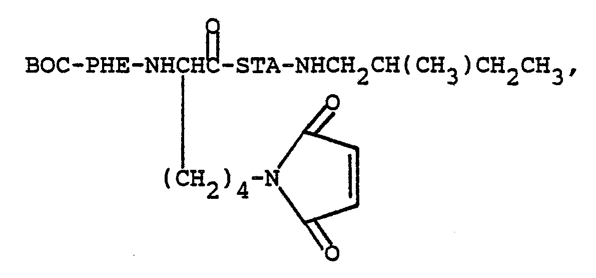 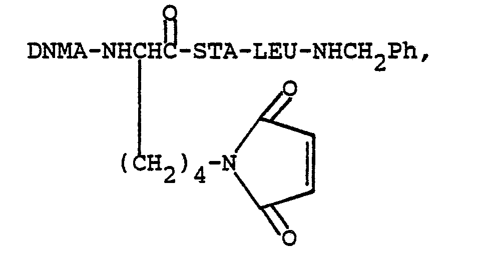 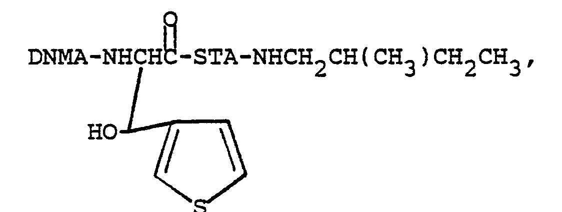 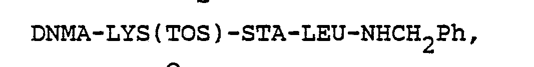 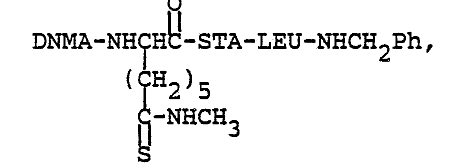 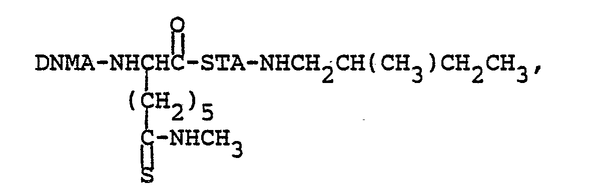 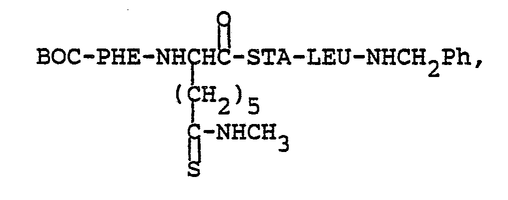 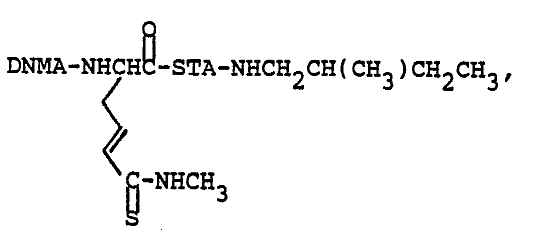 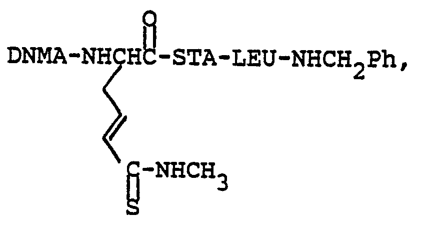 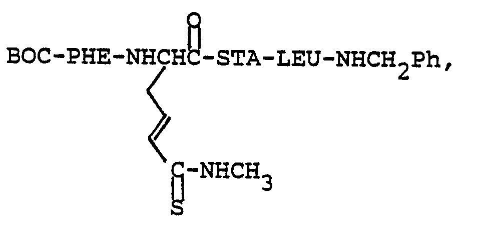 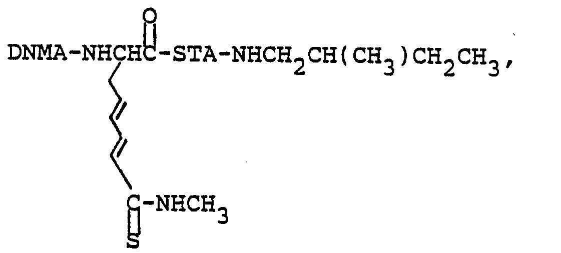 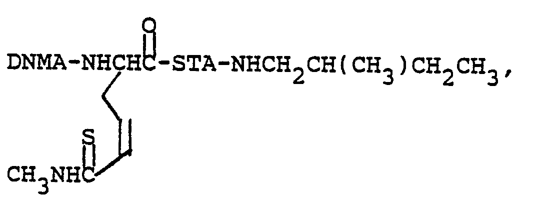 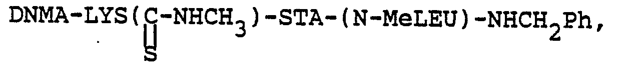 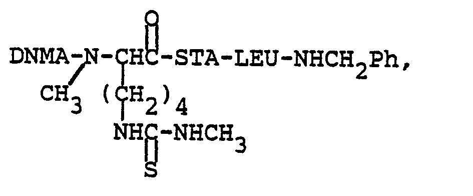 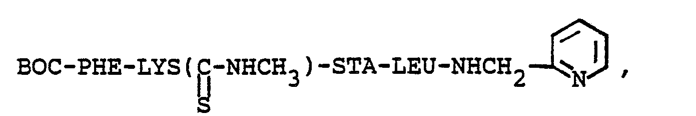 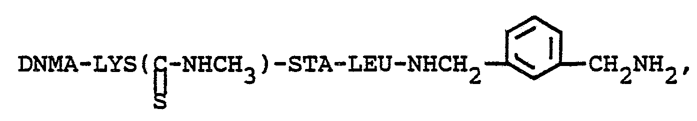 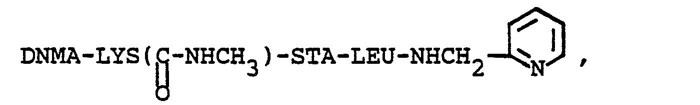 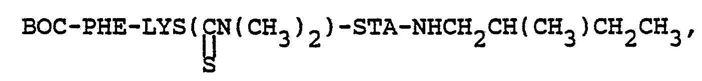 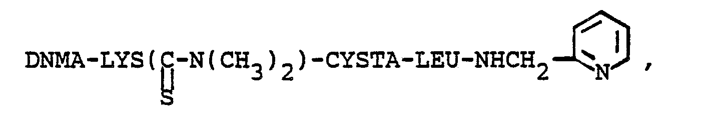 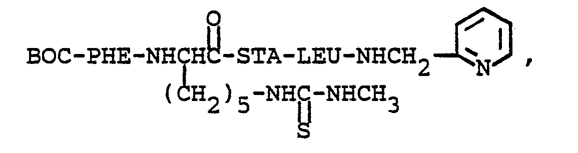 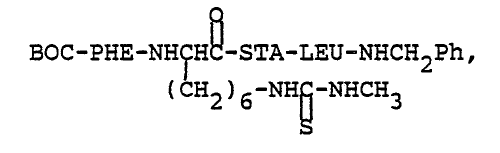 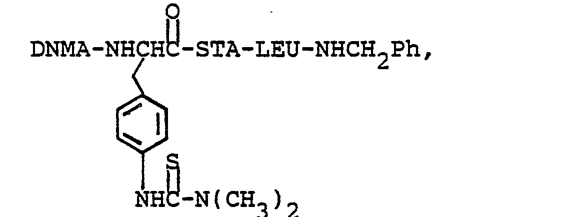 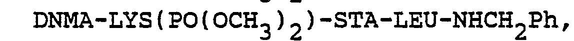 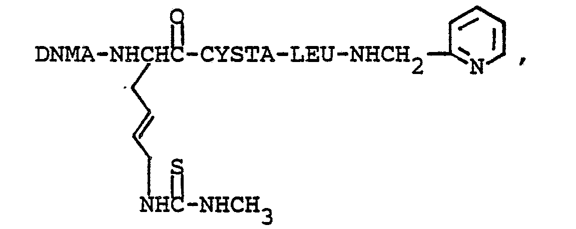 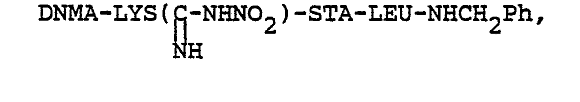 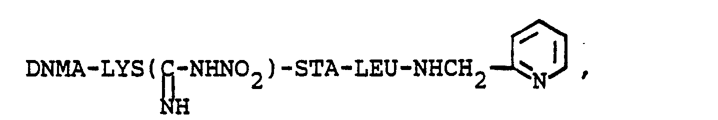 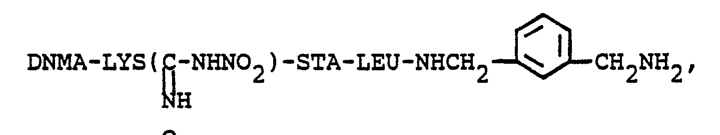 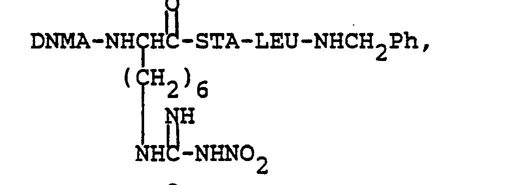 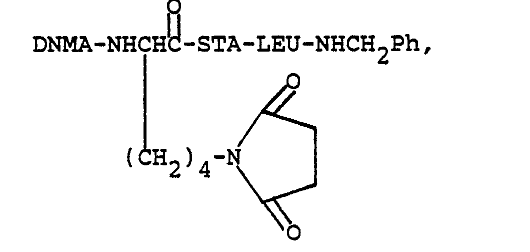 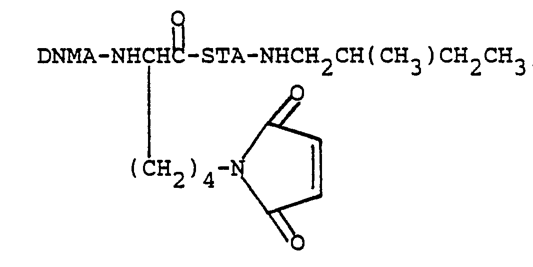 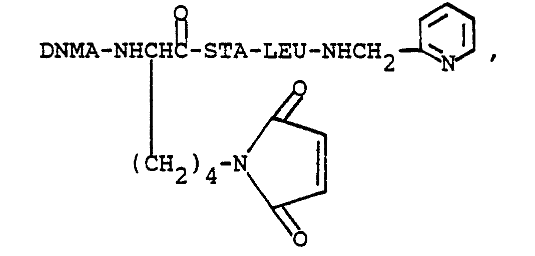 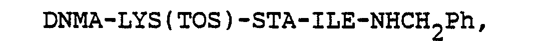 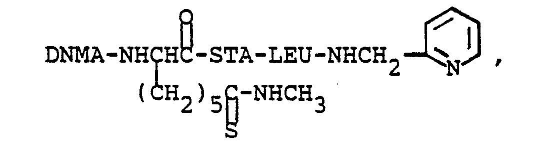 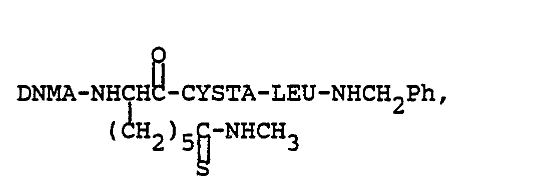 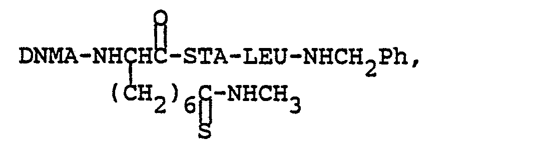 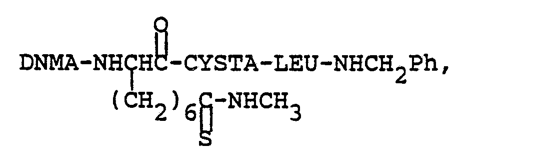 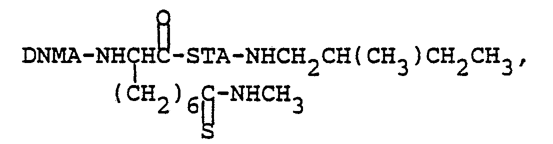 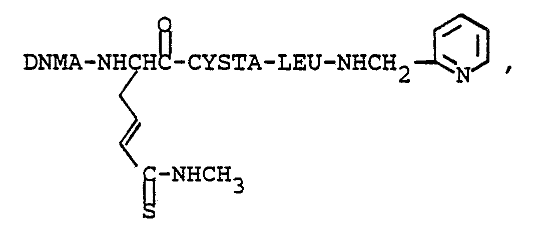 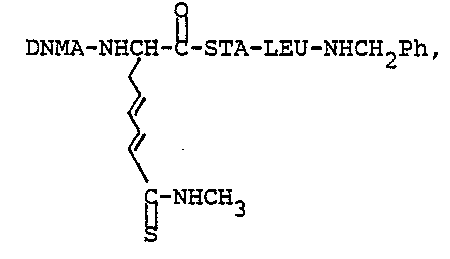 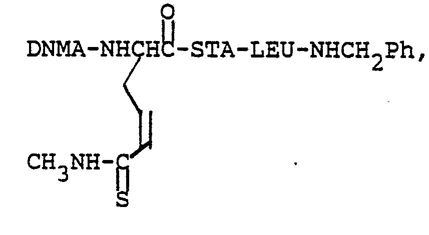 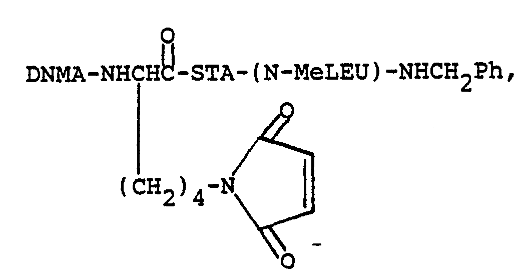 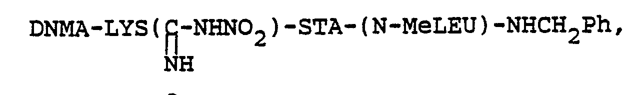 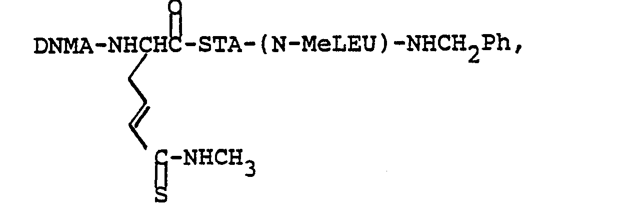 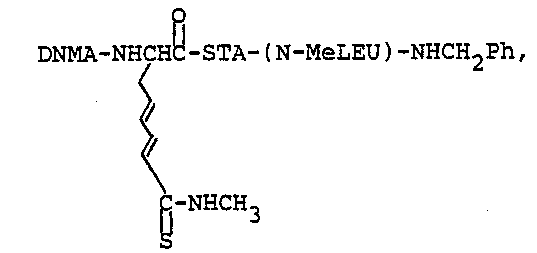 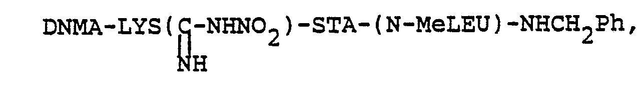 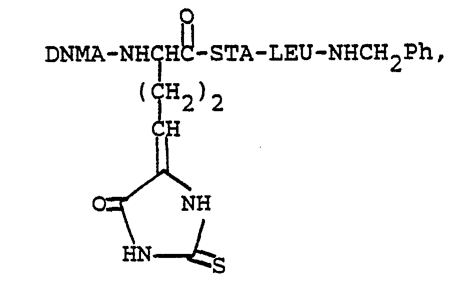 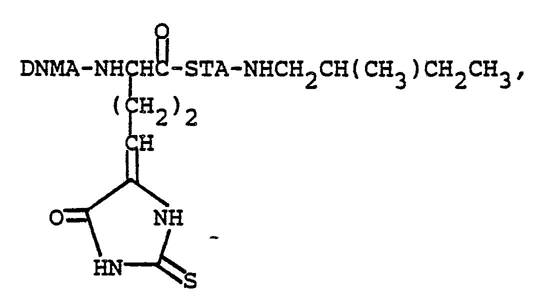 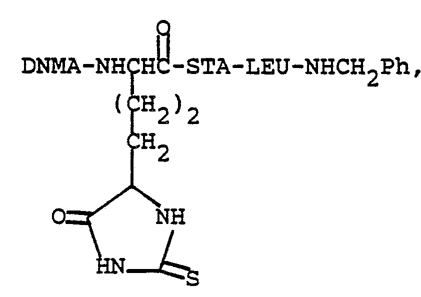 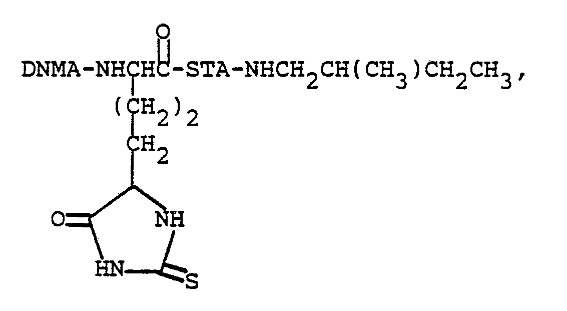 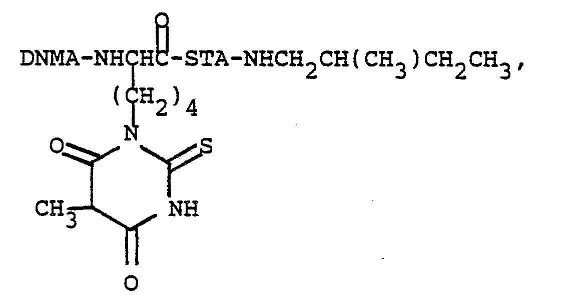 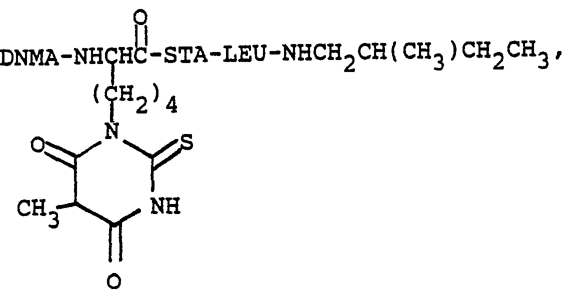 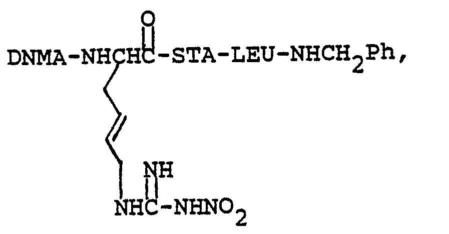 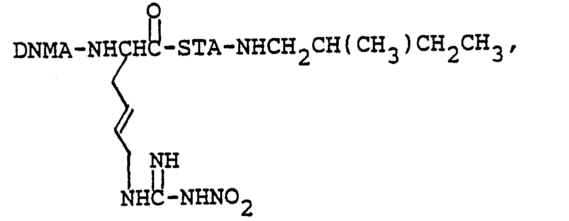 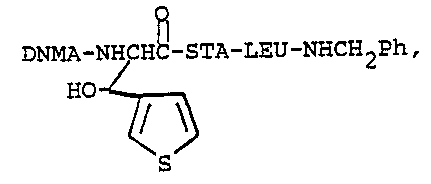 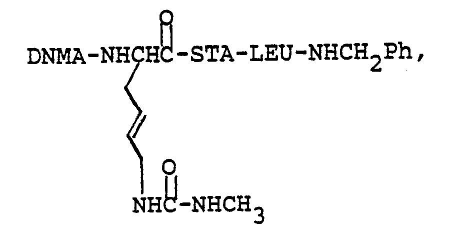 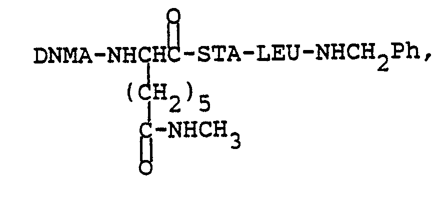 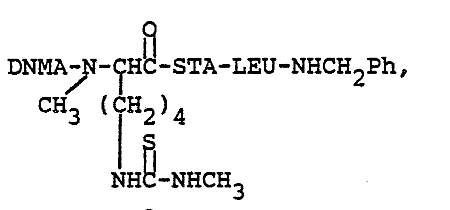 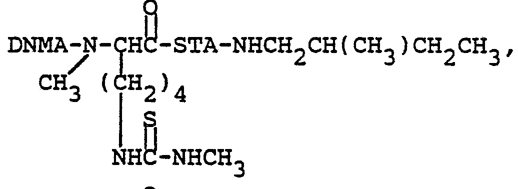 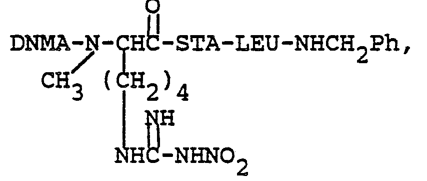 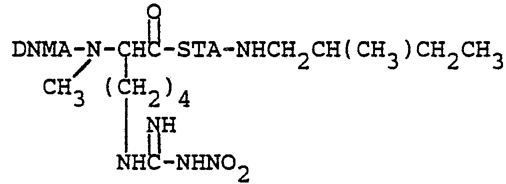 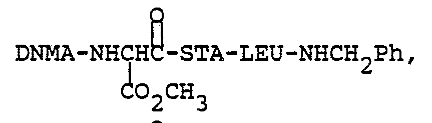 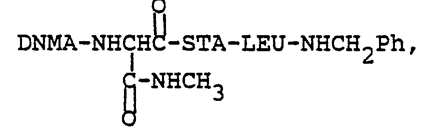 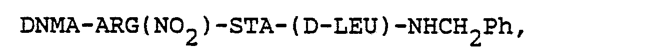 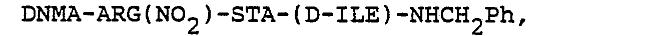 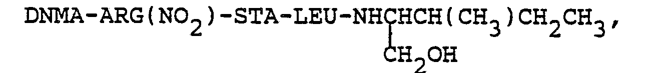 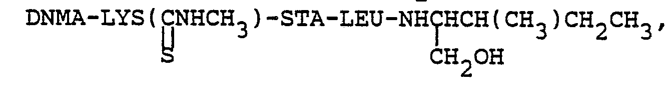 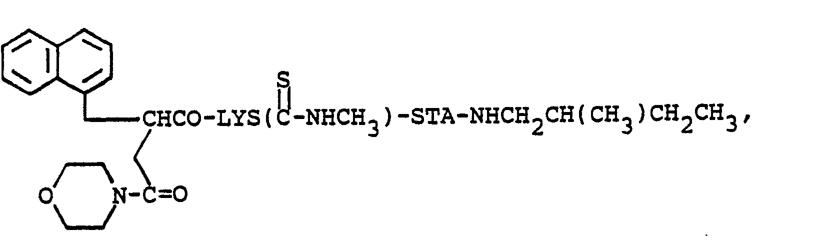 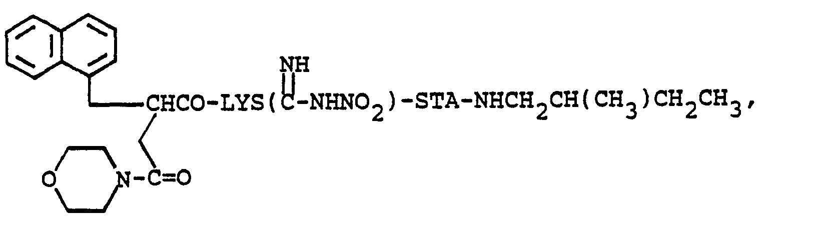 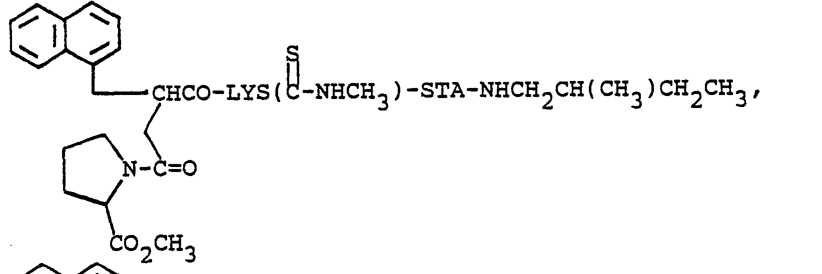 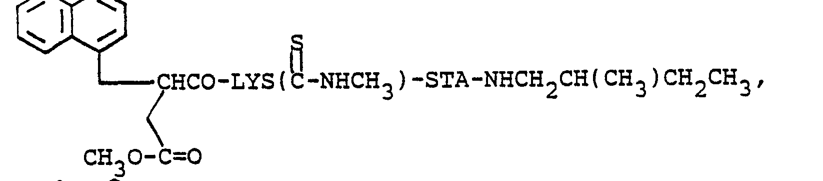 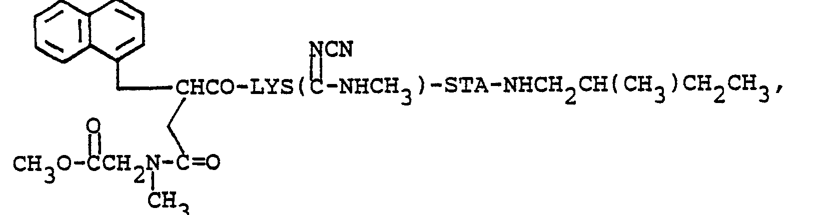 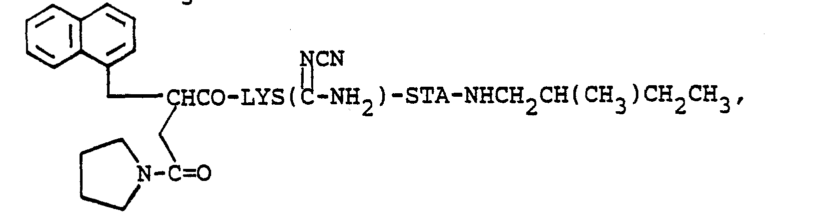 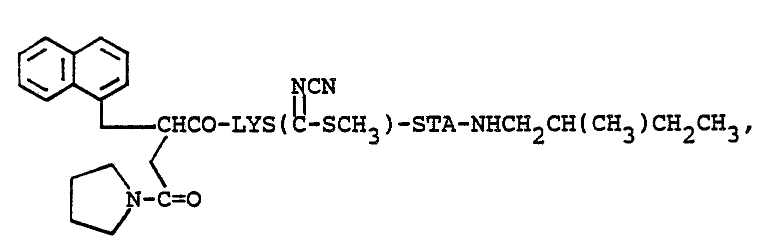 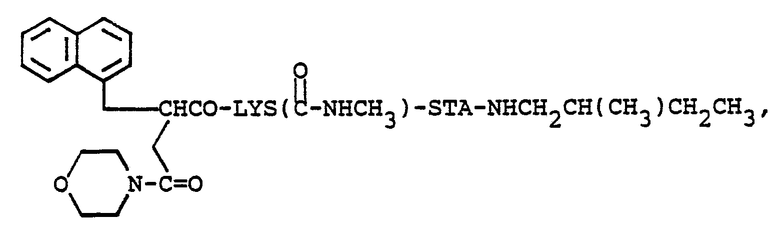 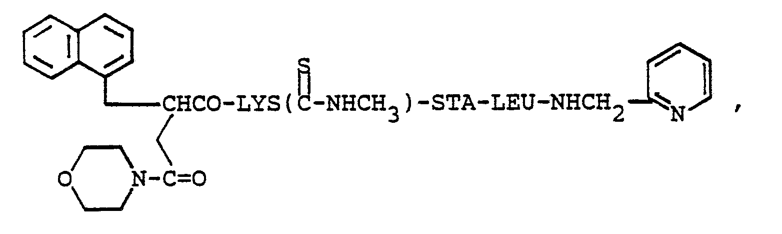 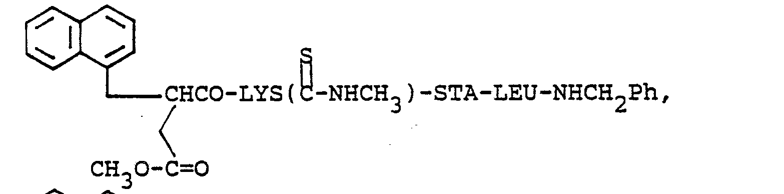 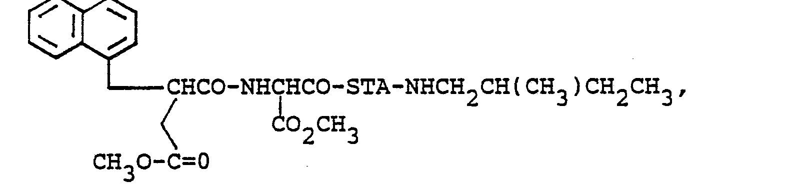 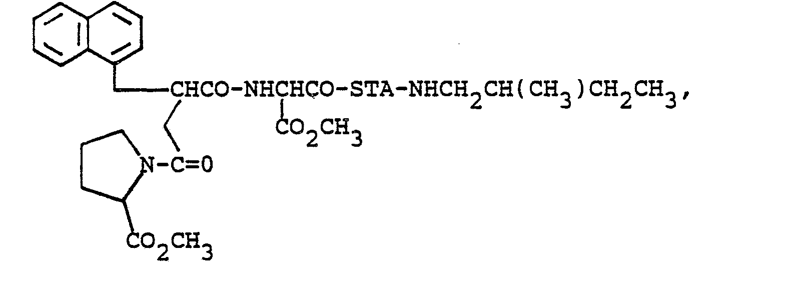 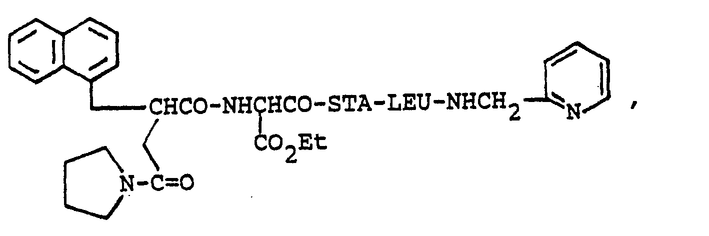 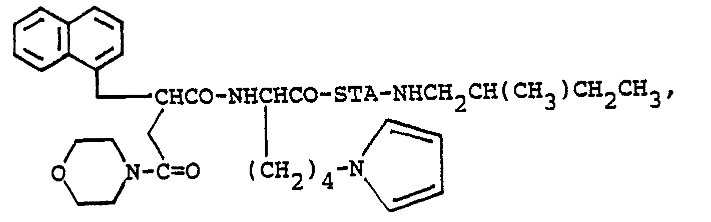 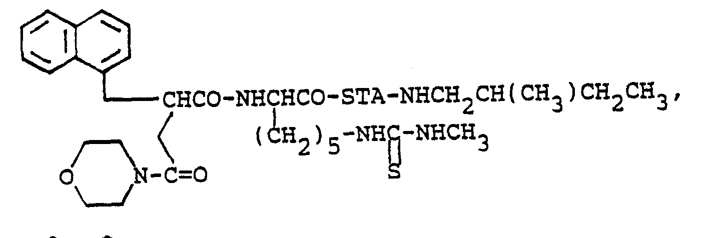 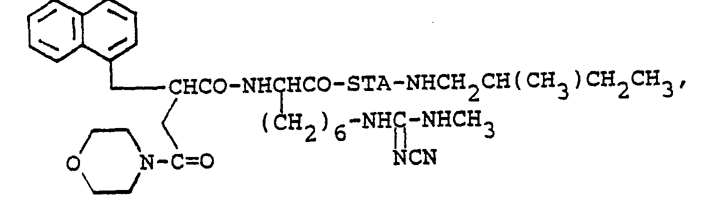 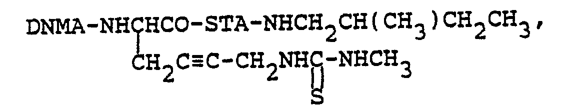 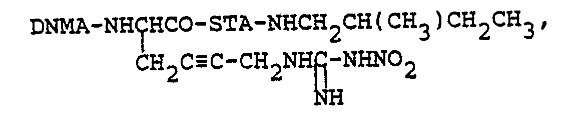 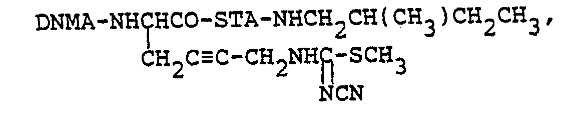 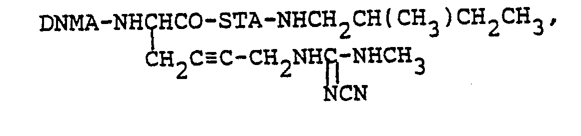 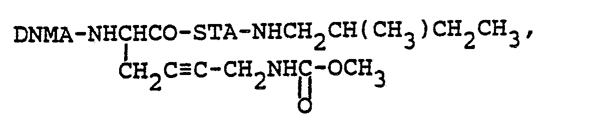 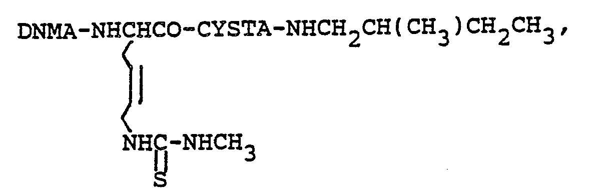 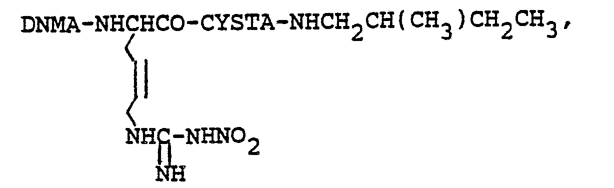 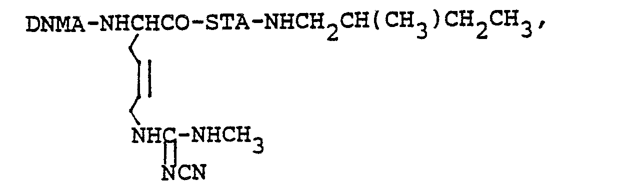 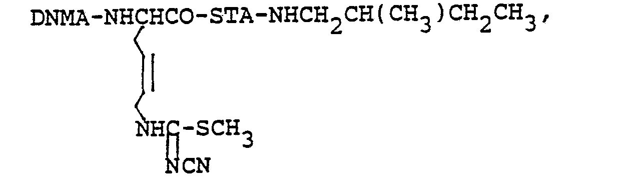 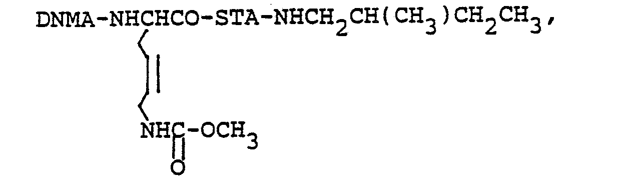 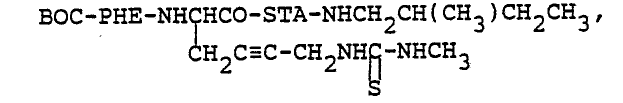 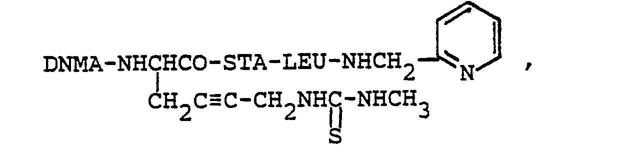 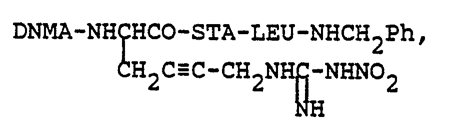 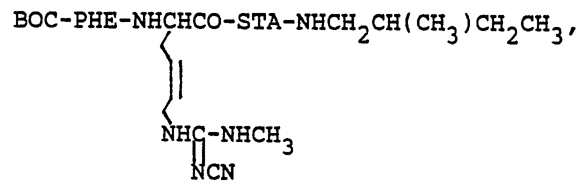 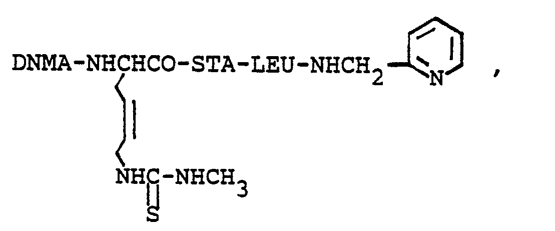 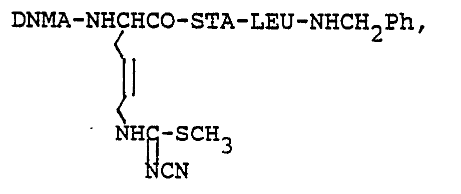 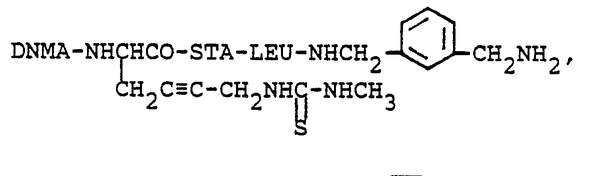 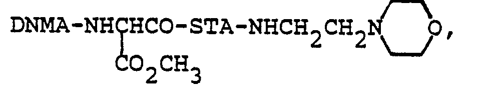 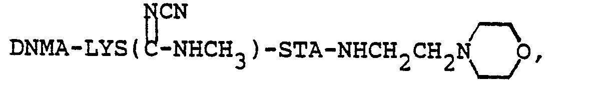 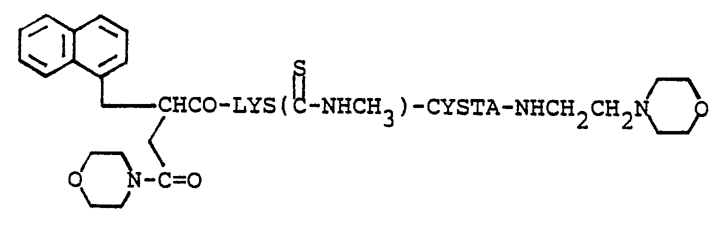 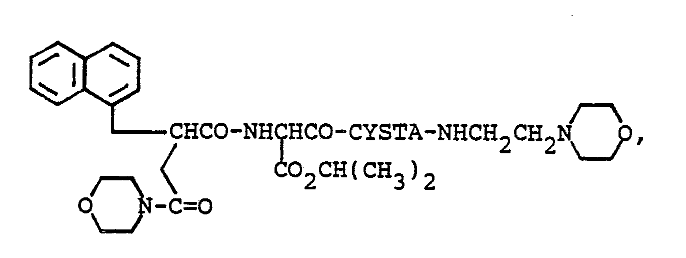 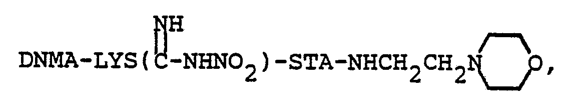 or

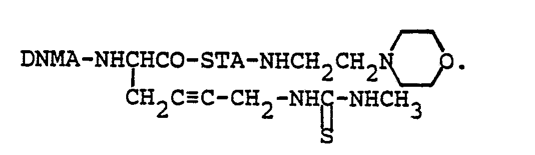

The compounds include solvates and hydrates and pharmaceutically acceptable acid addition salts of the basic compounds of formula I above.

The term pharmaceutically acceptable acid addition salt is intended to mean a relatively nontoxic acid addition salt either from inorganic or organic acids such as, for example, hydrochloric, hydrobromic, hydroiodic, sulfuric, phosphoric, acetic, citric, oxalic, malonic, salicylic, malic, benzoic, gluconic, fumaric, succinic, ascorbic, maleic, tartaric, methanesulfonic and the like. The salts are prepared by contacting the free base form with a sufficient amount of the desired acid to produce a salt in the conventional manner. The free base forms may be regenerated by treating the salt form with a base.

The modified peptides of the present invention possess one or more chiral centers and each center may exist in the R(D) or S(L) configuration. The present invention includes all enantiomeric and epimeric forms as well as the appropriate mixtures thereof.

Some of the above novel peptides may be prepared in accordance with well-known procedures for preparing peptides from their constituent amino acids. Other of the novel peptides of the present invention are prepared by a step-wise procedure or by a fragment coupling procedure depending upon the particular final product desired.

The following scheme illustrates novel methods of preparing certain peptides of the present invention.

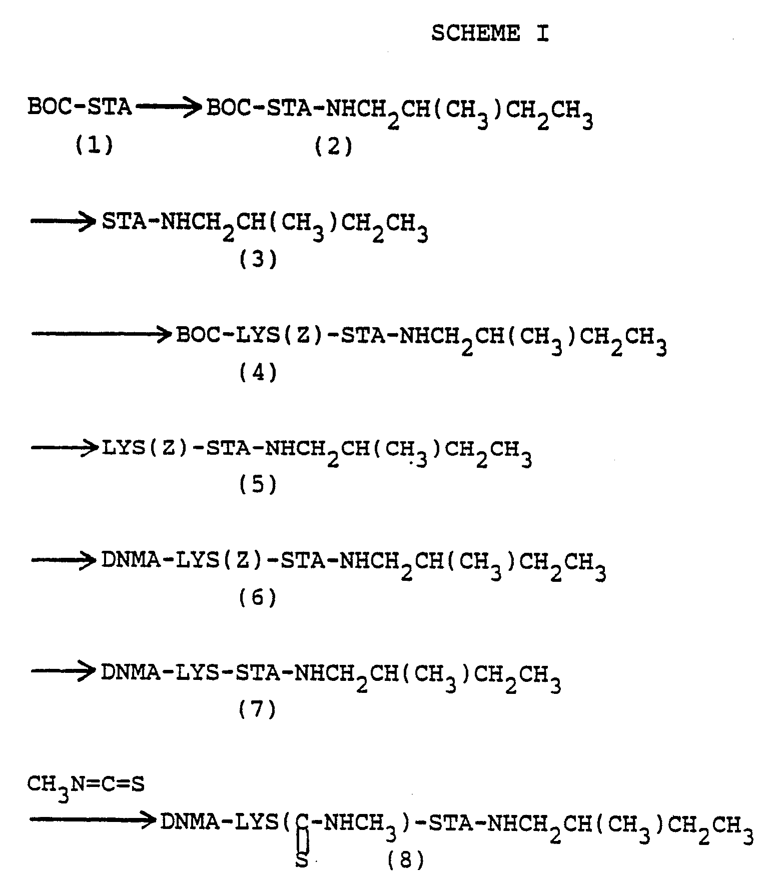

According to Scheme I above, BOC-protected statine (1) is reacted with a primary amine, for example, 2-methylbutylamine, to form the corresponding BOC-protected compound (2). The reaction takes place in an inert solvent much as methylene chloride, tetrahydrofuran, chloroform, dioxane, or ethylacetate. The preferred solvent is methylene chloride. The reaction time varies from 1 to 24 hours. Preferably it takes about 2 to 6 hours. The reaction temperature may also vary from about 15°C to 30°C. Preferably it is approximately 25°C.

The above compound (2) is reacted with a strong acid such as HCI to remove the BOC-protecting group thus forming corresponding compound (3) with a free amino terminus.

The protecting group is removed with a strong acid such as trifluoroacetic, HCI, or HBr. Preferably HCI is used.

The term protecting group refers to those groups intended to protect the N-terminus against undesirable reactions during synthetic procedures or to increase the solubility of the desired final compounds and includes but is not limited to Z and BOC.

Compound 3 is then reacted with BOC-protected LYS(Z) to form the corresponding compound (4). This reaction takes place in an inert solvent, for example DMF, with hydroxybenzotriazole, dicyclohexylcarbodiimide and triethylamine at temperatures of from 0°C to 25°C. The reaction may take as long as 72 hours.

This compound (4) is reacted with a strong acid, such as trifluoroacetic acid, to form the corresponding compound with a free amino terminus (5). The reaction takes place in an inert solvent, preferably dichloromethane, at about 25°C, taking from 0.5 to 2 hours.

This compound (5) is reacted with di-(1-naphthylmethyl)acetic acid in an inert solvent such as DMF at approximately room temperature for from 4 to 16 hours to form a DNMA-terminated peptide (6). The Z group is then removed by catalytic hydrogenation in a polar, inert solvent such as methanol forming peptide (7). The catalyst is preferably palladium on carbon.

This peptide (7) is reacted with methyl isocyanate or methyl isothiocyanate at about room temperature for from 2 to 16 hours in an inert solvent such as methylene chloride to form a compound of the present invention (8).

For compounds of the types illustrated in Examples 1 and 4 the BOC-protected statine is reacted with LEU.NHCH₂Ph.HCI to form the corresponding BOC-protected compound, for example, BOC-STA-LEU-NHCH₂Ph. This compound is reacted with HCI to form the corresponding compound with a free amino terminus. This is reacted with BOC-PHE-ARG(N0₂) to form a compound of the present invention.

The BOC-PHE-ARG(NOz) is prepared by reacting ARG(N0₂).OCH₃.HCI with BOC-PHE to form the corresponding methyl carboxylic acid ester, BOC-PHE-ARG(N0₂)-OCH₃. This ester is converted to the free carboxylic acid by reaction with a strong base such as sodium hydroxide.

Alternatively the diamide (STA-LEU-NHCH₂Ph) with a free amino terminus is reacted with Z-ORN(PHT) to form the corresponding compound, Z-ORN(PHT)-STA-LEU-NHCH₂Ph. This compound is hydrogenated thus removing the Z group to form ORN(PHT)-STA-LEU-NHCH₂Ph. This peptide is reacted with BOC-PHE to form a desired compound of the present invention.

The strategy of peptides chain assembly and selection and removal of protecting groups is discussed in Chapter 1, "The Peptide Bond," in "The Peptides. Analysis, Synthesis, Biology," E. Gross and J. Meienhofer, Eds., Academic Press, New York, NY, 1979, Vol. 1, p. 42-44.

The DCC/HOBT method of coupling is well known to those skilled in the art and is discussed in Chapter 5, "The Carbodiimide Method" by D. H. Rich and J. Singh in "The Peptides. Analysis, Synthesis, Biology," E. Gross and J. Meienhofer, Eds., Academic Press, New York, NY, 1979, Vol. 1, p. 241-261.

Peptide coupling depends on activating the carboxyl group of the protected amino acid prior to condensing it with another peptide containing a free amino terminus. In addition to the DCC coupling method described above, other methods of activating the carboxyl group of a protected amino acid include:
1) The azide method - described in Chapter 4 of the above reference.
2) The mixed anhydride method - described in Chapter 6 of the above reference.
3) The active ester method - described in Chapter 3 of the above reference.

The acyl groups derived from the substituted succinic acid amides may be prepared as follows. 1-Naphthaldehyde is reacted with diethyl succinate in a Stobbe condensation, and the corresponding di-acid is converted to the anhydride with acetic anhydride. Treatment with the appropriate amine gives 2-(1-naphthylmethylene)-3-(substituted aminocarbonyl)propionic acid. Catalytic hydrogenation gives the desired 2-(1-naphthylmethyl)-3-(substituted aminocarbonyl)propionic acid. This acid may be condensed with suitably protected amino acids using the coupling methods known to peptide chemistry, for example, the carbodiimide method. This is discussed in European Application Publication No. 206,807 and European Application Publication No. 200,406.

The compounds of the present invention are useful for treating renin-associated hypertension, congestive heart failure, and hyperaldosteronism. They are also useful as diagnostic tools for determining the presence of renin-associated hypertension or hyperaldosteronism.

The term lower alkyl refers to straight or branched chain alkyl radicals containing from 1 to 6 carbon atoms including but not limited to methyl, ethyl, n-propyl, isopropyl, n-butyl, iso-butyl, sec-butyl, 2-methylhexyl, n-pentyl, 1-methylbutyl, 2,2-dimethylbutyl, 2-methylpentyl, 2,2-dimethylpropyl, n-hexyi and the like.

Aryl means phenyl or other aromatic groups, including mono-or bicyclic, which may be substituted, especially monosubstituted, by F, Cl, Br, I, CF₃, OH, OR, or R.

Aralkyl is as described above for alkyl and aryl.

Pharmaceutical compositions which comprise an effective amount of the compound in combination with a pharmaceutically acceptable carrier are part of the present invention, as well as the methods of use of the compounds above for the manufacturing of those pharmaceuticals for treating renin-associated hypertension in a mammal.

Another equally important aspect of the present invention is a method of use of a compound of the invention in combination with a pharmaceutically acceptable carrier for treating hyperaldosteronism in a mammal.

Yet another important aspect of the present invention is a method of use of a compound of the invention in combination with a pharmaceutically acceptable carrier for treating congestive heart failure in a mammal.

The effectiveness of the aforementioned compounds is determined by a test for in vitro renin inhibitory activity. This activity is determined by a standard radioimmunoassay for angiotensin I. In this assay the enzyme, renin, incubated for two hours at 37° in the presence of a substrate, angiotensinogen, generates the product, angiotensin I. Test compounds are added to the incubation mixture. Relative activity is reported as the IC₅₀, which is the molar concentration of test compound causing a 50% inhibition of the renin activity or as the percent inhibition at 10 ⁶ molar concentration.

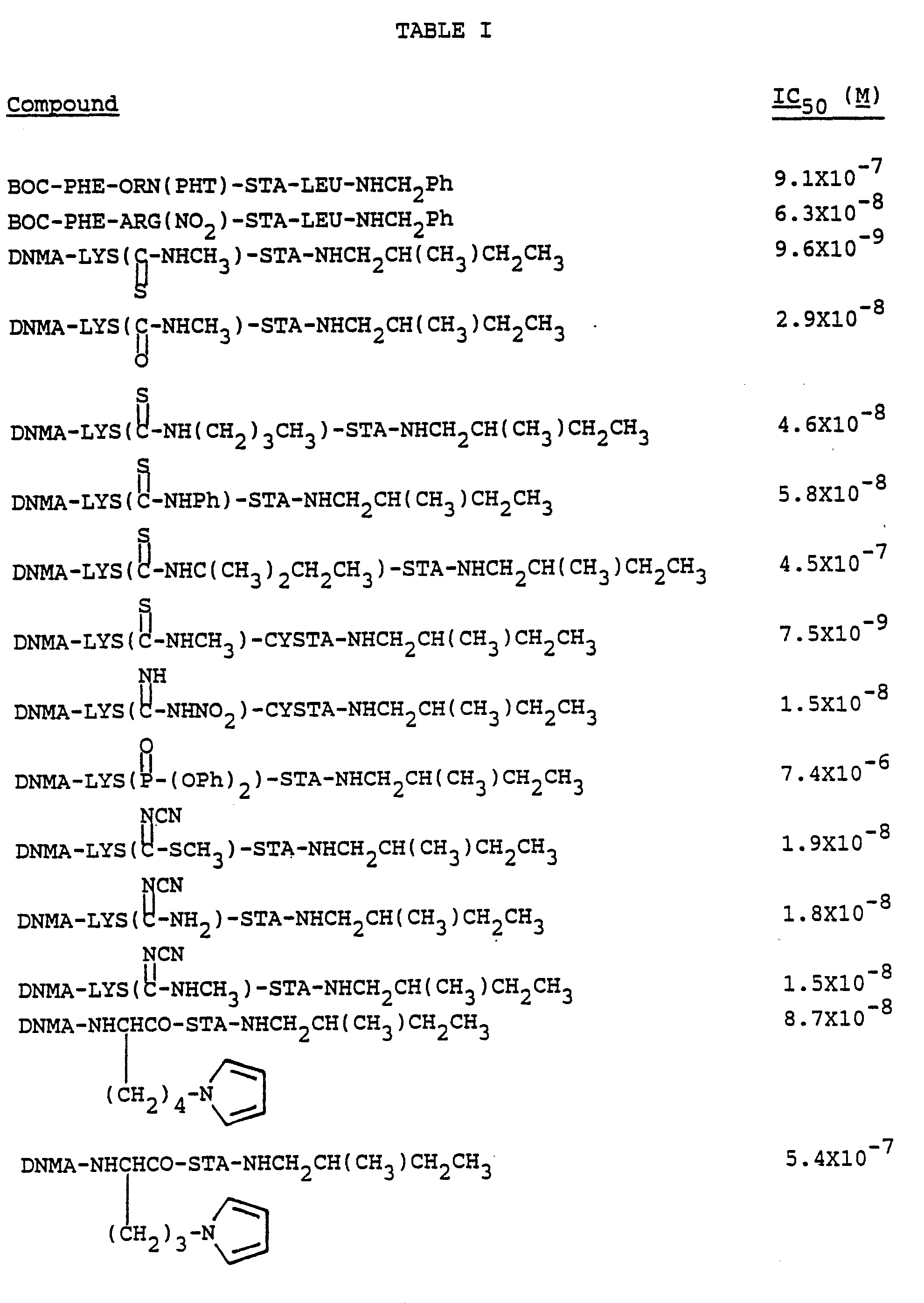

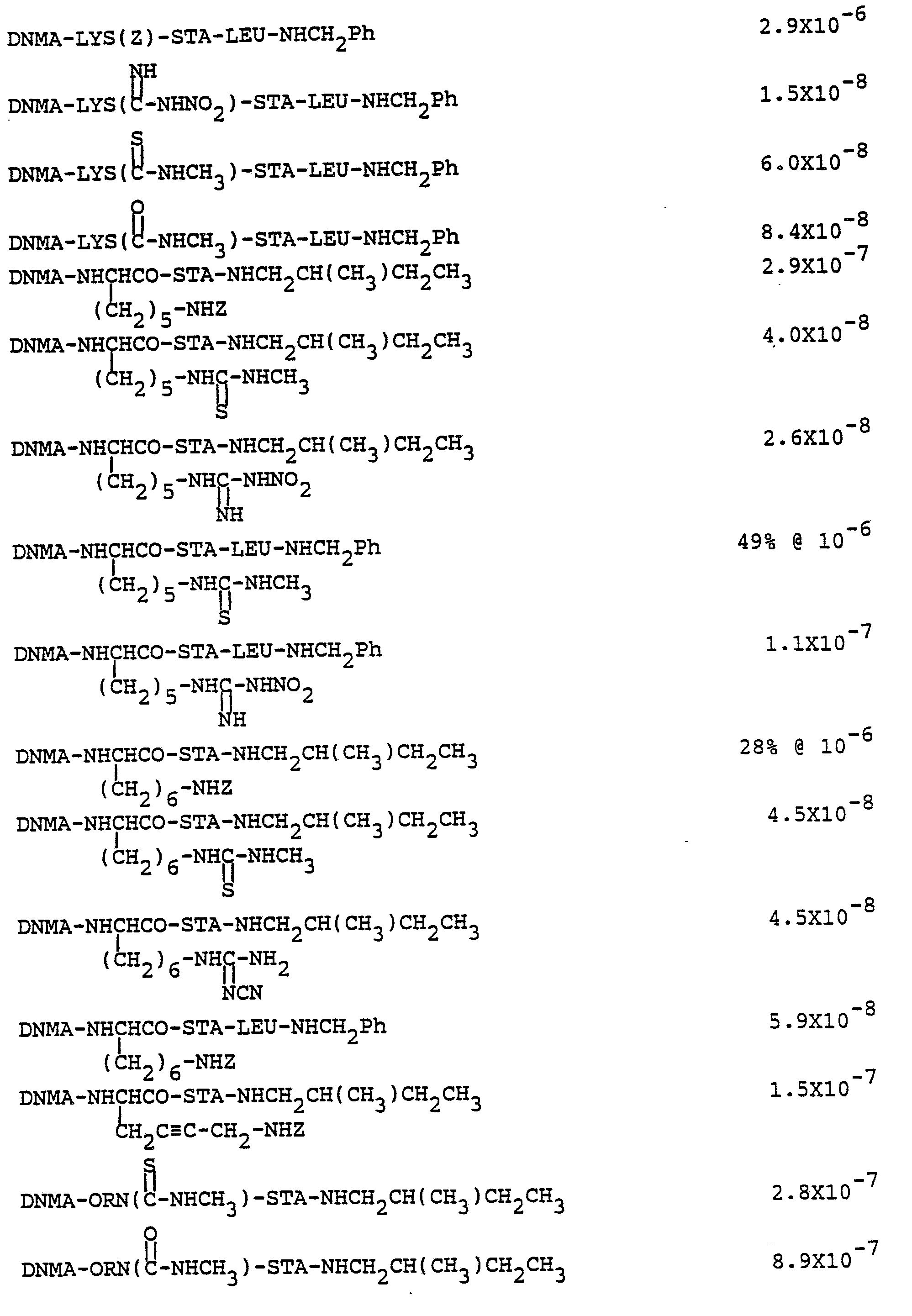

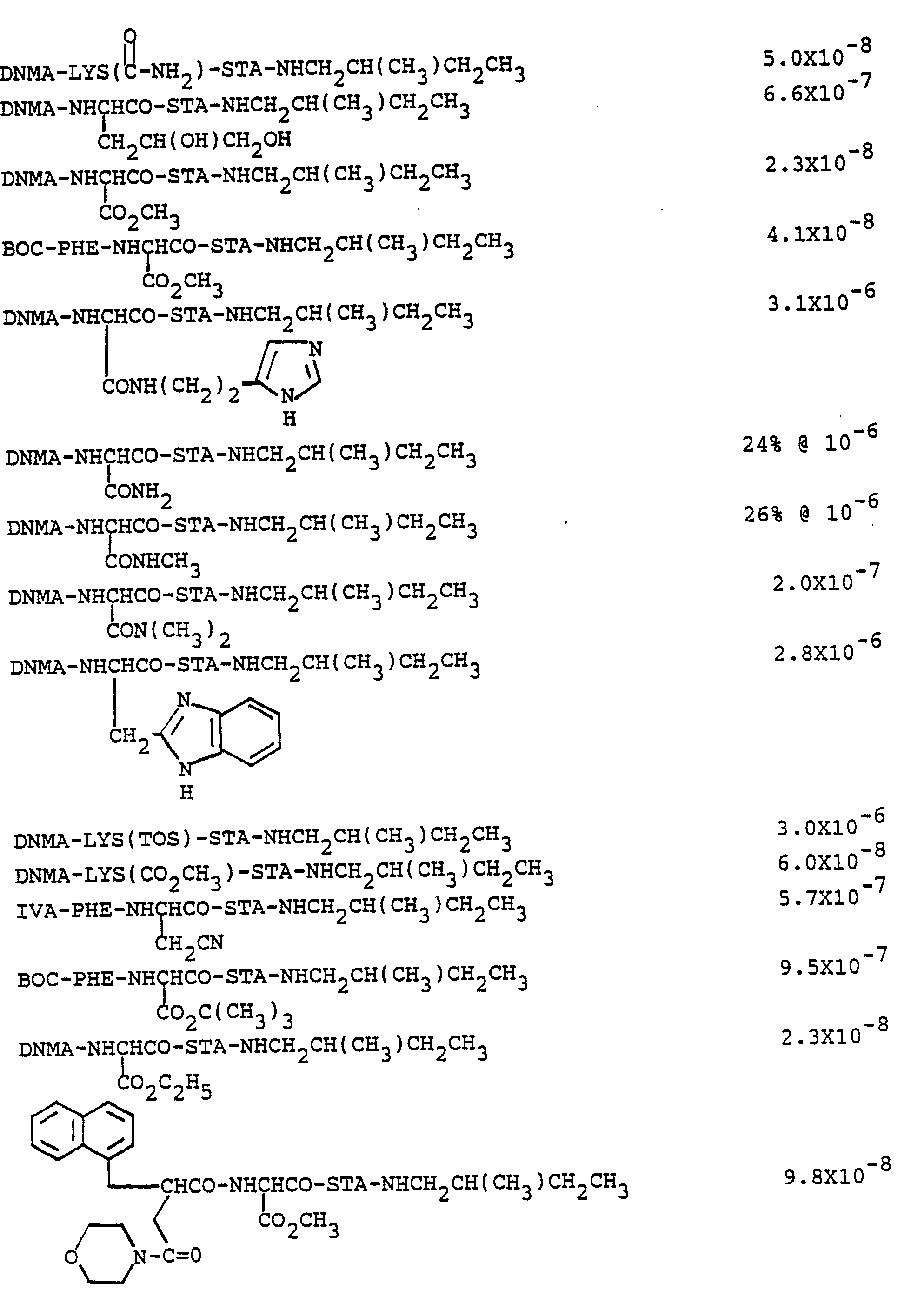

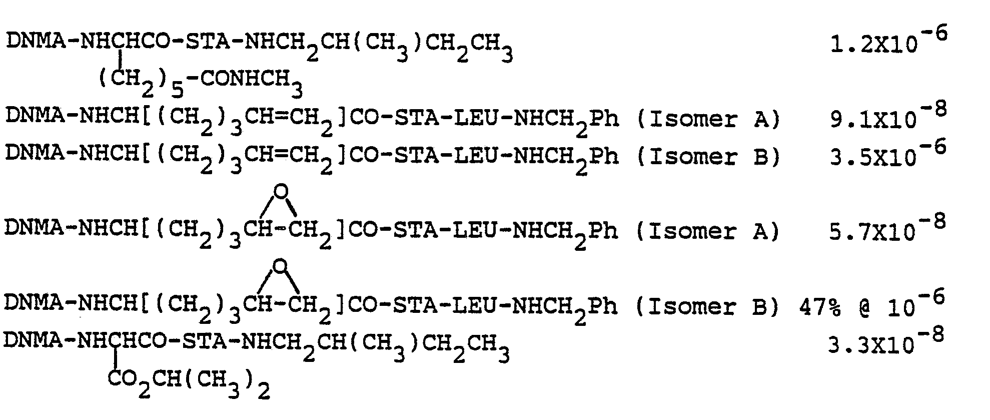

As can be seen from the above table, the compounds of the present invention have a significant effect on the activity of renin and thus are useful for the treatment of hypertension, congestive heart failure, and hyperaldosteronism.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders or tablet disintegrating agents; it can also be encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 to 10 to about 70 percent of the active ingredient. Suitable solid carriers are magnesium carbonate, magnesium stearate, talc, sugar, lactose, pectin, dextrin, starch, gelatin, tragacanth, methylcellulose, a low melting wax, cocoa butter, and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

The compounds of the present invention may be administered orally, buccally, parenterally, by inhalation spray, rectally or topically in dosage unit formulations containing conventional nontoxic pharmaceutically acceptable carriers, adjuvants and vehicles as desired. The term parenteral as used herein includes subcutaneous injections, intravenous, intramuscular, intrasternal injection or infusion techniques.

For preparing suppositories, a low melting wax such as a mixture of fatty acid glycerides or cocoa butter is first melted, and the active ingredient is dispersed homogeneously therein by stirring. The molten homogeneous mixture is then poured into convenient sized molds, allowed to cool, and thereby solidify.

Liquid form preparations include solutions, suspensions, and emulsions. As an example may be mentioned water or water/propylene glycol solutions for parenteral injection. Liquid preparations can also be formulated in solution in aqueous polyethyleneglycol solution. Aqueous suspensions suitable for oral use can be made by dispersing the finely divided active component in water with viscous material, i.e., natural or synthetic gums, resins, methylcellulose, sodium carboxymethylcellulose, and other well-known suspending agents.

Also included are solid form preparations which are intended to be converted, shortly before use, to liquid form preparations for either oral or parenteral administration. Such liquid forms include solutions, suspensions, and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring predetermined volumes of the liquid form preparation as with a syringe, teaspoon, or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners, solubilizing agents, and the like. The liquid utilized for preparing the liquid form preparation may be water, isotonic water, ethanol, glycerine, propylene glycol, and the like, as well as mixtures thereof. Naturally, the liquid utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules, and powders in vials or ampules. The unit dosage form can also be a capsule, cachet, or tablet itself, or it can be the appropriate number of any of these in packaged form.

The quantity of active compound in a unit dose of preparation may be varied or adjusted from 1 mg to 500 mg, preferably 5 to 100 mg according to the particular application and the potency of the active ingredient. The compositions can, if desired, also contain other compatible therapeutic agents.

In therapeutic use as renin inhibitors, the mammalian dosage range for a 70 kg subject is from 1 to 1500 mg of body weight per day or preferably 25 to 750 mg of body weight per day optionally in divided portions. The dosages, however, may be varied depending upon the requirements of the patient, the severity of the condition being treated and the compound being employed. Determination of the proper dosage for a particular situation is within the skill of the art. Generally, treatment is initiated with smaller dosages which are less than the optimum dose of the compound. Thereafter the dosage is increased by small increments until the optimum effect under the circumstances is reached. For convenience, the total daily dosage may be divided and administered in portions during the day if desired.

The following examples are provided to enable one skilled in the art to practice the present invention. These examples are not intended in any way to limit the scope of the invention but are illustrative thereof.

### EXAMPLE 1

### BOC-PHE-ARG(N0₂)-STA-LEU-NHCH₂Ph

A solution of 1.06 g of STA-LEU-NHCH₂PheHCI, 1.2 g BOC-PHE-ARG(NO₂), and 0.37 g HOBT in 80 ml of a 5/3 solution of CH₂CI₂/DMF was cooled in ice and treated with 0.7 ml of Et₃N. DCC (0.59 g) was then added and the solution kept at 0° for one hour, then at room temperature overnight. The solvent was removed under reduced pressure, and the residue taken up in EtOAc. Insolubles were filtered off and the filtrate was washed with Na₂C0₃ solution, citric acid solution, and brine. After drying over MgS0₄ and removal of the solvent under reduced pressure, the residue was chromatographed on silica gel, eluting with a gradient of CH₂CI₂/MeOH (97/3) to CH₂Cl₂/MeOH (92/8). Combining the appropriate fractions gave 1.74 g (82%) of the product as a white foam.

Calcd. for C₄₁H₆₃N₉O₉ (MW 826.01):
C, 59.62; H, 7.69; N, 15.26 Found
C, 59.49; H, 7.48; N, 14.59.

### EXAMPLE 2

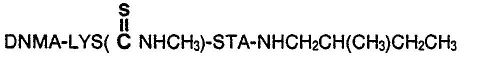

DNMA-LYS-STA-NHCH₂CH(CH₃)CH₂CH₃ (0.5 g) was dissolved in dichloromethane (15 ml) and cooled to 0°. Methyl isothiocyanate (0.053 g) was added, and the mixture was allowed to warm to 25° and stir for six hours. The solvent was evaporated and the crude product was purified by chromatography on silica gel, eluting with a gradient of EtOAc/hexane (1:1) to pure EtOAc. The appropriate fractions were combined to give 0.3 g of product.

Calcd. for C₄₅H₆₁N₅O₄S•0.25 C₄H₈O₂•0.5 HzO:

C, 69.13; H, 8.07; N, 8.76 Found C, 69.13; H, 8.19; N, 8.84.

EXAMPLE 3

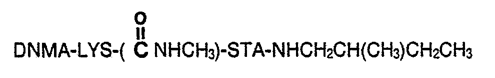

DNMA-LYS-STA-NHCH₂CH(CH₃)CH₂CH₃ (0.5 g) was dissolved in dichloromethane (15 ml) and cooled to 0°. Methyl isocyanate (0.042 ml) was added, and the mixture was allowed to warm to 25° and stir for 4 hours. EtOAc was added to the white suspension and the mixture was filtered and the solid dried to give 0.3 g of product.

Calcd. for C₄₅H₆₆N₅O₅•0.33 C₄H₈O₂•0.5 H₂O: C, 70.41; H, 8.25; N, 8.86 Found C, 70.26; H, 8.35; N, 8.82.

### EXAMPLE 4

### BOC-PHE-ORN(PHT)-STA-LEU-NHCH₂Ph

BOC-PHE (0.587 g, 2.21 mmole) and HOBT•H₂O (0.314 g, 3.32 mmole) was dissolved in 2 ml DMF, diluted with 20 ml CH₂C1₂, and cooled to -5°. DCC (0.48 g, 2.32 mmole) was added as a solution in 5 ml CH₂Cl₂, followed by a cooled solution of ORN(PHT)-STA-LEU-NHCH₂Ph (1.35 g, 2.17 mmole) in 10 ml CH₂CI₂. After stirring at 25° overnight, the mixture was filtered, stripped, and resuspended in EtOAc. The suspension was filtered, and the filtrate was washed with 1 N citric acid, giving a precipitate. The suspension was washed with brine, saturated NaHCO₃ solution and brine. The solids were dissolved by the addition of CHCl₃ and warming. Residual aqueous phase was separated, and the organic phase was dried over MgSO₄, filtered, and stripped to an oil. The oil was dissolved in CHCl₃, and a precipitate was obtained by the addition of Et₂0. The solid was filtered, washed with Et₂O, and dried giving 1.64 g of product. IR, NMR, and mass spectral analyses confirmed the structure.

Calcd. for C₄₈H₆₄N₆O₉ (MW 869.08):
C, 66.34; H, 7.42; N, 9.67 Found
C, 66.34; H, 7.57; N, 9.86

### EXAMPLE 5

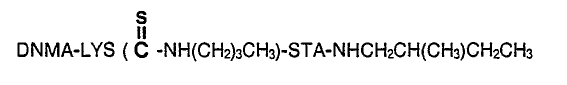

Using the procedure of Example 2, but substituting n-butyl isothicocyanate for methyl isothiocyanate gave the title compound. It was purified by chromatography on silica gel, eluting with EtOAc/hexane (3/1). The structure was confirmed by NMR and mass spectroscopy. Calcd. for C₄₈H₆₇N₅O₄S (MW 810.06):
C, 71.17; H, 8.34; N, 8.65 Found
C, 70.92; H, 8.33; N, 8.55.

### EXAMPLE 6

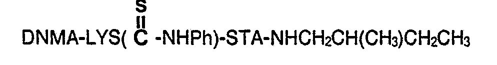

Using the procedure of Example 2 but substituting phenyl isothiocyanate for methyl isothiocyanate gave the title compound. It was purified by chromatography on silica gel, eluting with EtOAc/hexane (3/1). The material was transferred to a vial with the aid of CH₂CI₂. The structure was confirmed by NMR and mass spectroscopy.

Calcd. for C₅₀H₆₃N₅O₄S•0.2CH₂Cl₂ (MW 847.03): C, 71.18; H, 7.54; N, 8.27 Found C, 70.94; H, 7.56; N, 8.19.

### EXAMPLE 7

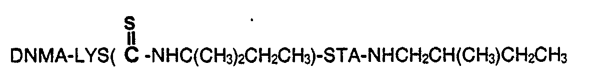

Using the procedure of Example 2 but substituting 2,2-dimethylpropyl isothiocyanate for methyl isothiocyanate gave the title compound. It was purified by chromatography on silica gel, eluting with EtOAc/hexane (1/1), then EtOAc. The structure was confirmed by NMR and mass spectroscopy. Calcd. for C₄₉H₆₉N₅O₄S (MW 824.08):
C, 71.41; H, 8.44; N, 8.50 Found
C, 71.50; H, 8.50; N, 8.43.

### EXAMPLE 8

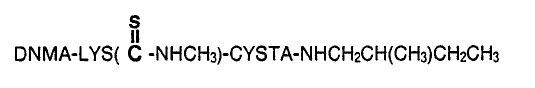

A solution of 500 mg (0.68 mmole) of DNMA-LYS-CYSTA-NHCH₂CH(CH₃)CH₂CH₃ in 10 ml CH₂Cl₂ was cooled in ice and 50 mg (0.68 mmole) of methyl isothiocyanate added. The solution was stirred for four days at room temperature, the solvent removed under reduced pressure, and the residue chromatographed on silica gel, eluting with EtOAc/hexane (1/1), then EtOAc. There was obtained 0.4 g of product. The structure was confirmed by NMR and mass spectroscopy.

Calcd. for C₄₈H₆₅N₅O₄S•0.44C (MW 846.80): C, 70.57; H, 8.16; N, 8.27 Found C, 70.92; H, 8.24; N, 8.41.

### EXAMPLE 9

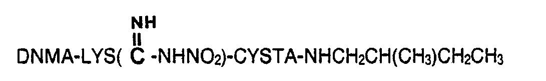

A solution of 400 mg (0.54 mmole) of DNMA-LYS-CYSTA-NHCH₂CH(CH₃)CH₂CH₃ in 10 ml EtOH was treated with 74 mg (0.54 mmole) of 2-methyl-1-nitro-2-thiopseudourea (J. Am. Chem. Soc. 76, 1877 (1954)) and stirred at room temperature for four days. The solvent was removed under reduced pressure and the residue chromatographed on silica gel, eluting with EtOAc/hexane (1/1), then EtOAc. There was obtained 0.35 g of product. The structure was confirmed by NMR and mass spectroscopy. Calcd. for C₄₇H₆₃N₇O₆•0.4C₄H₈O₂ (MW 857.27):

C, 68.09; H, 7.78; N, 11.44 Found

C, 67.58; H, 7.78; N, 11.58.

### EXAMPLE 10

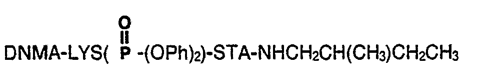

A solution of 500 g (0.72 mmole) of DNMA-LYS-STA-NHCH₂CH(CH₃)CH₂CH₃ in 15 ml of CH₂Ci₂/THF (4/1) was cooled in ice and 0.1 ml (0.72 mmole) of Et₃N added, followed by 0.15 ml (0.72 mmole) of diphenyl chlorophosphate. The solution was stirred at 0° for three hours and the solvent then removed. under reduced pressure. The residue was taken up in EtOAc and washed with saturated NaHC0₃ and saturated NaCI. Drying and removal of the solvent under reduced pressure gave the crude product which was purified by chromatography on silica gel, eluting with EtOAc/hexane (1/1) to EtOAc/hexane (5/1). There. was obtained 0.46 g of product. The structure was confirmed by NMR and mass spectroscopy.

Calcd. for C₅₅H₆₇N₄O₇P•H₂O (MW 945.10):
C, 69.89; H, 7.36; N, 5.93 Found C, 69.64; H, 7.17; N, 6.06.

### EXAMPLE 11

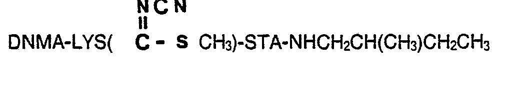

A solution of 0.31 g (0.44 mmole) of ONMA-LYS-STA-NHCH₂CH(CH₃)CH₂CH₃ in 10 ml CHCl₃ was treated with 70 mg (0.57 mmole) of dimethylcyanimidodithiocarbonate (Synthesis 332 (1975)) and stirred at room temperature overnight. The solvent was removed under reduced pressure and the residue chromatographed on silica gel, eluting with EtOAc/hexane (1/1), then EtOAc. There was obtained 0.3 g of product. The structure was confirmed by NMR and mass spectroscopy. Calcd. for C₄₆H₆₀N₆O₄S•H₂O (MW 811.00):

C, 68.12; H, 7.71; N, 10.36 Found

C, 68.23; H, 7.36; N, 10.25.

### EXAMPLE 12

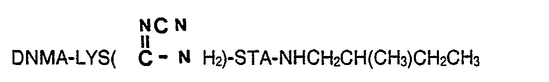

A solution of 0.3 g (0.43 mmole) of DNMA-LYS-STA-NHCH₂CH(CH₃)CH₂CH₃ in 10 ml CHCl₃ was treated with 0.11 g (0.5 mmole) of diphenyl cyanocarboimidate and allowed to stir at room temperature overnight. The solvent was removed under reduced pressure and the residue taken up in 10 ml MeOH and ammonia bubbled through until saturated. The solution was allowed to stir at room temperature for twenty-four hours, then at 30° for five hours. The solvent was removed under reduced pressure and the residue chromatographed on silica gel, eluting with EtOAc, then EtOAciMeOH (9/1). There was obtained 0.25 g of product. The structure was confirmed by NMR and mass spectroscopy.

Calcd. for C₄₅H₅₉N₇O₄•1.2H₂O (MW 783.60): C, 68.97; H, 7.90; N, 12.51 Found C, 68.71; H, 7.57; N, 12.20.

### EXAMPLE 13

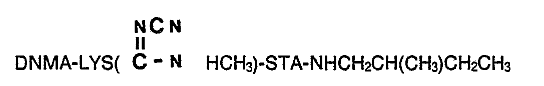

A solution of 0.3 g (0.43 mmole) of DNMA-LYS-STA-NHCH₂CH(CH₃)CH₂CH₃ in 10 ml CHCl₃ was treated with 0.11 g (0.5 mmole) of diphenyl cyanocarboimidate and stirred at room temperature for one hour. The solvent was removed under reduced pressure and the residue taken up in 10 ml MeOH and methylamine gas was bubbled through the solution until saturated. After stirring for one hour, the solvent was removed under reduced pressure and the residue chromatographed on silica gel, eluting with EtOAc, then EtOAc/MeOH (9/1). There was obtained 0.26 g of product. The structure was confirmed by NMR and mass spectroscopy.

Calcd. for C₄₆H₆₁N₇O₄•H₂O (MW 794.02): C, 69.58; H, 8.00; N, 12.35 Found C, 69.13; H, 7.91; N, 12.31.

### EXAMPLE 14

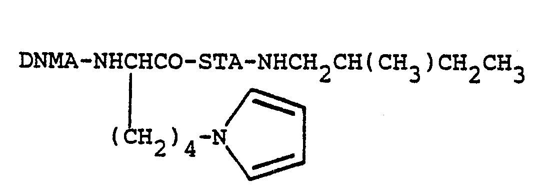

A solution of 0.5 g (1.2 mmole) of 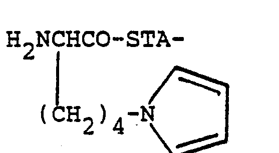 NHCH₂CH(CH₃)CH₂CH₃, 0.4 g (1.2 mmole) of di-(1-naphthylmethyl)acetic acid, and 0.16 g (1.2 mmole) of HOBT in 15 ml DMF was cooled in ice and 0.25 g (1.2 mmole) of DCC added. The solution was allowed to warm to room temperature and was stirred overnight. The mixture was filtered and the filtrate diluted with EtOAc and washed with H₂0, saturated NaHCO₃, then saturated NaCI. Drying and removal of the solvent under reduced pressure gave the crude product which was chromatographed on silica gel, eluting with EtOAc/hexane (1/1), then EtOAc/hexane (3/1). There was obtained 0.3 g of product. The structure was confirmed by NMR and mass spectroscopy.

Calcd. for C₄₇H₆₀N₄O₄ (MW 744.98): C, 75.77; H, 8.12; N, 7.52 Found C, 75.82; H, 8.15; N, 7.41.

### EXAMPLE 15

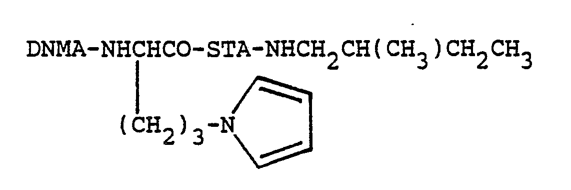

A solution of 0.51 g (1.5 mmole) of di-(1-naphthylmethyl)acetic acid, 0.61 g (1.5 mmole) of 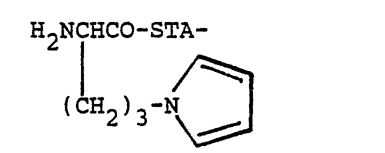 NHCH₂CH(CH₃)CH₂CH₃, and 0.2 g (1.5 mmole) of HOBT in 15 ml DMF was cooled in ice and 0.31 g (1.5 mmole) of DCC added. The solution was allowed to stir at room temperature overnight. The mixture was filtered and the filtrate diluted with EtOAc. This was washed with H₂0, saturated NaHC0₃, H₂0, and saturated NaCl. Drying and removal of the solvent under reduced pressure gave the crude product which was chromatographed on silica gel, eluting with EtOAc/hexane (1/1). There was obtained 0.4 g of product. The structure was confirmed by NMR and mass spectroscopy.

Calcd. for C₄₆H₅₈N₄O₄•0.5H₂O (MW 739.96): C, 74.66; H, 8.04; N, 7.57 Found C, 74.73; H, 8.02; N, 7.66.

### EXAMPLE 16

### DNMA-LYS(Z)-STA-LEU-NHCH₂Ph

A solution of 5.6 g (8.7 mmole) of LYS(Z)-STA-LEU-NHCH₂Ph, 3.0 g (8.8 mmole) of di-(1-naphthylmethyl)acetic acid, and 1.18 g (8.7 mmole) of HOBT in 50 ml DMF was cooled in ice and 1.8 g (8.7 mmole) of DCC added. The solution was allowed to warm to room temperature and stir overnight. The mixture was filtered and the filtrate diluted with EtOAc and washed with H₂0, saturated NaHC0₃, and saturated NaCI. Drying and removal of the solvent under reduced pressure gave the crude product which was chromatographed on silica gel, eluting with EtOAc/CHCl₃ (1/1), then EtOAc/CHCl₃ (4/1). There was obtained 5.5 g of the product. The structure was confirmed by NMR and mass spectroscopy.

Calcd. for C₅₉H₇₁N₅0₇ (MW 962.20): C, 73.64; H, 7.44; N, 7.28 Found C, 73.81; H, 7.46; N, 7.40.

### EXAMPLE 17

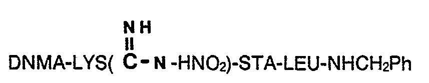

A solution of 1.0 g (1.2 mmole) of DNMA-LYS-STA-LEU-NHCHzPh in 50 ml EtOH was treated with 0.18 g (1.3 mmole) of 2-methyl-1-nitro-2-thiopseudourea and stirred for three days at room temperature with periodic warming to dissolve the white solid which formed. The solvent was then removed under reduced pressure and the residue chromatographed on silica gel, eluting with EtOAc/CHCl₃ (1/1), then EtOAc/CHCl₃ - (3/1). There was obtained 0.5 g of product. The structure was confirmed by NMR and mass spectroscopy.

Calcd. for C₅₂H₆₆N₈O₇•0.4CHCl₃ (MW 962.87): C, 65.36; H, 6.95; N, 11.64 Found C, 65.43; H, 7.09; N, 11.49.

### EXAMPLE 18

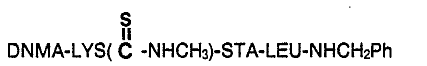

A suspension of 1.5 g (1.8 mmole) of DNMA-LYS-STA-LEU-NHCH₂Ph in 20 ml of CHCl₃ was treated with a small amount of DMF to effect solution, and then treated with 90 mg (1.23 mmole) of methyl isothiocyanate and 0.25 ml (1.8 mmole) of Et₃N and stirred at room temperature overnight. The solvent was removed under reduced pressure and the residue chromatographed on silica gel, eluting with CHCI₃. There was obtained 1.2 g of product. The structure was confirmed by NMR and mass spectroscopy.

Calcd. for C₅₃H₆₈N₆O₅S•0.4CHCl₃ (MW 948.88): C, 67.59; H, 7.27; N, 8.86 Found C, 67.37; H, 7.39; N, 8.94.

### EXAMPLE 19

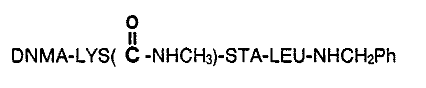

A solution of 1.0 g (1.2 mmole) of DNMA-LYS-STA-LEU-NHCHzPh in 20 ml DMF was treated with 0.08 ml (1.4 mmole) of methyl isocyanate and 0.2 ml (1.4 mmole) of Et₃N and stirred at room temperature for two days. The solvent was removed under high vacuum and the residue chromatographed on silica gel, eluting with CHCl₃/EtOAc (9/1). Since the product was contaminated with a little DMF, it was taken up in EtOAc and washed with H₂0. Drying and removal of the solvent under reduced pressure gave 0.9 g of the product. The structure was confirmed by NMR and mass spectroscopy.

Calcd. for C₅₃H₆₈N₆O₆•1.3H₂O (MW 908.54): C, 70.06; H, 7.83; N, 9.25 Found C, 70.08; H, 7.70; N, 9.37.

### EXAMPLE 20

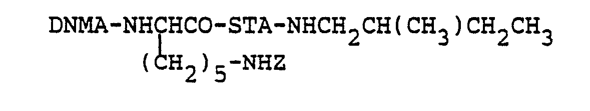

A solution of 2.1 g (3.4 mmole) of 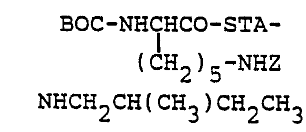 in 20 ml CH₂CI₂ was treated with 3 ml TFA and stirred for three hours at room temperature. The solvent was removed under reduced pressure and the residue mixed with sodium carbonate solution and extracted with EtOAc. The EtOAc was washed with saturated NaCI, dried, and the solvent removed under reduced pressure leaving the intermediate

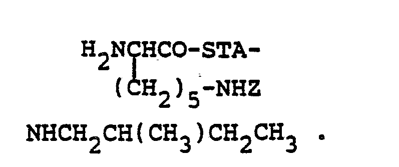

This was taken up in 15 ml DMF and treated with 1.15 g (3.4 mmole) of di-(1-naphthytmethyl)acetic acid and 0.46 g (3.4 mmole) of HOBT. The solution was cooled in ice and 0.7 g (3.4 mmole) of DCC added and the solution allowed to stir at room temperature overnight. The mixture was filtered and the filtrate diluted with EtOAc and washed with H₂0, saturated NaHC0₃, and saturated NaCI. Drying and removal of the solvent under reduced pressure gave the crude product which was chromatographed on silica gel, eluting with hexane/EtOAc (3/1), then hexane/EtOAc (1/1). There was obtained 1.6 g of product. The structure was confirmed by NMR and mass spectroscopy.

Calcd. for C₅₂H₆₆N₄O₆•0.5H₂O (MW 852.09): C, 73.29; H, 7.93; N, 6.58 Found C, 72.92; H, 7.65; N, 6.40.

### EXAMPLE 21

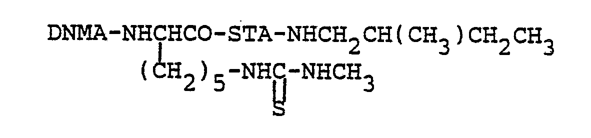

A solution of 0.3 g (0.4 mmole) of 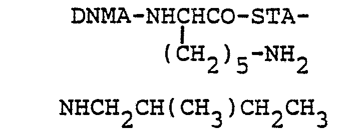 in 10 ml CHCl₃ was treated with 34 mg (0.46 mmole) of methyl isothiocyanate and stirred at room temperature overnight. The solvent was removed under reduced pressure and the crude product chromatographed on silica gel, eluting with EtOAc. There was obtained 0.3 g of product. The product was transferred to a vial with the aid of CH₂CI₂. The structure was confirmed by NMR and mass spectroscopy.

Calcd. for C₄₆H₆₃N₅O₄S•0.3CH₂Cl₂ (MW 807.49): C, 68.86; H, 7.94; N, 8.67 Found C, 68.73; H, 8.03; N, 8.46.

### EXAMPLE 22

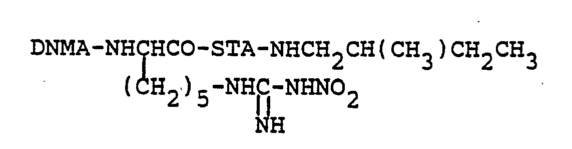

A solution of 0.42 g (0.6 mmole) of

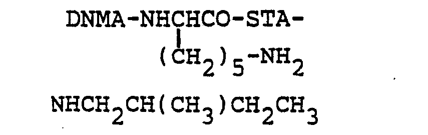

in 15 ml EtOH was treated with 0.1 g (0.7 mmole) of 2-methyl-1-nitro-2-thiopseudourea and stirred at room temperature overnight. The solvent was then removed under reduced pressure and the residue chromatographed on silica gel, eluting with hexane/EtOAc (1/1). The material was transferred to a vial with the aid of CH₂Cl₂ giving 0.4 g of product. The structure was confirmed by NMR and mass spectroscopy.

Caicd. for C₄₅H₆₁N₇O₆•0.3CH₂Cl₂ (MW 821.48): C, 66.23; H, 7.56; N, 11.94 Found C, 66.07; H, 7.73; N, 11.86.

### EXAMPLE 23

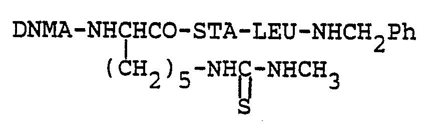

A solution of 0.5 g (0.6 mmole) of

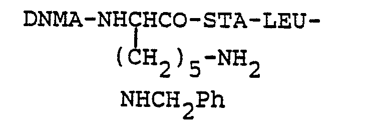

in 15 ml of CH₂CI₂ (3/1) was cooled to 0° and 44 mg (0.6 mmole) of methyl isothiocyanate added together with a few drops of Et₃N. The solution was allowed to warm to room temperature and was stirred overnight. The solvent was removed under reduced pressure and the residue chromatographed on silica gel, eluting with EtOAc. The material was transferred to a vial with the aid of CH₂Cl₂. There was obtained 0.5 g of product. The structure was confirmed by NMR and mass spectroscopy.

Calcd. for C₅₄H₇₀N₆O₅S•0.33C₄H₈O₂•0.1 CH₂Cl₂ (MW 952.72): C, 69.86; H, 7.71; N, 8.82 Found C, 69.59; H, 7.54; N, 9.03.

### EXAMPLE 24

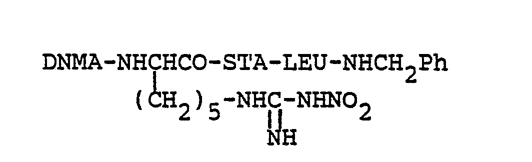

4 solution of 0.5 g (0.6 mmole) of

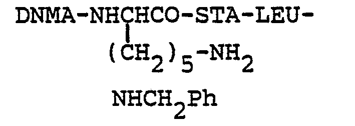

in 10 ml EtOH was treated with 0.13 g (1.0 mmole) of 2-methyl-1-nitro-2-thiopseudourea and stirred at room temperature overnight. The solvent was removed under reduced pressure and the residue chromatographed on silica get, eluting with EtOAc/hexane (1/1), then EtOAc. The material was transferred to a vial with the aid of CH₂Cl₂ to give 0.4 g of product. The structure was confirmed by NMR and mass spectroscopy. Calcd. for C₅₃H₆₈N₈O₇•0.33C₄H₈O₂•0.33CH₂Cl₂ (MW 986.12):

C, 66.55; H, 7.29; N, 11.36 Found

C, 66.26; H, 7.22; N, 11.58.

### EXAMPLE 25

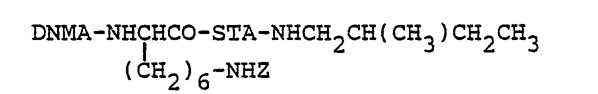

A solution of 1.3 g (2.0 mmole) of 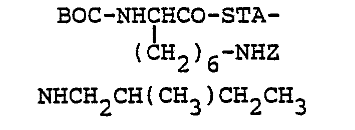 in 20 ml CH₂CI₂ was treated with 3 ml TFA and allowed to stir for three hours. The solvent was removed under reduced pressure, mixed with sodium carbonate solution, and extracted with EtOAc. The EtOAc was washed with saturated NaCl, dried, and the solvent removed under reduced pressure to give

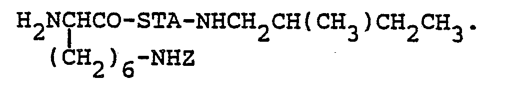

This was dissolved in 15 ml DMF and 0.7 g (2.1 mmole) of di-(1-naphthylmethyl)acetic acid and 0.28 (2.1 mmole) of HOBT added. The solution was cooled in ice and 0.42 g (2.0 mmole) of DCC added. The solution was stirred at room temperature overnight. The mixture was filtered and the filtrate diluted with EtOAc. The EtOAc was washed with H₂0, saturated NaHC0₃, and saturated NaCI. Drying and removal of the solvent under reduced pressure gave the crude product which was chromatographed on silica gel, eluting with hexane/EtOAc (3/1), then hexane/EtOAc (1/1). There was obtained 1.4 g of product The structure was confirmed by NMR and mass spectroscopy. Calcd. for C₅₃H₆₈N₄O₆•0.14C₄H₈O₂ (MW 869.44):
C, 73.99; H, 8.01; N, 6.44 Found
C, 73.29; H, 8.08; N, 6.42.

### EXAMPLE 26

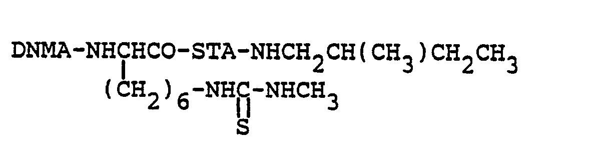

A solution of 0.42 g (0.6 mmole) of 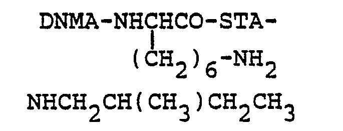 in 10 ml CHCI₃ was cooled in ice and treated with 48 mg (0.66 mmole) of methyl isothiocyanate, and the solution then stirred at room temperature overnight. The solvent was removed under reduced pressure and the residue chromatographed on silica gel, eluting with EtOAc. There was obtained 0.34 g of product. The structure was confirmed by NMR and mass spectroscopy. Calcd. for C₄₇H₆₅N₅O₄S•0.33C₄H₈O₂ (MW 825.10):

C, 70.33; H, 8.26; N, 8.49 Found

C, 69.94; H, 8.24; N, 8.56.

### EXAMPLE 27

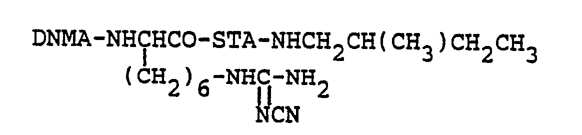

A solution of 0.67 g (0.9 mmole) of

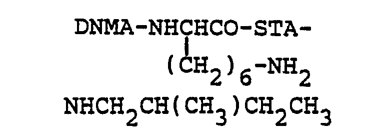

in 10 ml CHCI₃ was treated with 0.2 g (0.8 mmole) of diphenyl cyanocarboimidate and allowed to stir at room temperature overnight. The solvent was evaporated under reduced pressure and the residue taken up in 20 ml MeOH and ammonia bubbled through until saturated. The solution was allowed to stir at room temperature overnight, the solvent removed under reduced pressure, and the residue chromatographed on silica gel, eluting with EtOAc/MeOH (9/1). There was obtained 0.6 g of product. The structure was confirmed by NMR and mass spectroscopy.

Calcd. for C₄₇H₆₃N₇O₄•0.75CH₃OH (MW 814.06): C, 70.45; H, 8.17; N, 12.05 Found C, 70.34; H, 8.31; N, 12.10.

### EXAMPLE 28

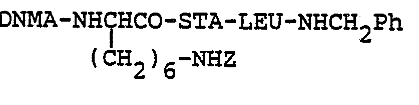

A solution of 1.0 g (1.3 mmole) of

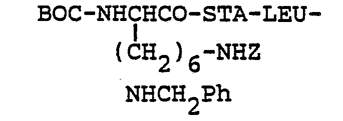

in 20 ml of CH₂CI₂ was treated with 4 ml of TFA and allowed to stir at room temperature for two hours. The solvent was removed under reduced pressure and the residue taken up in EtOAc and washed with saturated NaHC0₃ and saturated NaCI. Drying and removal of the solvent under reduced pressure gave

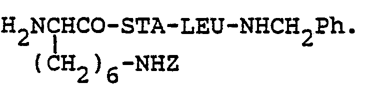

This was taken up in 15 ml DMF and 0.44 g (1.3 mmole) of di-(1-naphthylmethyl)acetic acid and 0.18 g (1.3 mmole) of HOBT added. The solution was cooled in ice and 0.27 g (1.3 mmole) DCC was added and the solution allowed to stir at room temperature for three days. The mixture was filtered and the filtrate diluted with EtOAc and washed with H₂0, saturated NaHC0₃, and saturated NaCI. Drying and removal of the solvent under reduced pressure gave the crude product which was chromatographed on silica gel, eluting with EtOAc/hexane (1/1). There was obtained 1.0 g of product. The structure was confirmed by NMR and mass spectroscopy.

Calcd. for C₆₁H₇₅N₅O₇ (MW 990.25): C, 73.98; H, 7.63; N, 7.07 Found

C, 73.67; H, 7.87; N, 7.24.

### EXAMPLE 29

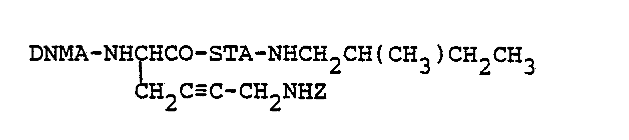

A solution of 1.3 g (2.4 mmole) of 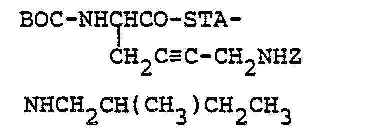 in 30 ml CH₂Cl₂ was treated with 4 ml of TFA and allowed to stir at room temperature for two hours. The solvent was removed under reduced pressure and the residue mixed with sodium carbonate solution and extracted with EtOAc. The EtOAc was washed with H₂0 and saturated NaCI. Drying and removal of the solvent under reduced pressure gave

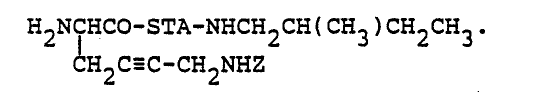

This was taken up in 15 ml DMF and 0.81 g (2.4 mmole) of di-(1-naphthylmethyl)acetic acid and 0.32 g (2.4 mmole) of HOBT added. The solution was cooled in ice and 0.49 g (2.4 mmole) of DCC added and the solution stirred at room temperature overnight. The mixture was filtered and the filtrate diluted with EtOAc and washed with H₂0, saturated NaHCO₃, and saturated NaCI. Drying and removal of the solvent under reduced pressure gave the crude product which was chromatographed on silica gel, eluting with EtOAc/hexane (1/1). There was obtained 0.2 g of product. The structure was confirmed by NMR and mass spectroscopy.

Calcd. for C₅₁H₆₀N₄O₆•0.33C₄H₈O₂ (MW 854.10): C, 73.57; H, 7.39; N, 6.56 Found C, 73.04; H, 7.36; N, 6.71.

### EXAMPLE 30

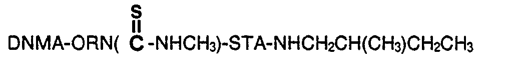

A solution of 0.47 g (0.7 mmole) of DNMA-ORN-STA-NHCH₂CH(CH₃)CH₂CH₃ in 30 ml CH₂CI₂ was cooled to 0° and 51 mg (0.7 mmole) of methyl isothiocyanate added, and the solution allowed to stir at room temperature overnight. The solvent was then removed under reduced pressure and the residue chromatographed on silica gel, eluting with EtOAc. The material was transferred to a vial with the aid of CH₂CI₂. There was obtained 0.3 g of product. The structure was confirmed by NMR and mass spectroscopy. Calcd. for C₄₄H₅₉N₅O₄S•0.33C₄H₈O₂•0.25CH₂Cl₂ (MW 804.26):

C, 68.05; H, 7.79; N, 8.71 Found C, 67.71; H, 7.80; N, 8.74.

### EXAMPLE 31

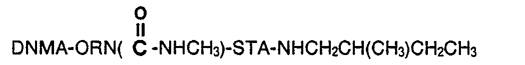

A solution of 0.47 g (0.7 mmole) of DNMA-ORN-STA-NHCH₂CH(CH₃)CH₂CH₃ in 30 ml CH₂Cl₂ was cooled in ice and 0.041 ml (0.7 mmole) of methyl isocyanate added and the solution allowed to stir at room temperature overnight. The solvent was removed under reduced pressure and the residue chromatographed on silica gel, eluting with EtOAc/MeOH (9/1). There was obtained 0.3 g of product. The structure was confirmed by NMR and mass spectroscopy.

Calcd. for C₄₄H₅₉N₅O₅•CH₃OH (MW 769.99): C, 70.19; H, 8.25; N, 9.10 Found C, 69.79; H, 8.06; N, 9.44.

### EXAMPLE 32

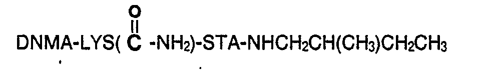

A solution of 0.3 g (0.4 mmole) of DNMA-LYS-STA-NHCH₂CH(CH₃)CH₂CH₃ in 15 ml dioxane was treated with 0.03 ml of HOAc followed by 30 mg (0.46 mmole) of NaOCN in 1 ml H₂0. The solution was warmed to 45° for four hours, cooled, and partitioned between EtOAc and saturated NaHCOs. The EtOAc was washed with saturated NaCl, dried, and the solvent removed under reduced pressure giving the crude product which was chromatographed on silica gel, eluting with EtOAc, then EtOAc/MeOH (9/1). There was obtained 0.2 g of product, mp 134-137°. The structure was confirmed by NMR and mass spectroscopy.

Calcd. for C₄₄H₅₉N₅O₅•CH₃OH (MW 769.99): C, 70.19; H, 8.25; N, 9.10 Found C, 70.27; H, 8.23; N, 9.15.

### EXAMPLE 33

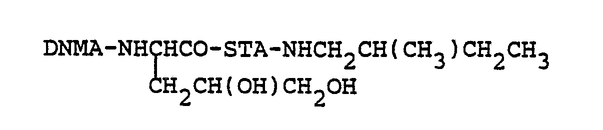

A solution of 350 mg (0.53 mmole) of 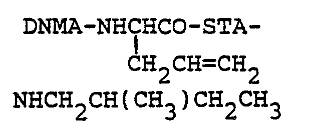 in 10 ml dioxane was treated with 8.1 ml (0.63 mmole) of a 2% solution of osmium tetroxide in dioxane. The solution was allowed to stir at room temperature for three days, then treated with hydrogen sulfide gas. The mixture was filtered and the solvent removed under reduced pressure. The residue was chromatographed on silica gel, eluting with CHCl₃/MeOH (97/3). The material was taken up in CHCl₃ and precipitated with hexane. There was obtained 131 mg of the product as a white solid. The structure was confirmed by NMR and mass spectroscopy.

Calcd. for C₄₂H₅₅N₃O₆•0.8H₂O (MW 712.30): C, 70.82; H, 8.01; N, 5.90 Found C, 70.91; H, 8.12; N, 5.81.

### EXAMPLE 34

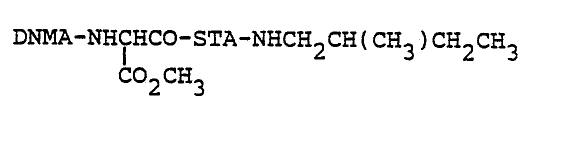

A solution of 1.80 g (5.29 mmole) di-(1-naphthylmethyl) acetic acid, 1.90 g (5.29 mmole) of 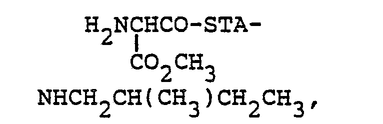 0.75 g (5.55 mmole) of HOBT, and 1.14 g (5.55 mmole) of DCC in 50 ml DMF was stirred at 25° for six hours. The mixture was then filtered, and the solvent removed under high vacuum. The residue was taken up in EtOAc and washed with 1 N citric acid, saturated NaCI, saturated NaHCOs, and saturated NaCI. After drying, the solvent was evaporated under reduced pressure to a glass, 3.98 g. The product was chromatographed on silica gel, eluting with EtOAc. Combination of the appropriate fractions gave a solid, 2.18 g. The structure was confirmed by NMR, IR, and mass spectroscopy. Calcd. for C₄₁H₅₁N₃O₆•0.33H₂O (MW 687.88): C, 71.59; H, 7.57; N, 6.11; H₂0, 0.87 Found C, 71.95; H, 7.61; N, 6.19; H₂0, 1.00.

### EXAMPLE 35

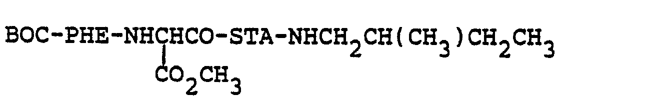

A solution of 4.37 g (16.5 mmole) of BOC-PHE, 5.92 g (16.5 mmole) of 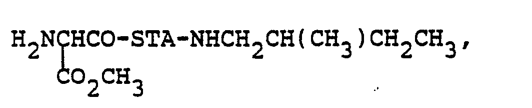 and 2.29 g (17.0 mmole) of HOBT in 150 ml DMF was cooled to 0°. 3.50 g (17 mmole) of DCC was added, and the mixture was stirred at 4° overnight. The mixture was filtered, and the solvent was removed under high vacuum. The residue was taken up in EtOAc and washed with 1 N citric acid, saturated NaCl, saturated NaHC0₃, and saturated NaCI. After drying, the solution was evaporated under reduced pressure to an oil, which was chromatographed on silica gel, eluting with EtOAc. Combination of the appropriate fractions gave a white solid, 4.88 g. The structure was confirmed by NMR and mass spectroscopy.

Calcd. for C₃₁H₅₀N₄O₈ (MW 606.77): C, 61.36; H, 8.31; N, 9.23 Found C, 60.95; H, 8.23; N, 9.16.

### EXAMPLE 36

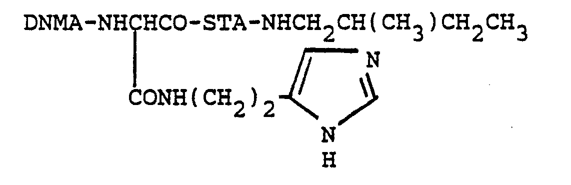

A solution of 0.96 g (1.44 mmole) of 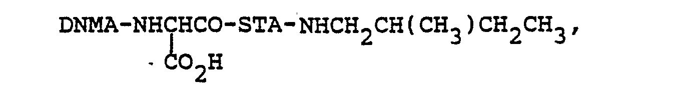 0.2 g (1.48 mmole) of HOBT, and 0.30 g (1.48 mmole) of DCC in 20 ml DMF was cooled to 0°. A solution of 0.27 g (1.48 mmole) of histamine•2HCl and 0.32 g (2.97 mmole) of Et₃N in 10 ml DMF was added. After stirring overnight at 4°, the mixture was filtered, and the solvent removed under high vacuum. The residue was taken up in EtOAc, filtered, and washed with 1 N citric acid, saturated NaCI, saturated NaHCOs, and saturated NaCI. After drying, the solvent was removed under reduced pressure and the residue was chromatographed on silica gel, eluting with a gradient of 2 to 10% MeOH in CHCl₃. Combination of the appropriate fractions gave the product as a foam, 0.89 g. The structure was confirmed by NMR and mass spectroscopy.

Calcd. for C₄₅H₅₆N₆O₅•0.25CHCl₃ (MW 790.83): C, 68.72; H, 7.17; N, 10.62; Cl, 3.63 Found C, 68.39; H, 7.19; N, 10.60; CI, 3.13

### EXAMPLE 37

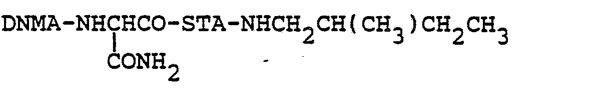

4 solution of 0.6 g (0.88 mmole) of 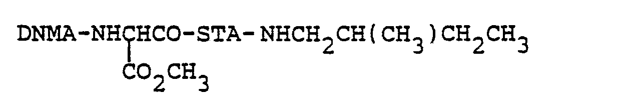 in 20 ml MeOH was cooled to -30° and saturated with NH₃ gas. The mixture was allowed to warm to room temperature over three hours, and the solvent was removed under reduced pressure. The residue was suspended in EtzO, filtered, and the solvent evaporated. The residue was chromatographed on silica gel, eluting with CHCl₃/MeOH (98/2). The appropriate fractions were combined to give a solid, 0.35 g. The structure was confirmed by NMR and mass spectroscopy.

Calcd. for C₄₀H₅₀N₄O₅•0.125CHCl₃ (MW 681.79): C, 70.69; H, 7.41; N, 8.22; Cl, 1.95 Found C, 70.75; H, 7.66; N, 8.27; Cl, 1.92.

### EXAMPLE 38

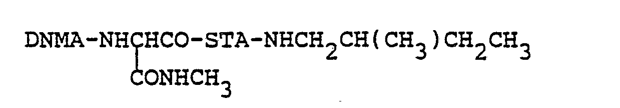

A solution of 0.6 g (0.88 mmole) of 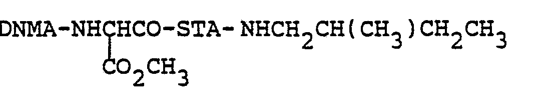 in 5 ml MeOH was cooled to -30° and saturated with liquid CH₃NH₂. After one hour, the solvent was removed in vacuo and the residue was chromatographed on silica gel, eluting with CHCl₃/MeOH (98/2). The appropriate fractions were combined to give a white foam, 0.50 g. The structure was confirmed by NMR and mass spectroscopy.

Calcd. for C₄₁H₅₂N₄O₅•0.08CHCl₃ (MW 690.44): C, 71.46; H, 7.60; N, 8.11; Cl, 1.23 Found C, 71.09; H, 7.70; N, 8.15; Cl, 1.10.

### EXAMPLE 39

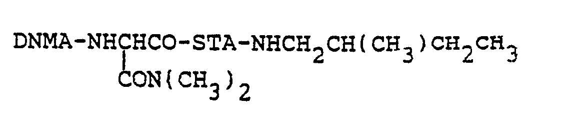

A solution of 0.92 g (1.35 mmole) of 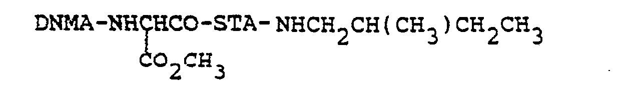 in 10 ml MeOH was saturated with liquid dimethylamine. After stirring for three hours, the solvent was removed under reduced pressure. The residue was chromatographed on silica gel, eluting with EtOAc. The appropriate fractions were combined to a solid, 0.41 g. The structure was confirmed by NMR and mass spectroscopy.

Calcd. for C₄₂H₅₄N₄O₅•0.6H₂O (MW 705.73): C, 71.48; H, 7.88; N, 7.94; H₂0, 1.53 Found C, 71.17; H, 7.78; N, 7.73; H₂0, 1.18.

### EXAMPLE 40

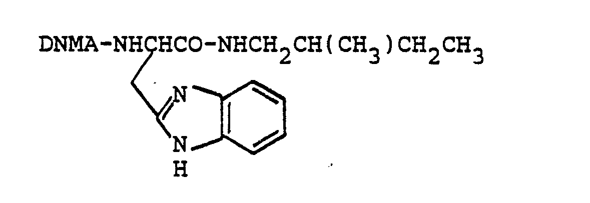

A solution of 0.55 g (1.62 mmole) of di-(1-naphthylmethyl) acetic acid, 0.69 g (1.62 mmole) of 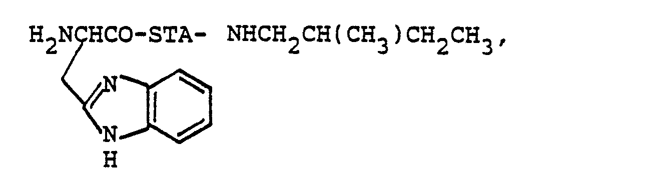 and 0.23 g (1.7 mmole) of HOBT in 20 ml DMF was treated with 0.35 g (1.7 mmole) of DCC and stirred at room temperature overnight. The mixture was filtered and the DMF removed under high vacuum. The residue was taken up in EtOAc and washed with 1 N citric acid, saturated NaCI, saturated NaHC0₃, and saturated NaCI. Drying and removal of the solvent under reduced pressure gave the crude product which was chromatographed on silica gel, eluting with EtOAc. Combining the appropriate fractions gave material which was taken up in CH₂Cl₂ and precipitated by addition to Et₂O. The solid was collected to give 0.28 g of product. The structure was confirmed by NMR and mass spectroscopy.

Calcd. for C₄₇H₅₅N₅O₄ (MW 753.95): C, 74.87; H, 7.35; N, 9.29 Found C, 74.47; H, 7.35; N, 9.01.

### EXAMPLE 41

### DNMA-LYS(TOS)-STA-NHCH₂CH(CH₃)CH₂CH₃

A solution of 0.44 g (1.3 mmole) of di-(1-naphthylmethyl) acetic acid, 0.63 g (1.22 mmole) of LYS(TOS)-STA-NHCH₂CH(CH₃)CH₂CH₃, and 0.185 g (1.37 mmole) of HOBT in 35 ml of DMF was treated with 0.28 g (1.37 mmole) of DCC and the solution stirred at room temperature overnight. The mixture was filtered, and the solvent removed under high vacuum. The residue was taken up in EtOAc and washed with 1 N citric acid, saturated NaCl, saturated NaHC0₃, and saturated NaCI. After drying, the solution was evaporated under reduced pressure and the residue chromatographed on silica gel, eluting with a gradient of 0 to 5% MeOH in CHCI₃. Combination of the appropriate fractions gave the product as a white foam, 0.99 g. The structure was confirmed by NMR and mass spectroscopy.

Calcd. for C₅₀H₆₄N₄O₆S•0.07CHCl₃ (MW 857.51): C, 70.13; H, 7.53; N, 6.53; CI, 0.87 Found C, 69.78; H, 7.49; N, 6.40; CI, 0.90.

### EXAMPLE 42

### DNMA-LYS(CO₂CH₃)-STA-NHCH₂CH(CH₃)CH₂CH₃

A solution of 0.124 g (0.7 mmole) of N-methyloxycarbonylsuccinimide and 0.50 g (0.72 mmole) of DNMA-LYS-STA-NHCH₂CH(CH₃)CH₂CH₃ in 2 ml DMF was stirred overnight at room temperature. The solvent was removed under high vacuum and the residue was taken up in EtOAc. The solution was washed with 1 N citric acid, saturated NaCI, saturated NaHC0₃, and saturated NaCI. After drying, the solvent was removed under reduced pressure, and the residue triturated with Et₂O, giving the crude product as a solid. This was chromatographed on silica gel, eluting with a gradient of 0 to 1% MeOH in CHCI₃. The crude product was taken up in CHCl₃ and added to excess Et₂O giving a solid which was collected and dried. There was obtained 0.24 g of the product as a white solid. The structure was confirmed by NMR and mass spectroscopy.

Calcd. for C₄₅H₆₀N₄O₆ (MW 753.00): C, 71.77; H, 8.03; N, 7.44 Found C, 71.57; H, 8.07; N, 7.44.

### EXAMPLE 43

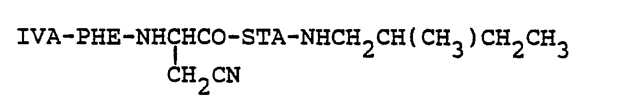

A solution of 0.92 g (3.7 mmole) of IVA-PHE and 0.53 g (3.89 mmole) of HOBT in 4 ml DMF and 30 ml CH₂CI₂ was cooled to -5° and 0.8 g (3.89 mmole) of DCC added followed by 1.26 g (3.7 mmole) of

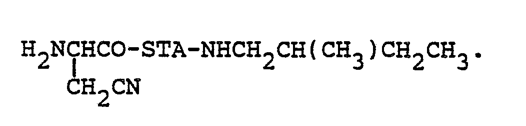

The solution was stirred at room temperature overnight, filtered, and the solvent removed under reduced pressure. The residue was taken up in EtOAc and washed with 1 N citric acid, saturated NaCI, saturated NaHC0₃, and saturated NaCI. The solution was dried, the volume reduced under reduced pressure, and the solution diluted with Et₂O. A precipitate formed which was collected giving the crude product. This was chromatographed on silica gel, eluting with CHCl₃/MeOH (98/2). The product was taken up in CHCl₃ and precipitated by the addition of Et₂O. There was obtained 1.04 g of product. The structure was confirmed by NMR and mass spectroscopy.

Calcd. for C₃₁H₄₉N₅O₅ (MW 571.74): C, 65.12; H, 8.64; N, 12.25 Found

C, 65.00; H, 8.40; N, 12.21.

### EXAMPLE 44

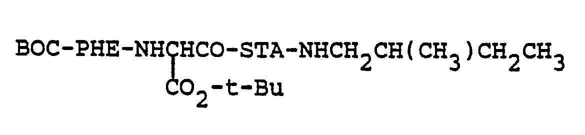

To a solution of 2.11 g (5 mmole) of 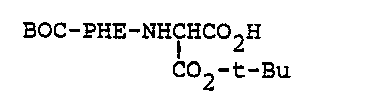 and 1.12 g (5.0 mmole) of STA-NHCH₂CH(CH₃)CH₂CH₃ in 155 ml CH₂Cl₂ was added a solution of 0.69 g (5.1 mmole) of HOBT in 8 ml DMF. The mixture was cooled to 0° and 1.05 g (5.1 mmole) of DCC was added. After warming to 25° overnight, the mixture was filtered and the solvent removed under reduced pressure. The residue was taken up in EtOAc and washed with 1 N citric acid, saturated NaCI, saturated NaHC0₃, and saturated NaCl. Drying and removal of the solvent under reduced pressure gave the crude product which was chromatographed on silica gel, eluting with CHCl₃/EtOAc (50/50). Combining the appropriate fractions gave 3.34 g of the product as a white foam. The structure was confirmed by NMR and mass spectroscopy. Calcd. for C₃₄H₅₆N₄O₈•0.6H₂O (MW 659.65):

C, 61.91; H, 8.74; N, 8.49; H₂0, 1.64 Found C, 61.74; H, 8.56; N, 8.54; H₂0, 1.82.

### EXAMPLE 45

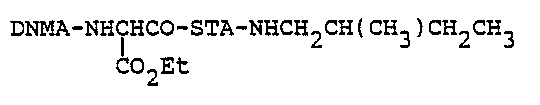

To a solution of 2.29 g (4.88 mmole) of 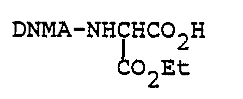 and 1.09 g (4.88 mmole) of STA-NHCH₂CH(CH₃)CH₂CH₃ in 50 ml CH₂C1₂ was added a solution of 0.68 g (5.02 mmole) of HOBT in 3 ml DMF. After cooling to 0°, 1.04 g (5.02 mmole) of DCC was added, and the mixture was stirred at room temperature overnight. The mixture was filtered, and the solvent removed under reduced pressure. The residue was taken up in Et₂O and washed with 1 N citric acid, saturated NaCl, saturated NaHC0₃, and saturated NaCI. After drying, the solvent was removed under reduced pressure and the residue was chromatographed on silica gel, eluting with EtOAc/hexane (50/50). Combination of the appropriate fractions gave a solid, 2.33 g. The structure was confirmed by NMR and mass spectroscopy. Calcd. for C₄₂H₅₃N₃O₆•0.4H₂O (MW 703.11): C, 71.75; H, 7.71; N, 5.98; H₂O, 1.02 Found C, 71.65; H, 7.63; N, 5.64; H₂0, 0.93.

### EXAMPLE 46

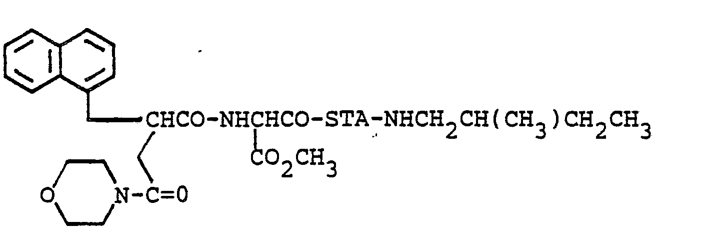

To a solution of 1.08 g (3.3 mmole) of 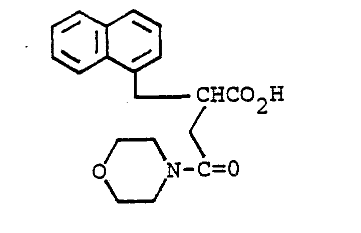 and 1.2 g (3.3 mmole) of 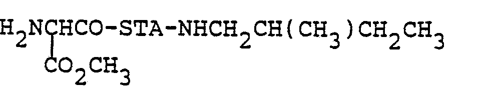 in 65 ml CH₂CI₂ was added a solution of 0.46 g (3.4 mmole) of HOBT in 8 ml DMF. The mixture was cooled to 0° and 0.7 g (3.4 mmole) of DCC was added. After stirring overnight at room temperature, the mixture was filtered, and the solvent removed under reduced pressure. The residue was taken up in EtOAc and washed with 1 N citric acid, saturated NaCl, saturated NaHC0₃, and saturated NaCl. After drying, the solvent was removed under reduced pressure and the residue was chromatographed on silica gel, eluting with EtOAc/CHCl₃/MeOH (45/45/10). Combination of the appropriate fractions gave foam, 1.18 g. The structure was confirmed by NMR and mass spectroscopy.

Calcd. for C₃₆H₅₂NaO₈•1.0H₂O•0.0625CHCl₃ (MW 695.28): C, 62.30; H, 7.85; N, 8.07; Cl, 0.96; H₂0, 2.59 Found C, 62.64; H, 7.86; N, 8.16; Cl, 1.03; H₂0, 2.76.

### EXAMPLE 47

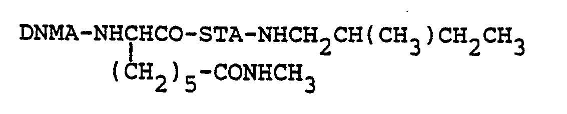

A solution of 0.4 g (1.0 mmole) of 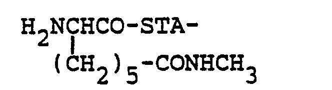 NHCH₂CH(CH₃)CH₂CH₃ and 0.32 g (1.0 mmole) of di-(1-naphthylmethyl)acetic acid in 20 ml of CH₂CI₂ was cooled in ice and 203 mg (2.0 mmole) of Et₃N added, followed by 280 mg (1.1 mmole) of bis(2-oxo-3-oxazolidinyl)phosphinic chloride. After stirring at room temperature overnight, the mixture was washed with 1 N HCI, saturated NaHC0₃, and saturated NaCI. Drying and removal of the solvent under reduced pressure gave the crude product which was chromatographed on silica gel, eluting with EtOAc, then EtOAc/EtOH (1/1). There was obtained 0.46 g of product, mp 127-131 °.

### EXAMPLE 48

### DNMA-NHCH(CH₂CH₂CH₂CH=CH₂)CO-STA-LEU-NHCH₂Ph (ISOMER A)

To a solution of 4.9 g (10.4 mmole) of H₂NCH(CH₂CH₂CH₂CH=CH₂)CO-STA-LEU-NHCH₂Ph and 3.54 g (10.4 mmole) of di-(1-naphthylmethyl)acetic acid in 100 ml DMF was added 1.47 g (10.9 mmole) of HOBT followed by 2.25 g (10.9 mmole) of DCC. The mixture was stirred for fifteen hours at room temperature and the urea filtered off. The DMF was removed under reduced pressure and the residue taken up in EtOAc. The organic solution was washed with saturated NaHC0₃, dried, and concentrated under reduced pressure to afford the crude product. Chromatography on silica gel, eluting with a gradient of 0-2% MeOH in CHCl₃ gave 1.8 g of the desired product. The structure was confirmed by NMR spectroscopy.

Calcd. for C₅₂H₆₄N₄O₅•0.25CHCl₃ (MW 854.91): C, 73.40; H, 7.58; N, 6.55 Found C, 73.09; H, 7.72; N, 6.79.

### EXAMPLE 49

### DNMA-NHCH(CH2CH2CH2CH=CH2)CO-STA-LEU-NHCH2Ph (ISOMER B)

Continued elution from the chromatography column described in Example 48 gave 2.2 g of Isomer B, epimeric at the alpha carbon of the 5-pentenyl amino acid. The structure was confirmed by NMR spectroscopy.

Calcd. for C₅₂H₆₄N₄O₅ (MW 825.06): C, 75.69; H, 7.82; N, 6.79 Found C, 75.52; H, 8.04; N, 6.75.

### EXAMPLE 50

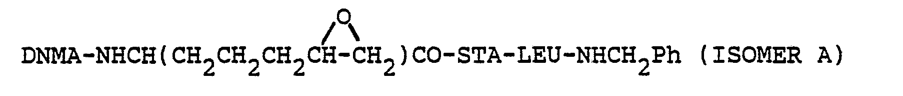

To a solution of 1.55 g (1.88 mmole) of DNMA-NHCH(CH₂CH₂CH₂CH=CH₂)CO-STA-LEU-NHCH₂Ph (Isomer A) in 20 ml of CH₂CI₂ was added 0.486 g (2.82 mmole) of m-chloroperbenzoic acid. The solution was stirred for twelve hours, diluted with EtOAc, washed with saturated NaHC0₃, dried, and concentrated under reduced pressure to give the crude product. Chromatography on silica gel, eluting with a gradient of 0-2% MeOH in CHCl₃ gave the pure product. The structure was confirmed by NMR spectroscopy.

Calcd. for C₅₂H₆₄N₄O₆ (MW 841.06): C, 74.25; H, 7.67; N, 6.66 Found C, 74.07; H, 7.78; N, 6.45.

### EXAMPLE 51

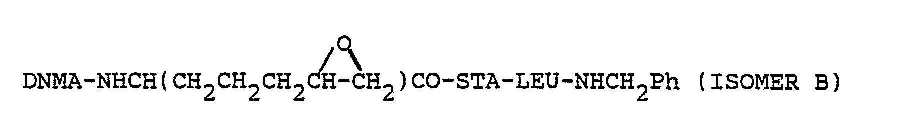

In a manner similar to Example 50, 2.2 g (2.67 mmole) of DNMA-NHCH(CH₂CH₂CH₂CH=CH₂)CO-STA-LEU-NHCH₂Ph (Isomer B) gave 1.5 g of product. The structure was confirmed by NMR spectroscopy. Calcd. for C₅₂H₆₄N₄O₆ (MW 841.06): C, 74.25; H, 7.67; N, 6.66 Found C, 74.21; H, 7.57; N, 6.62.

### EXAMPLE 52

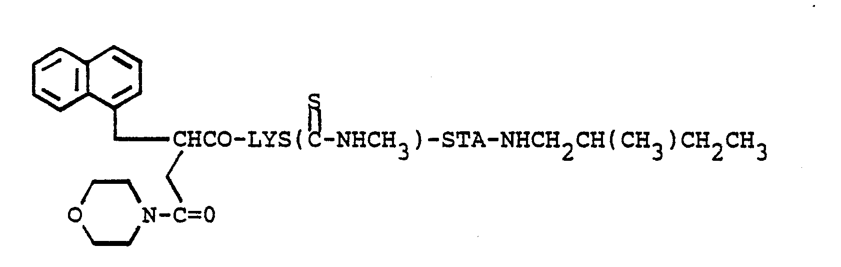

Following the procedure of Example 2, but using 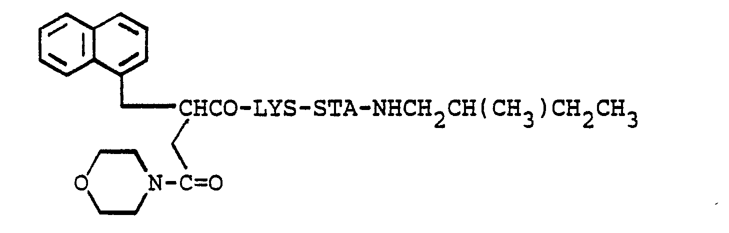 gives the title compound.

### EXAMPLE 53

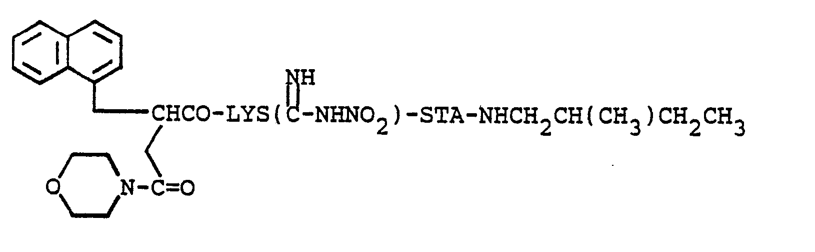

Following the procedure of Example 9, but using 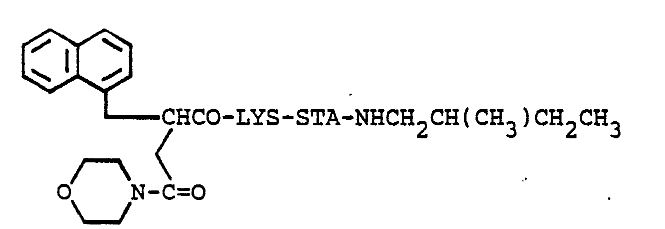 gives the title compound.

### EXAMPLE 54

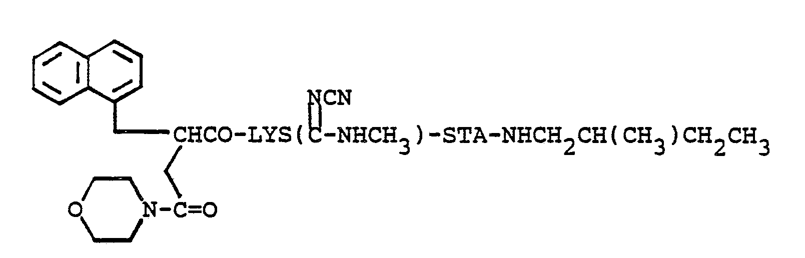

Followinq the procedure of Example 13, but using 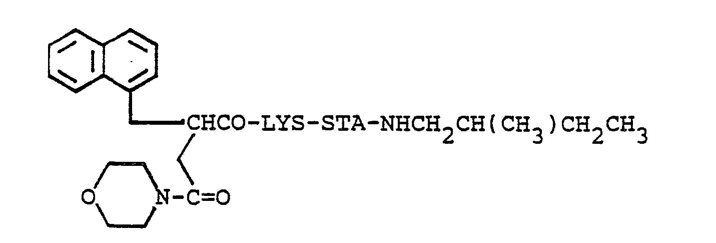 gives the title compound.

### EXAMPLE 55

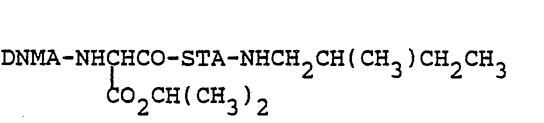

Following the procedure of Example 45, but substituting 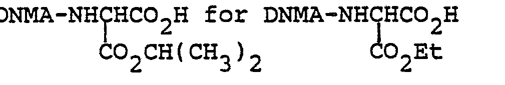 gave the title compound as a solid foam. The structure was confirmed by NMR and mass spectroscopy.

Calcd. for C₄₃H₅₅N₃O₆•0.25H₂O•0.03CHCl₃ (MW 718.02): C, 71.98; H, 7.80; N, 5.85; H₂0, 0.63; CI, 0.44 Found C, 72.10; H, 7.66; N, 5.66; H₂0, 0.69; Cl, 0.57.

### INTERMEDIATES FOR EXAMPLE 1

### BOC-PHE-ARG(N0₂)-OCH₃

A solution of 3.7 g ARG(N0₂)-OCH₃eHCI in 50 ml DMF was cooled in ice and treated with 1.85 ml of Et₃N. To this was then added successively 1.85 g HOBT, 3.64 g BOC-PHE, and then 2.83 g DCC. The solution was kept at 0° for one hour, then at room temperature for five days. The solvent was removed under reduced pressure, and the residue taken up in EtOAc. Insolubles were filtered off, and the filtrate washed with Na₂CO₃ solution, citric acid solution, then brine. After drying over MgS0₄ and removal of the solvent under reduced pressure, the residue was chromatographed on silica gel, eluting with CH₂Cl₂/MeOH (97:3). The appropriate fractions were combined to give 4.79 g (72.7%) of the product as a white foam.

### BOC-PHE-ARG(NO₂)

A solution of 3.92 g of BOC-PHE-ARG(N0₂)-OCH₃ in 50 ml of MeOH was treated with 9 ml of 1 N NaOH solution and stirred at room temperature for one hour. The solvent was removed under reduced pressure, and the residue taken up in H₂0 and washed with EtOAc. The aqueous phase was acidified with citric acid and extracted with EtOAc. The EtOAc was washed with brine and dried over MgSO₄. After filtering, removal of the solvent under reduced pressure gave 3.66 g (96%) of the product as a white foam.

### INTERMEDIATES FOR EXAMPLES 2, 3, AND 5-13

### BOC-LYS(Z)-STA-NHCH₂CH(CH₃)CH₂CH₃

BOC-LYS(Z) (1.98 g), STA-NHCH₂CH(CH₃)CH₂CH₃*HCt (1.5 g), hydroxybenzotriazole (0.72 g), and triethyl amine (0.74 ml) were mixed together in dimethylformamide (15 ml) and cooled to 0°. Dicyclohexylcarbodiimide (1.1 g) was added, and the mixture was allowed to warm slowly to 25° and then stir for 72 hours. The mixture was filtered, and the filtrate was extracted with EtOAc and water. The organic phase was washed with water, sodium bicarbonate solution, and brine. The organic phase was dried over sodium sulfate, filtered, and evaporated. The residue was eluted from silica gel with 1:1 EtOAc/hexane to give 2.7 g of product.

### LYS(Z)-STA-NHCH₂CH(CH₃)CH₂CH₃

BOC-LYS(Z)-STA-NHCH₂CH(CH₃)CH₂CH₃ (2.7 g) was dissolved in dichloromethane (30 ml), and trifluoroacetic acid (4 ml) was added. The mixture was stirred for two hours at 25°. The solvent was evaporated, and the residue was extracted with EtOAc and sodium carbonate solution. The organic phase was washed with brine, dried over sodium sulfate, filtered, and evaporated to give 2.1 g of the product.

### DNMA-LYS(Z)-STA-NHCH₂CH(CH₃)CH₂CH₃

LYS(Z)-STA-NHCH₂CH(CH₃)CH₂CH₃ (2.1 g), di-(1-naphthylmethyl)acetic acid (1.51 g), and hydroxybenzotriazole (0.6 g) were dissolved in dimethylformamide (20 ml) and cooled to 0°. Dicyclohexylcarbodiimide (0.92 g) was added, and the mixture was allowed to warm slowly to 25° and stir for 24 hours. The mixture was filtered, and the filtrate was extracted with ethyl acetate and water. The organic phase was washed with water, sodium bicarbonate, and brine. The organic phase was dried over sodium sulfate, filtered, and evaporated. The crude produce was recrystallized from choroform to give 1.95 g of product. The residue from the mother liquor was purified by chromatography on silica gel, eluting with CHCb/EtOAc (3:1). combining the appropritate fractions gave an additional 0.5 g of product.

### DNMA-LYS-STA-NHCH₂CH(CH₃)CH₂CH₃

DNMA-LYS(Z)-STA-NHCH₂CH(CH₃)CH₂CH₃ (1.95 g) was dissolved in methanol (30 ml) and palladium on carbon (20%) (0.2 g) was added. The flask was flushed with hydrogen and stirred for six hours. The flask was flushed with nitrogen, and the mixture was filtered. The solvent was evaporated to give 1.6 g of product.

### INTERMEDIATES FOR EXAMPLES 4

### Z-ORN(PHT)-STA-LEU-NHCH₂Ph

Z-ORN(PHT) (French Pat. 1,430,140) (1.60 g, 4.03 mmole) and HOST•H₂O (0.544 g, 4.23 mmole) were dissolved in 10 ml DMF, diluted to 90 ml with CH₂CI₂ and cooled to -5°. DCC (0.87 g, 4.23 mmole) was charged as a solution in 20 ml of cooled CH₂C1₂ followed by a cooled suspension of STA-LEU-NHCH₂Ph.HCI (1.67 g, 4.03 mmole) in a mixture of 40 ml CH₂Cl₂ and Et₃N (0.56 ml, 4.23 mmole). After stirring 16 hours at 25°, the mixture was filtered, stripped, and resuspended in EtOAc. The suspension was washed with 1 N citric acid, brine, saturated NaHC0₃ solution, and brine. The suspension was stripped to a paste, triturated with H₂0, filtered, and dissolved in THF. The THF solution was dried over MgS0₄, filtered, and stripped to a solid, 3.14 g. Recrystallization from THF/Et₂O gave 2.8 g of the product as a solid. IR, NMR, and mass spectral analyses confirmed the structure.

### ORN(PHT)-STA-LEU-NHCH₂Ph

Z-ORN(PHT)-STA-LEU-NHCH₂Ph (2.59 g, 3.43 mmole) was dissolved in 60 ml HOAc. Twenty percent palladium on charcoal catalyst (0.10 g) was added, and the mixture was purged with hydrogen gas overnight. The mixture was filtered, stripped, and the residue dissolved in EtOAc. The solution was washed with 5% NaOH and then brine. The solution was dried over MgS0₄, filtered, and stripped to a foam, 2.03 g. The foam was chromatographed on silica gel eluting with a gradient of 0 to 5% MeOH in CHCl₃, giving a white foam, 1.52 g. NMR, IR, and mass spectral analyses confirmed the structure.

### INTERMEDIATES FOR EXAMPLE 8

### BOC-CYSTA-NHCH₂CH(CH₃)CH₂CH₃

A solution of 6.78 g (21.5 mmole) of BOC-CYSTA and 2.9 g (21.5 mmole) of HOBT in 100 ml DMF was cooled in ice and 4.48 g (21.5 mmole) of DCC in 10 ml DMF added, followed by 1.88 g (21.5 mmole) of S-(- )-1-amino-2-methylbutane. Keep at 0° for one-half hour, then let stir at room temperature overnight. The mixture was filtered and the solvent removed under high vacuum. The residue was taken up in EtOAc and washed with 1 N HCI, saturated NaHC0₃, and saturated NaCI. Drying and removal of the solvent under reduced pressure gave the crude product. Chromatography on silica gel, eluting with CHCl₃/MeOH (99/1) gave 8.2 g of the product as an oil. The structure was confirmed by mass spectroscopy.

### CYSTA-NHCH₂CH(CH₃)CH₂CH₃•HCl

A solution of 8.2 g (21.3 mmole) of BOC-CYSTA-NHCH₂CH(CH₃)CH₂CH₃ in 85 ml of CH₂Cl₂ was saturated with HCI gas, stirred for one hour, then resaturated with HCI gas and allowed to stir for an additional three hours. The solution was diluted with Et₂0 and the precipitated solid collected and dried. There was obtained 5.47 g of the product as a white solid. The structure was confirmed by mass spectroscopy.

### BOC-LYS(Z)-CYSTA-NHCH₂CH(CH₃)CH₂CH₃

A solution of 1.7 g (4.6 mmole) of BOC-LYS(Z), 1.5 g (4.7 mmole) of CYSTA-NHCH₂CH(CH₃)-CH₂CH₃•HCl, and 0.63 g (4.7 mmole) of HOBT in 20 ml DMF was cooled in ice and treated with 0.85 ml (6.1 mmole) of Et₃N followed by 0.96 g (4.7 mmole) of DCC. The mixture was allowed to stir at room temperature for two days. The mixture was filtered and the filtrate diluted with EtOAc. The organic phase was washed with H₂0, saturated NaHC0₃, and saturated NaCI. Drying and removal of the solvent under reduced pressure gave the crude product which was purified by chromatography on silica gel, eluting with EtOAc/hexane (1/1), then EtOAc/hexane (3/1). There was obtained 2.6 g of product, of sufficient purity for using in the next reaction.

### DNMA-LYS(Z)-CYSTA-NHCH₂CH(CH₃)CH₂CH₃

A solution 2.6 g (3.3 mmole) of BOC-LYS(Z)-CYSTA-NHCH₂CH(CH₃)CH₂CH₃ in 20 ml of CH₂Cl₂ was treated with 5 ml of TFA and stirred at room temperature for five hours. The solvent was removed under reduced pressure and the residue taken up in EtOAc and washed with saturated NaHC0₃, then saturated NaCI. Drying and removal of the solvent under reduced pressure gave crude LYS(Z)-CYSTA-NHCHzCH-(CH₃)CH₂CH₃.

This was taken up in 20 ml DMF and 1.12 g (3.3 mmole) of di-(1-naphthylmethyl)acetic acid and 0.45 g (3.3 mmole) of HOBT added. The solution was cooled in ice and 0.68 g (3.3 mmole) of DCC added and the solution allowed to warm to room temperature overnight. The mixture was filtered and the filtrate diluted with EtOAc. The solution was washed with H₂0, saturated NaHCO₃, and saturated NaCI. Drying and removal of the solvent under reduced pressure gave the crude material which could be purified by recrystallization from EtOAc. There was obtained 2.8 g of product. The structure was confirmed by NMR spectroscopy.

### DNMA-LYS-CYSTA-NHCH₂CH(CH₃)CH₂CH₃

A solution of 2.8 g of DNMA-LYS(Z)-CYSTA-NHCH₂CH(CH₃)CH₂CH₃ in 30 ml MeOH was treated with 0.2 g of 20% Pd/C and stirred under a hydrogen atmosphere for six hours. The mixture was filtered, and the solvent removed under reduced pressure to give 2.0 g of the product. The structure was confirmed by NMR and mass spectroscopy.

### INTERMEDIATES FOR EXAMPLE 14

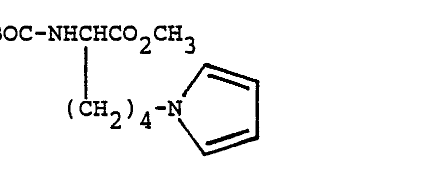

A solution of 1.7 g (6.5 mmole) of α-BOC-LYS-OCH₃ in 30 ml HOAc was treated with 0.86 g (6.5 mmole) of 2,5-dimethoxyfuran and heated to reflux while allowing 15 ml of HOAc to distill off. The remainder of the HOAc was removed under reduced pressure. The residue was taken up in EtOAc and washed with saturated NaHC0₃, then saturated NaCI. Drying and removal of the solvent under reduced pressure gave the crude product which was purified by chromatography on silica gel, eluting with hexane/EtOAc (4/1). There was obtained 0.9 g of product. The structure was confirmed by NMR spectroscopy.

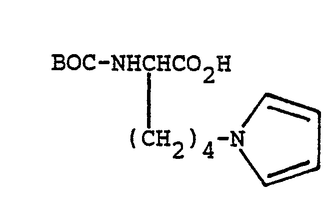

A solution of 0.9 g (2.9 mmole) of 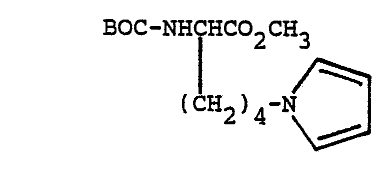 in 15 ml dioxane was cooled to -15° and 0.12 g (3.0 mmole) of NaOH in 10 ml H₂O added, and the solution allowed to stir at room temperature for two hours. The solution was acidified with citric acid and extracted with EtOAc. The EtOAc was washed with H₂0, then saturated NaCi. Drying and removal of the solvent under reduced pressure gave 0.75 g of product, of sufficient purity for use in the following step. The structure was confirmed by NMR spectroscopy.

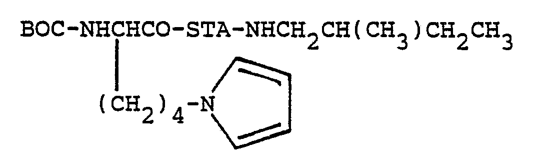

A solution of 0.75 g (2.5 mmole) of

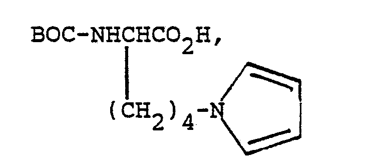

0.34 g (2.5 mmole) of HOBT, and 0.71 g (2.5 mmole) of STA-NHCH₂CH(CH₃)CN₂CH₃•HCl in 15 ml of DMF was cooled in ice and 0.35 ml (2.5 mmole) of Et₃N added, followed by 0.52 g (2.5 mmole) of DCC. The cooling was removed and the solution allowed to stir at room temperature overnight. The mixture was filtered and the filtrate diluted with EtOAc and washed with H₂0, saturated NaHC0₃, and saturated NaCI. Drying and removal of the solvent under reduced pressure gave the crude product which was chromatographed on silica gel, eluting with EtOAc/hexane (1/1). There was obtained 0.8 g of product. The structure was confirmed by NMR spectroscopy.

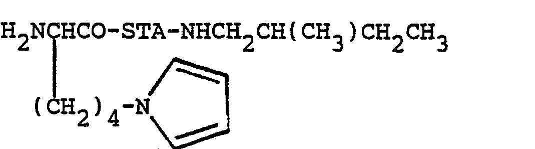

A solution of 0.8 g of 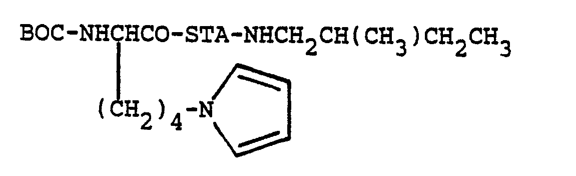 in 25 ml MeOH was treated with 10% HCI until the solution became cloudy. The mixture was allowed to stir at room temperature for eight hours. The solvent was removed under reduced pressure and the residue mixed with sodium carbonate solution and extracted with EtOAc. The solution was washed with saturated NaCi, dried, and the solvent removed under reduced pressure to give 0.5 g of product of sufficient purity for use in the following step. The structure was confirmed by NMR spectroscopy.

### INTERMEDIATES FOR EXAMPLE 15

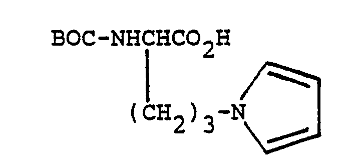

A solution of 1.2 g (5.2 mmole) of α-BOC-ORN in 30 ml of HOAc was treated with 0.8 g (6.1 mmole) of 2,5-dimethoxyfuran and heated to reflux while allowing 15 ml of HOAc to distill off. The remainder of the HOAc was removed under reduced pressure. The residue was taken up in dilute NaOH, acidified with 1 N citric acid and extracted with CHCl₃. The CHC1₃ was dried and the solvent removed under reduced pressure giving the crude product which was purified by taking up in EtOAc and filtering through a plug of silica gel. Removal of the solvent under reduced pressure gave 0.7 g of the product as a yellow oil. The structure was confirmed by NMR spectroscopy.

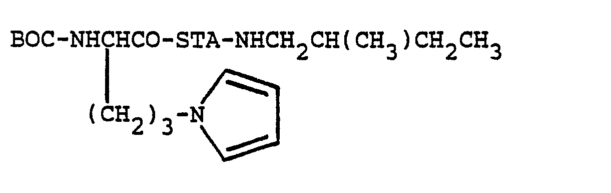

A solution of 0.55 g (1.9 mmole) of 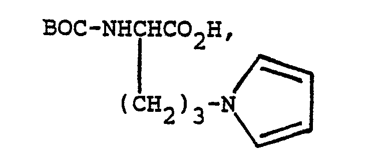 0.54 g (1.9 mmole) of STA.NHCH₂CH(CH₃)CH₂CH₃•HCl, and 0.25 g (1.9 mmole) of HOST in 15 ml DMF was cooled in ice and 0.27 ml (1.9 mmole) of Et₃N was added, followed by 0.4 g (1.9 mmole) of DCC. The solution was allowed to warm to room temperature and stir overnight. The mixture was filtered and the filtrate diluted with EtOAc and washed with H₂0, saturated NaHC0₃, and saturated NaCI. Drying and removal of the solvent under reduced pressure gave the crude product which was chromatographed on silica gel, eluting with EtOAc/hexane (1/1). There was obtained 0.76 g of product, mp 136-139°. The structure was confirmed by NMR spectroscopy.

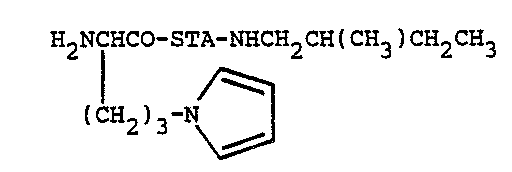

A solution of 0.76 g (1.5 mmole) of 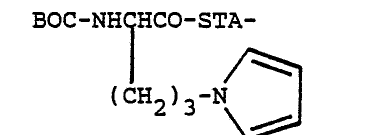 NHCH₂CH(CH₃CH₂CH₃ in 20 ml MeOH was treated with 15 ml 10% HCI and stirred at room temperature for three hours. The solvent was removed under reduced pressure, and the residue was mixed with saturated NaHC0₃ and extracted with EtOAc. Drying and removal of the solvent under reduced pressure gave 0.61 g of product, of sufficient purity for use in the following reaction. The structure was confirmed by NMR spectroscopy.

### INTERMEDIATES FOR EXAMPLE 16

### BOC-LYS(Z)-STA-LEU-NHCH₂Ph

A solution of 4.0 g (10.5 mmole) of BOC-LYS(Z), 4.35 g (10.5 mmole) of STA-LEU-NHCH₂Ph•HCl, and 1.42 g (10.5 mmole) of HOBT in 30 ml DMF was cooled in ice and 1.46 ml (10.5 mmole) of Et₃N added, followed by 2.2 g (10.7 mmole) of DCC. The solution was stirred at room temperature for three days. The mixture was filtered and the filtrate diluted with EtOAc and washed with H₂0, saturated NaHC0₃, and saturated NaCI. Drying and removal of the solvent under reduced pressure gave the crude product which was purified by chromatography on silica gel, eluting with EtOAc/hexane (1/1), then EtOAc. There was obtained 6.6 g of product. The structure was confirmed by NMR spectroscopy.

### LYS(Z)-STA-LEU-NHCH₂Ph

A solution of 6.6 g (8.9 mmole) of BOC-LYS(Z)-STA-LEU-NHCH₂Ph in 100 ml CH₂Cl₂ was treated with 15 ml TFA and stirred at room temperature for two hours. The solvent was removed under reduced pressure and the residue mixed with sodium carbonate solution and extracted with EtOAc. The EtOAc was washed with saturated NaCI, dried, and the solvent removed under reduced pressure to give 5.6 g of the product, of sufficient purity to use in the following reaction.

### INTERMEDIATE FOR EXAMPLES 17-22

### DNMA-LYS-STA-LEU-NHCH₂Ph

A solution of 2.0 g (2.1 mmole) of DNMA-LYS(Z)-STA-LEU-NHCH₂Ph in 30 ml MeOH was treated with 0.2 g 20% Pd/C and stirred under a hydrogen atmosphere for five hours. The mixture was filtered and the solvent removed under reduced pressure to give 1.8 g of the product, sufficiently pure for use in the following reactions.

### INTERMEDIATES FOR EXAMPLE 20

### CH₃SO₃(CH₂)₅-NHZ

A solution of 4.0 g (16.9 mmole) of HO-(CH₂)₅-NHZ in 70 ml CHCl₃ was cooled to -20° and 2.35 ml (16.9 mmole) of Et₃N added followed by the dropwise addition of 1.3 mi. (16.9 mmole) of methanesulfonyl chloride. The solution was stirred at -20° for two hours, then washed with saturated NaHC0₃ and saturated NaCI. Drying and removal of the solvent under reduced pressure left 5.6 g of the crude product, sufficiently pure for use in the following reaction.

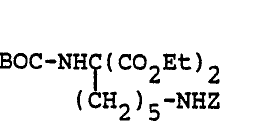

0.85 g (17.7 mmole) of NaH (50% in mineral oil) was washed free of oil and suspended in 20 ml of DMSO. To this was added 4.8 g (17.4 mmole) of BOC-NHCH(CO₂Et)₂ in portions and the suspension stirred until the evolution of hydrogen had ceased. This was then treated with 5.6 g (17.8 mmole) of CH₃SO₃-(CH₂)₅-NHZ and 2.0 g of KI and stirred at room temperature for four days. The mixture was diluted with EtOAc and washed with 1 N citric acid, H₂0, saturated NaHC0₃, then saturated NaCI. Drying and removal of the solvent under reduced pressure left 9.7 g of the crude product, sufficiently pure for use in the following step.

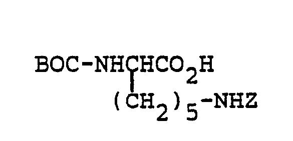

A solution of 9.7 g (19.6 mmole) of 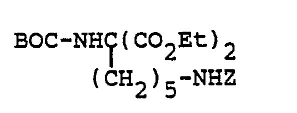 in 20 ml of dioxane was treated with 1.6 g (40 mmole) of NaOH in 10 ml of H₂0 and the solution stirred at room temperature for three hours. The solution was diluted with H₂0 and extracted with Et₂O. The aqueous phase was brought to pH 1 and extracted with EtOAc. This was washed with H₂0 and saturated NaCI. Drying and removal of the solvent under reduced pressure gave the malonic acid.

This was dissolved in 100 ml toluene and heated at reflux for four hours. The toluene was removed under reduced pressure and the residue chromatographed on silica gel, eluting with hexane/EtOAc (5/1). There was obtained 1.9 g of product. The structure was confirmed by NMR spectroscopy.

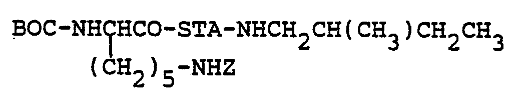

A solution of 1.9 g (4.8 mmole) of 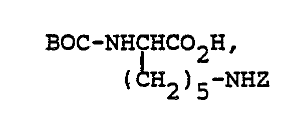 1.1 g (4.5 mmole) of STA-NHCH₂CH(CH₃CH₂CH₃, and 0.65 g (4.8 mmole) of HOBT in 20 ml DMF was cooled in ice and 0.94 g (4.6 mmole) of DCC added and the solution stirred at room temperature overnight. The mixture was filtered and the filtrate diluted with EtOAc and washed with H₂0, saturated NaHC0₃, then saturated NaCl. Drying and removal of the solvent under reduced pressure left the crude product which was chromatographed on silica gel, eluting with hexaneiEtOAc (1/1). There was obtained 2.1 g of product, sufficiently pure for use in the following step.

### INTERMEDIATE FOR EXAMPLE 21 AND 22

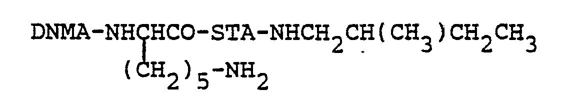

A solution of 0.4 g (0.5 mmole) of 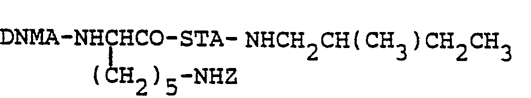 in 20 ml MeOH was treated with 0.1 g of 20% Pd/C and stirred under hydrogen for three hours. The mixture was then filtered and the solvent removed under reduced pressure to give 0.3 g of the product, sufficiently pure for use in the following reactions.

### INTERMEDIATES FOR EXAMPLE 25

### CH₃S0₃(CH₂)s-NHZ

A solution of 4.0 g (15.9 mmole) of HO-(CH₂)₆-NHZ in 70 ml CHCl₃ was cooled to -20° and 2.35 ml (16.9 mmole) of Et₃N added followed by the dropwise addition of 1.3 ml (16.9 mmole) of methanesulfonyl chloride. The solution was stirred at -20° for two hours, then washed with saturated NaHCO₃ and saturated NaCI. Drying and removal of the solvent under reduced pressure left 5.8 g of the crude product, sufficiently pure for use in the following reaction.

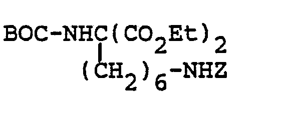

0.85 g (17.7 mmole) of NaH (50% in mineral oil) was washed free of the oil and suspended in 20 ml DMSO. To this was added 4.8 g (17.4 mmole) of BOC-NHCH(C0₂Et)₂ in portions and the suspension stirred until the evolution of hydrogen had ceased. This was then treated with 5.8 g (17.6 mmole) of CH₃SO₃-(CH₂)₆-NHZ and 2.0 g of KI and stirred at room temperature for four days. The mixture was diluted with EtOAc and washed with 1 N citric acid, H₂0, saturated NaHC0₃, and saturated NaCI. Drying and removal of the solvent under reduced pressure gave 9.0 g of crude product, sufficiently pure for use in the following reaction.

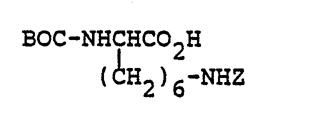

A solution of 9.0 g (17.7 mmole) of 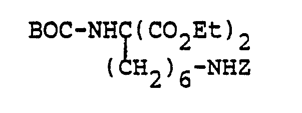 in 30 ml dioxane was treated with 1.5 g (37.5 mmole) of NaOH in 10 ml H₂0 and the solution stirred at room temperature for three hours. The solution was diluted with H₂0 and extracted with Et₂O. The pH was brought to 1 and the mixture extracted with EtOAc, and the EtOAc washed with H₂O and saturated NaCl. Drying and removal of the solvent under reduced pressure gave the malonic acid. This was taken up in 100 ml toluene and heated at reflux for four hours. The solvent was removed under reduced pressure and the residue chromatographed on silica gel, eluting with hexane/EtOAc (5/1), to give 1.1 g of product.

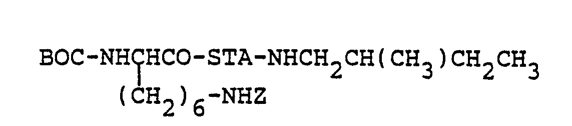

A solution of 1.1 g (2.9 mmole) of 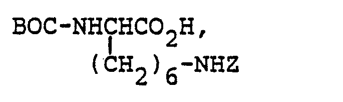 0.71 g (2.9 mmole) of STA-NHCH₂CH(CH₃)CH₂CH₃, and 0.39 g (2.9 mmole) of HOBT in 20 ml DMF was cooled in ice and 0.6 g (2.9 mmole) of DCC added and the solution left stirring at room temperature overnight. The mixture was then filtered and the filtrate diluted with EtOAc. The EtOAc was washed with H₂0, saturated NaHCOs, and saturated NaCI. Drying and removal of the solvent under reduced pressure gave the crude product which was chromatographed on silica gel, eluting with EtOAc/hexane (1/1). There was obtained 1.3 g of product.

### INTERMEDIATE FOR EXAMPLES 26 AND 27

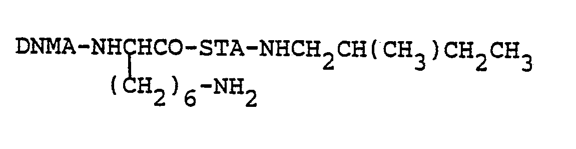

A solution of 0.5 g (0.6 mmole) of 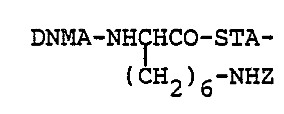 NHCH₂CH(CH₃)CH₂CH₃ in 20 ml MeOH was treated with 0.1 g of 20% Pd/C and stirred under hydrogen for four hours. The mixture was filtered and the solvent removed under reduced pressure to give 0.42 g of the crude product, sufficiently pure to use in the following steps.

### INTERMEDIATE FOR EXAMPLE 28

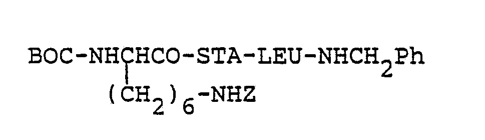

A solution of 0.86 g (2.3 mmole) of 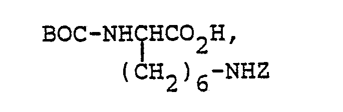 0.86 g (2.1 mmole) of STA-LEU-NHCH₂Ph.HCI, and 0.3 g (2.2 mmole) of HOBT in 15 ml DMF was cooled in ice and treated with 0.4 ml (2.9 mmole) of Et₃N followed by 0.5 g (2.4 mmole) of DCC. The solution was allowed to stir at room temperature overnight. The mixture was filtered and the filtrate diluted with EtOAc and washed with H₂0, saturated NaHC0₃, and saturated NaCI. Drying and removal of the solvent under reduced pressure gave the crude product which was chromatographed on silica gel, eluting with EtOAc/hexane (1/1). There was obtained 1.0 g of product. The structure was confirmed by NMR spectroscopy.

### INTERMEDIATES FOR EXAMPLE 29

### ClCH₂C≡C-CH-2NHZ

A solution of 1.0 g (7.1 mmole) of ClCH₂C≡C-CH₂NH2•HCl (Ann Chim. 13, 656 (1958)) in 20 ml of THF/H₂0 (1/1) was cooled in ice and treated with 0.3 g (7.5 mmole) of NaOH in 5 ml of H₂0, then treated dropwise with 1.0 ml (7.1 mmole) of benzyl chloroformate while simultaneously adding dropwise a solution of 0.3 g (7.5 mmole) of NaOH in 5 ml H₂0. The solution was allowed to stir at 0° for two hours, then was extracted with EtOAc. The EtOAc was washed with saturated NaHC0₃ and saturated NaCI. Drying and removal of the solvent under reduced pressure gave 1.8 g of the product as an oil which solidified on standing. The structure was confirmed by NMR spectroscopy.

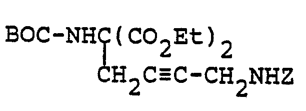

0.33 g (6.9 mmole) of NaH (50% in mineral oil) was washed free of the oil and suspended in 15 ml DMSO. To this was added 1.9 g (6.8 mmole) of BOC-NHCH(C0₂Et)₂ and the suspension stirred until the evolution of hydrogen had ceased. This was then treated with 1.8 g (6.6 mmole) of CICH₂C=C-CH₂NHZ and 2.0 g KI and stirred at room temperature overnight. The solution was diluted with EtOAc and washed with H₂0, saturated NaHCO₃, and saturated NaCI. Drying and removal of the solvent under reduced pressure gave the crude product which was chromatographed on silica gel, eluting with hexane/EtOAc (9/1). There was obtained 2.5 g of product. The structure was confirmed by NMR spectroscopy.

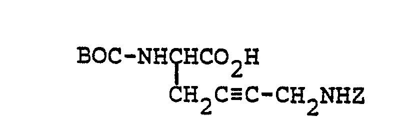

A solution of 2.5 g (5.4 mmole) of 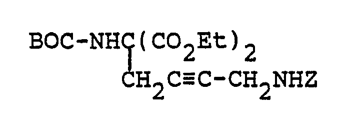 in 20 ml THF was treated with 1.0 g (25.0 mmole) of NaOH in 20 ml H₂0 and the solution stirred at room temperature for six hours. The solution was acidified with 1 N citric acid and extracted with EtOAc. The EtOAc was washed with H₂0 and saturated NaCI. Drying and removal of the solvent under reduced pressure gave the malonic acid.

This was taken up in 25 ml toluene and heated at reflux for two hours. The solvent was removed under reduced pressure to give the crude product which was chromatographed on silica gel, eluting with hexane/EtOAc (3/1). There was obtained 1.6 g of product, sufficiently pure for use in the following reaction.

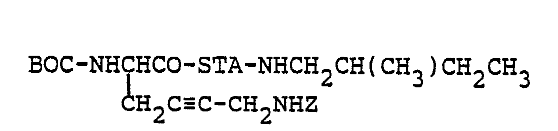

A solution of 1.6 g (4.3 mmole) of 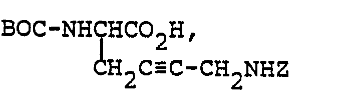 1.0 g (4.1 mmole) of STA-NHCH₂CH(CH₃)CH₂CH₃, and 0.57 g (4.2 mmole) of HOBT in 15 ml DMF was cooled in ice and treated with 0.88 g (4.3 mmole) of DCC and the solution allowed to stir at room temperature for four days. The mixture was filtered and the filtrate diluted with EtOAc and washed with H₂O, saturated NaHC0₃, and saturated NaCI. Drying and removal of the solvent under reduced pressure gave the crude product which was chromatographed on silica gel, eluting with EtOAc/hexane (1/1). There was obtained 2.5 g of product.

### INTERMEDIATES FOR EXAMPLES 30 AND 31

### BOC-ORN(Z)-STA-NHCH₂CH(CH₃)CH₂CH₃

A solution of 1.3 g (3.5 mmole) of BOC-ORN(Z), 1.0 g (3.6 mmole) of STA-NHCH₂CH(CH₃)CH₂CH₃•HCl. and 0.48 g (3.6 mmole) of HOBT in 20 ml DMF was cooled in ice and 0.5 ml (3.6 mmole) of Et₃N added followed by 0.74 g (3.6 mmole) of DCC. The solution was allowed to stir at room temperature overnight. The mixture was filtered and the filtrate diluted with EtOAc and washed with H₂0, saturated NaHC0₃, then saturated NaCI. Drying and removal of the solvent under reduced pressure gave the crude product which was chromatographed on silica gel, eluting with EtOAc/hexane (1/1). There was obtained 1.5 g of product. The structure was confirmed by NMR and mass spectroscopy.

### ORN(Z)-STA-NHCH₂CH(CH₃)CH₂CH₃

A solution of 1.25 g (2.1 mmole) of BOC-ORN(Z)-STA-NHCH₂CH(CH₃)CH₂CH₃ in 50 ml CH₂CI₂ was treated with 6 ml TFA and stirred at room temperature for two hours. The solvent was removed under reduced pressure and the residue taken up in EtOAc and washed with saturated NaHC0₃ and saturated NaCI. Drying and removal of the solvent under reduced pressure left 1.0 g of product. The structure was confirmed by NMR spectroscopy.

### DNMA-ORN(Z)-STA-NHCH2CH(CHs)CHzCH3

A solution of 0.72 g (2.1 mmole) of di-(1-naphthylmethyl) acetic acid, 1.0 g (2.0 mmole) of ORN(Z)-STA-NHCH₂CH(CH₃)CH₂CH₃, and 0.29 g (2.1 mmole) of HOBT in 20 ml DMF was cooled in ice and treated with 0.44 g (2.1 mmole) of DCC and the solution stirred at room temperature for three days. The mixture was filtered and the filtrate diluted with EtOAc and washed with H₂0, saturated NaHCO₃, and saturated NaCI. Drying and removal of the solvent under reduced pressure gave the crude product which was chromatographed on silica gel, eluting with EtOAc/hexane (1/1), then EtOAc. There was obtained 1.27 g of product. The structure was confirmed by NMR and mass spectroscopy.

### DNMA-ORN-STA-NHCH₂CH(CH₃)CH₂CH₃

A solution of 1.1 g of DNMA-ORN(Z)-STA-NHCH₂CH(CH₃)CH₂CH₃ in 20 ml MeOH was treated with 0.1 g 20% Pd/C and stirred under hydrogen for five hours. The mixture was filtered and the solvent removed under reduced pressure to give 1.0 g of product, sufficiently pure for use in the following reactions.

### INTERMEDIATES FOR EXAMPLE 33

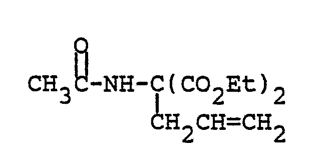

To 150 ml absolute EtOH was added 4.6 g (0.2 g.atom) of sodium. When all had reacted, the solution was treated with 43.5 g (0.2 mole) of diethyl acetylaminomalonate. The solution was warmed to 50° for one hour, then recooled and treated dropwise with a solution of 17.4 ml (0.2 mole) of allyl bromide in 100 ml absolute EtOH. The mixture was kept at 50° for twenty-four hours, then stripped to an oily solid. The material was taken up in EtzO and washed twice with H₂0, then saturated NaCI. Drying and removal of the solvent under reduced pressure left 42.4 g of the crude product. The structure was confirmed by NMR spectroscopy.

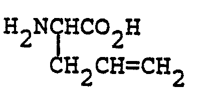

A solution of 42.4 g of 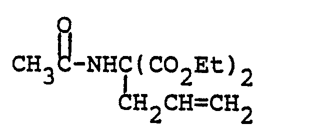 in 200 ml of concentrated HCI was heated at reflux for seven hours, then stripped under reduced pressure to a viscous oil. This was taken up in 140 ml absolute EtOH and concentrated NH₄0H was added until basic to litmus. The solution was cooled and the precipitated product collected by filtration. There was obtained 16.3 g of product. The structure was confirmed by NMR spectroscopy.

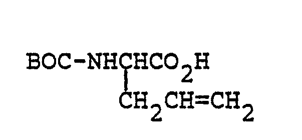

A suspension of 16.1 a (0.14 mole) of 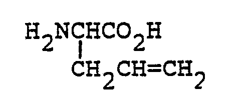 in 150 ml dioxane was treated with 70 ml (0.14 mole) of 2 N NaOH causing solution. Di-t-butyldicarbonate (34.0 g, 0.15 mole) was added and the solution stirred at room temperature for two hours. The pH was adjusted to 8.5 and the solution diluted with H₂0 and washed twice with Et₂O. The pH was then brought to 2.0 and washed twice with Et₂O. The Et₂O was washed with saturated NaCl, dried, and the solvent removed under reduced pressure giving 19.7 g of the product as a clear oil. The structure was confirmed by NMR and mass spectroscopy.

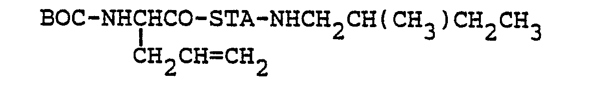

A solution of 2.16 g (10.0 mmole) of 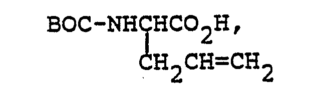 1.35 g (10.0 mmole) of HOBT and 2.81 g (10.0 mmole) of STA-NHCH₂CH(CH₃)CH₂CH₃•HCl in 30 ml DMF was cooled in ice and 1.4 ml (10.0 mmole) of Et₃N added, followed by 2.1 g (10.0 mmole) of DCC and the solution allowed to stir at room temperature overnight. The mixture was filtered and the solvent removed under high vacuum. The residue was taken up in EtOAc and washed with 1 N HCI, saturated NaHC0₃, and saturated NaCI. Drying and removal of the solvent under reduced pressure gave the crude product which was chromatographed on silica gel, eluting with CHCl₃/MeOH (98/2). There was obtained 1.94 g of the product as a white foam. The structure was confirmed by NMR and mass spectroscopy.

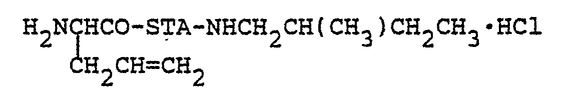

A solution of 1.94 g (4.4 mmole) of 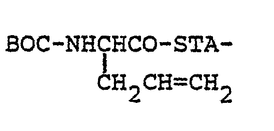 NHCH₂CH(CH₃)CH₂CH₃ in 20 ml CH₂Cl₂ was treated with 10 ml TFA and stirred for one hour. The solvent was removed under reduced pressure and the residue taken up in CH₂Cl₂ and the solvent removed again. The residue was then taken up in CH₂Cl₂ and treated with HCI gas. The solvent was removed under reduced pressure to give 1.66 g of the product, of sufficient purity to use in the following step.

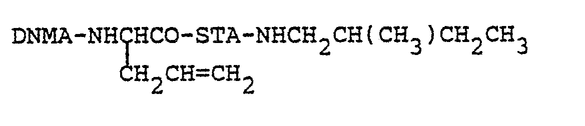

A solution of 1.5 g (4.4 mmole) of di-(1-naphthylmethyl) acetic acid and 0.6 g (4.4 mmole) of HOBT in 20 ml DMF was cooled in ice and 0.92 g (4.4 mmole) of DCC added and the solution stirred at 0° for fifteen minutes.

To this was added a cold solution of 1.66 g (4.4 mmole) of 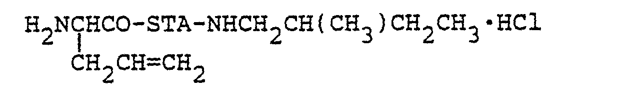 and 0.62 ml (4.4 mmole) of Et₃N in 20 ml DMF. The solution was allowed to stir at room temperature overnight. The mixture was poured into H₂0 and extracted three times with EtOAc. The EtOAc was washed with H₂0, 1 N HCI, saturated NaHC0₃, and saturated NaCI. Drying and removal of the solvent under reduced pressure gave the crude product which was chromatographed on silica gel, eluting with CHCl₃/MeOH (98/2). There was obtained 2.4 g of product contaminated with some starting di-(1-naphthylmethyl)acetic acid. The residue was taken up in Et₂O and washed twice with 1 N NaOH, H₂0, then saturated NaCl. Drying and removal of the solvent under reduced pressure gave 2.12 g of the product as a white foam. The structure was confirmed by NMR and mass spectroscopy.

### INTERMEDIATES FOR EXAMPLES 34 AND 35

### AMENOMALONIC ACID, METHYL BENZYL ESTER

Methyl, benzyl, isonitrosomalonate was prepared from 97 g of methyl, benzyl malonate by the procedure described in Org. Syn. Coll. Vol. 5, p. 373. The 100 g of crude product obtained was reduced to the amino derivative by the procedure described in J. Am. Chem. Soc., 75, 1970 (1953). The 94 g of crude product was used in the following step without further purification.

### BOC-AMINOMALONIC ACID, METHYL BENZYL ESTER

A solution of 94 g of aminomalonic acid, methyl benzyl ester in 75 ml Et₂0 was cooled to 5° and treated with 91.66 g of di-t-butyldicarbonate. After standing at 4° overnight, the solvent was removed under reduced pressure giving an oil. Chromatography on silica gel, eluting with hexane/EtOAc (85/15) gave 66.97 g (49% yield) of the product as an oil which solidified on standing. The structure was confirmed by NMR and mass spectroscopy.

### BOC-AMINOMALONIC ACID, METHYL ESTER

To a solution of 16.17 g (13.5 mmole) of BOC-aminomalonic acid, methyl benzyl ester in 250 ml MeOH was added 0.66 g of 20% Pd/C catalyst. The suspension was purged with hydrogen gas for one and one-half hours, after which the suspension was filtered and the solvent removed under reduced pressure at 30°, giving a syrup, 12.5 g. The product was kept at 4° until use in the following reaction.

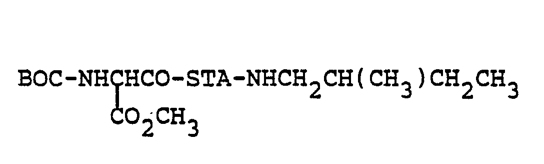

A solution of 2.50 g (10.7 mmole) of BOC-aminomalonic acid, methyl ester and 1.52 g (11 mmole) of HOBT in 50 ml DMF was combined with a solution of 3.01 g (10.7 mmole) of STA-NHCH₂CH(CH₃)-CH₂CH₃•HCl and 1.57 ml (11.0 mmole) Et₃N in 25 ml DMF. To the mixture was added 2.32 g (11.0 mmole) DCC, and the mixture stirred for five hours at room temperature, then kept at 4° overnight. The mixture was filtered, and the filtrate evaporated under high vacuum to a glass, 4.29 g. The crude product was chromatographed on silica gel, eluting with EtOAc. Combination of the appropriate fractions gave a white foam, 3.09 g, of sufficient purity for use in the following reaction. The structure was confirmed by NMR and mass spectroscopy.

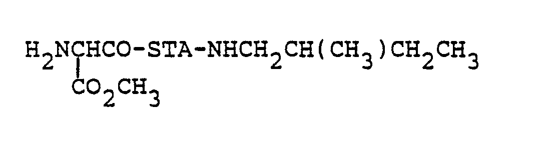

To a solution of 2.86 g (6.22 mmole) of 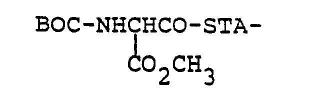 NHCH₂CH(CH₃)CH₂CH₃ in 75 ml CH₂Cl₂ was added 10 ml TFA. After two hours at room temperature the solvent was removed under reduced pressure, and the residue partitioned between Et₂0 and saturated NaCl which had been adjusted to pH 9.5 with solid sodium carbonate. The organic phase was washed with saturated NaCI, dried, and evaporated to a foam, 1.62 g. The material was sufficiently pure for use in the following reaction.

### INTERMEDIATE FOR EXAMPLE 36

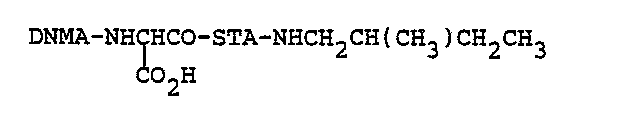

To a solution of 1.79 g (2.63 mmole) of 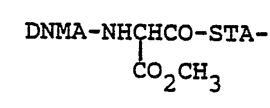 NHCH₂CH(CH₃)CH₂CH₃ in 25 ml MeOH was added 5.0 ml 1 N NaOH. After stirring one and one-half hours at room temperature, 4.9 ml 1 N HCI was added. The solvent was removed under reduced pressure and the residue was taken up in EtOAc. The mixture was filtered and the filtrate was washed with 1 N HCI, saturated NaCl, and dried. Removal of solvent under reduced pressure gave a foam, 1.78 g. The structure was confirmed by NMR and mass spectroscopy.

Calcd. for C₄₀H₄₉N₃O₆•0.25C₄H₈O₂ (MW 689.84): C, 71.38; H, 7.45; N, 6.09 Found C, 71.03; H, 7.46; N, 6.16.

### INTERMEDIATES FOR EXAMPLE 40

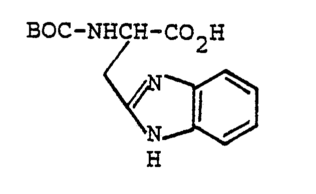

A solution of 5.45 g (19.6 mmole) of 2-(2-benzimidazole) alanine•2HCl (J. Chem. Soc., 1600 (1951)) in 100 ml CH₂Cl₂ was treated with 4.49 g (20.6 mmole) of di-t-butyldicarbonate and 8.6 ml (61.7 mmole) of Et₃N and stirred at room temperature overnight. The solvent was removed under reduced pressure and the residue taken up in 1 N NaOH. Adjusting the pH to 3.0 gave a white solid which was collected and washed with H₂O. There was obtained 4.92 g of product, sufficiently pure to use in the following reaction. The structure was confirmed by NMR and mass spectroscopy.

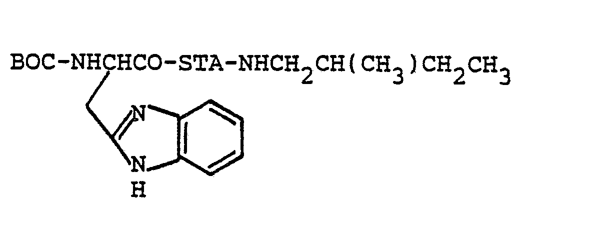

A suspension of 1.09 g (3.56 mmole) of BOC-2-(2-benzimidazole)alanine, 0.5 g (3.74 mmole) of HOBT, and 1.0 g (3.56 mmole) of STA-NHCH₂CH(CH₃)CH₂CH₃•HCl in 15 mt DMF was treated dropwise with Et₃N until solution occurred. The solution was then treated with 0.77 g (3.74 mmole) of DCC and stirred at room temperature overnight. The mixture was filtered and the DMF removed under high vacuum. The residue was taken up in EtOAc and washed with 1 N citric acid, saturated NaHC0₃, and saturated NaCI. Drying and removal of the solvent under reduced pressure gave the crude product which was chromatographed on silica gel, eluting with CHCb/MeOH (98/2). There was obtained 1.6 g of product sufficiently pure for use in the following step. The structure was confirmed by NMR and mass spectroscopy.

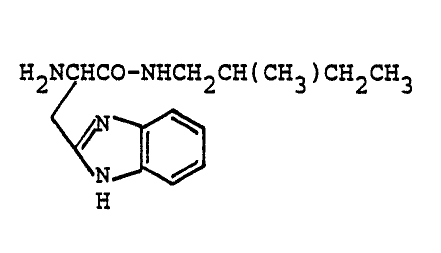

A solution of 1.6 g of 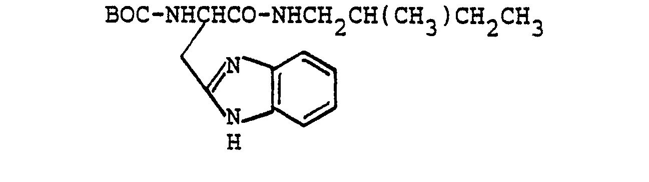 in 20 ml CH₂C1₂ was treated with 20 ml TFA and allowed to stir at room temperature for two hours. The solvent was removed under reduced pressure and the residue redissolved in CH₂CI₂ and the solvent removed again. The residue was taken up in EtOAc and washed with 1 N NaOH, then saturated NaCI. Drying and removal of the solvent under reduced pressure gave the crude product which was chromatographed on silica gel, eluting with CHCl₃/MeOH (95/5). There was obtained 0.77 g of product of sufficient purity to use in the following step. The structure was confirmed by mass spectroscopy.

### INTERMEDIATES FOR EXAMPLE 41

### BOC-LYS(TOS)-STA-NHCH₂CH(CH₃)CH₂CH₃

To a solution of 0.72 g (1.83 mmole) of BOC-LYS(TOS) and 0.3 g (2.1 mmole) of HOBT in 15 ml DMF at -5° was added a solution of 0.56 g (2.0 mmole) of STA-NHCH₂CH(CH₃)CH₂CH₃•HCl and 0.29 ml (2.1 mmole) Et₃N in 20 ml DMF, and the combined solutions treated with 0.43 g (2.1 mmole) DCC. After stirring at room temperature overnight, the mixture was filtered, the solvent removed under high vacuum, and the residue taken up in EtOAc. The solution was washed with 1 N citric acid, saturated NaCl, saturated NaHC0₃, and saturated NaCI. After drying, the solvent was removed under reduced pressure and the residue was chromatographed on silica gel, eluting with a gradient of 0 to 5% MeOH in CHCI₃. The product was recovered by combination of the appropriate fractions giving a white foam, 0.80 g, of suitable purity for use in the following reaction. The structure was confirmed by mass spectroscopy.

### LYS(TOS)-STA-NHCH₂CH(CH£)CH₂CH₃

To a solution of 0.80 g (1.3 mmole) of BOC-LYS(TOS)-STA-NHCH₂CH(CH£)CH₂CH₃ in 30 ml CH₂CI₂ was added 15 ml TFA. After stirring at 25° for one and one-half hours the mixture was evaporated to an oil, taken into EtOAc and washed with 5 N NaOH, and saturated NaCI. After drying, the solution was evaporated under reduced pressure to a foam, 0.67 g, of sufficient purity for use in the following reaction. The structure was confirmed by mass spectroscopy.

### INTERMEDIATE FOR EXAMPLE 42

### N-(METHYLOXYCARBONYL)SUCCINIMIDE

To a solution of 19.8 g (200 mmole) of succinimide and 22 ml (200 mmole) of N-methylmorpholine in 350 ml THF at -5° was added 15.5 ml (200 mmole) methylchloroformate over 20 minutes. After stirring for two hours at 25°, the solvent was removed under reduced pressure and the residue was taken up in EtOAc. The suspension was filtered, and washed with saturated NaCi, saturated NaHC0₃, and saturated NaCI. After drying, the solution was concentrated under reduced pressure and EtzO was added, giving the product as a crystalline solid, 10.0 g. The structure was confirmed by NMR and mass spectroscopy.

### INTERMEDIATES FOR EXAMPLE 43

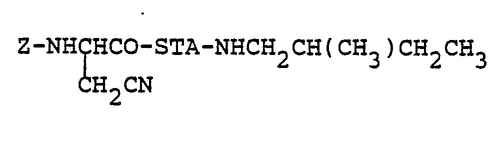

A solution of 1.54 g (6.2 mmole) of (R,S)-Z-β-cyanoalanine (J. Org. Chem. 26, 3356 (1961)) and 0.88 g (6.51 mmole) of HOBT in 5 ml DMF and 45 ml CH₂Cl₂ was cooled in ice and 1.34 g (6.51 mmole) of DCC added, followed by 1.85 g (6.2 mmole) of STA-NHCH₂CH(CH₃)CH₂CH₃•HCl and 1.36 ml (9.7 mmole) of Et₃N. The solution was stirred at room temperature overnight. The mixture was filtered and the solvent removed under reduced pressure. The residue was taken up in EtOAc and washed with 1 N citric acid, saturated NaCI, saturated NaHC0₃, and saturated NaCI. Drying and removal of the solvent under reduced pressure gave the crude product which could be crystallized from EtOAc/Et₂O. There was obtained 2.31 g of the product as a white solid. The structure was confirmed by NMR and mass spectroscopy.

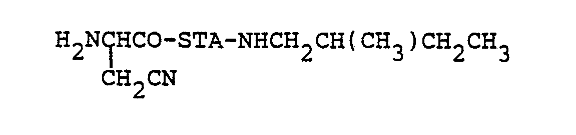

A solution of 2.11 g of 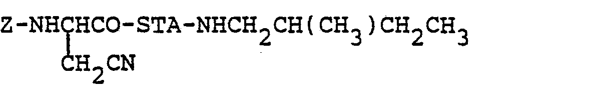 in 80 ml MeOH was treated with 0.2 g of 20% Pd/C and stirred under a hydrogen atmosphere for one and one-half hours. The mixture was filtered and the solvent removed under reduced pressure giving 1.47 g of the product. The structure was confirmed by NMR and mass spectroscopy.

### INTERMEDIATES FOR EXAMPLE 44

### Z-AMINOMALONIC ACID, METHYL t-BUTYL ESTER

### Methyl, t-butyl isonitrosomalonate was prepared from 50 g of methyl, t-butylmalonate by the procedure described in Org. Syn. Coll. Vol. 5, p. 373. The 54 g of crude product thus obtained was reduced to aminomalonic acid methyl, t-butyl ester by the procedure described in J. Am. Chem. Soc., 75, 1970 (1953). To the filtrate of this reaction mixture was added 64.4 g (258 mmoles) N-(benzyloxycarbonyloxy)-succinimide. The mixture was stirred overnight at 25°, filtered, and the solvent removed under reduced pressure. The residue was taken up in EtOAc and washed with 1 N citric acid, saturated NaCI, saturated NaHCO₃, and saturated NaCI. After drying, the solvent was removed under reduced pressure and the residue was chromatographed on silica gel, eluting with hexane/EtOAc (75/25). The appropriate fractions were combined to give an oil, 30.1 g, of sufficient purity for use in the following reaction. The structure was confirmed by NMR and mass spectroscopy.

### AMINOMALONIC ACID, METHYL t-BUTYL ESTER

To a solution of 3.23 g (10 mmole) of Z-aminomalonic acid, methyl t-butyl ester in 25 ml THF was added 0.1 g of 20% Pd/C catalyst. The mixture was purged with hydrogen gas over five hours, filtered, and the solvent removed in vacuo giving an oil, 1.79 g, of sufficient purity for use in the next reaction.

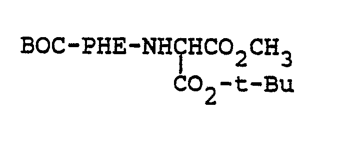

A solution of 2.65 g (10 mmole) of BOC-PHE, 1.39 g (10.3 mmole) of HOBT, and 1.89 g (10 mmole) of aminomalonic acid methyl, t-butyl ester in 80 ml DMF was cooled in ice, and 2.12 g (10.3 mmole) of DCC added. The solution was stirred at 0° for three hours, then at room temperature overnight. The mixture was filtered, and the solvent evaporated under high vacuum. The residue was taken up in EtOAc, and washed with 1 N citric acid, saturated NaCl, saturated NaHC0₃, and saturated NaCI. The solvent was evaporated under reduced pressure and the residue was chromatographed on silica gel, eluting with hexane/EtOAc (75/25). The appropriate fractions were combined giving a white foam, 3.69 g. The material was sufficiently pure for use in the following reaction. The structure was confirmed by NMR and mass spectroscopy.

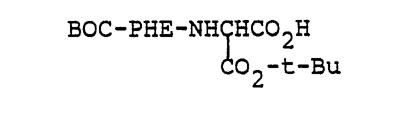

To a solution of 3.44 g (7.88 mmole) of 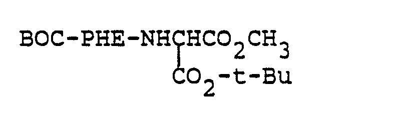 in 20 ml dioxane was added 8.0 ml 1 N NaOH. After one hour, 1 ml 1 N HCI was added, and the solvent was removed under reduced pressure at 25°. The residue was taken up in EtOAc and washed with 1 N citric acid and saturated NaCI. After drying, the solvent was removed under reduced pressure giving a foam, 2.92 g, sufficiently pure for use in the following reaction. The structure was confirmed by NMR and mass spectroscopy.

### INTERMEDIATES FOR EXAMPLE 45

### MALONIC ACID, ETHYL, BENZYL ESTER

To a solution of 17.22 g (114 mmole) of ethyl malonyl chloride in 150 ml THF at 10° was added a solution of 12.19 ml (118 mmole) benzyl alcohol and 16.4 ml (118 mmole) Et₃N in 25 ml THF over thirty minutes. The mixture was stirred overnight, filtered, and the solids washed with THF. The filtrate was evaporated under reduced pressure to an oil, taken up in EtOAc, and washed with 1 N citric acid, saturated NaCI, saturated NaHC0₃, and saturated NaCI. After drying, the solvent was removed at reduced pressure, and the residue was distilled. Collecting the fraction boiling from 118 to 122° at 0.4 mmHg gave the product as an oil, 19.5 g, of sufficient purity for use in the following reaction. The structure was confirmed by NMR and mass spectroscopy.

### BOC-AMINOMALONIC ACID, ETHYL, BENZYL ESTER

Ethyl, benzyl isonitrosomalonate was prepared from 19 g of ethyl benzyl malonate by the procedure described in Org. Syn. Coll. Vol. 5, p. 373. The 24.5 g of crude product thus obtained was reduced to aminomalonic acid, ethyl, benzyl ester by the procedure described in J. Am. Chem. Soc., 75, 1970 (1953), and was immediately converted to BOC-aminomalonic acid, ethyl, benzyl ester by the addition of 19.2 g (88 mmole) of di-t-butyldicarbonate to the reduction mixture. The crude product was chromatographed on silica gel, eluting with hexane/EtOAc (75/25), giving an oil 15.54 g. The product was of sufficient purity for use in the following reaction. The structure was confirmed by NMR and mass spectroscopy.

### AMINOMALONIC ACID, ETHYL BENZYL ESTER HCI

A solution of 8.29 g (24.6 mmole) BOC-aminomalonic acid, ethyl benzyl ester in 100 ml CH₂CI₂ was purged with HCI gas occasionally over five hours. The solvent was removed under reduced pressure and the residue was triturated with Et₂0. After decanting the Et₂O, the residue was stripped of volatiles under high vacuum, giving a crystalline solid, 6.5 g, of sufficient purity for use in the following reaction. The structure was confirmed by NMR and mass spectroscopy.

### DI-(1-NAPHTHYLMETHYL)ACETYL CHLORIDE

A solution of 14.4 g (42 mmole) of di-(1-naphthylmethyl) acetic acid in 100 ml thionyl chloride was stirred at 22° overnight and the solvent removed under reduced pressure. The resulting solid was recrystallized from Et₂O and hexane, giving the product as a beige solid, 13.5 g of sufficient purity for use in the following reaction.

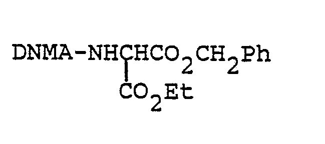

A solution of 3.93 g (11 mmole) of di-(1-naphthylmethyl) acetyl chloride and 3.0 g (11 mmole) of aminomalonic acid, ethyl benzyl ester, hydrochloride in 125 ml THF was cooled to 5° and 3.3 ml (22.6 mmole) Et₃N was added. After stirring overnight at 22°, the mixture was filtered and the solvent removed under reduced pressure. The residue was taken up in EtOAc and washed with 1 N citric acid, saturated NaCI, saturated NaHC0₃, and saturated NaCI. After drying, the solvent was removed under reduced pressure and the residue was chromatographed on silica gel, eluting with hexane/EtOAc (60/40). Combination of the appropriate fractions gave the product as a glassy solid, 5.49 g. The structure was confirmed by NMR and mass spectroscopy.

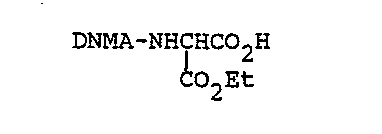

To a solution of 5.3 g (9.47 mmole) of 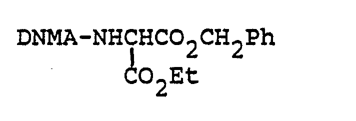 in 100 ml absolute ethanol was added 0.5 g of 20% Pd/C catalyst. The suspension was purged with hydrogen for four hours, filtered, and the solvent removed under reduced pressure giving a white foam, 4.58 g, of sufficient purity for use in the following reaction.

### INTERMEDIATES FOR EXAMPLE 47

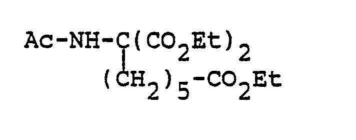

Sodium (2.67 g; 0.116 g.atom) in 115 ml of EtOH was heated until dissolved, and the resulting solution treated dropwise with 26.7 g (0.123 mole) of diethyl acetamidomalonate. The solution was refluxed for one-half hour and then treated dropwise with 25.8 g (0.116 mole) of ethyl 6-bromohexanoate and the resulting solution heated at reflux for eighteen hours. The solution was then filtered and concentrated under reduced pressure. The residue was taken up in Et₂O and washed with H₂0 and saturated NaCI. Drying and removal of the solvent under reduced pressure gave 40.4 g of the product as an oil, sufficiently pure to use in the following reaction.

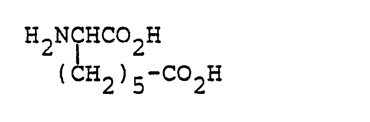

k solution of 39.8 g (0.11 mole) of 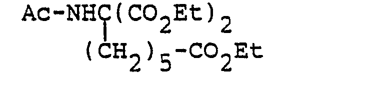 in 200 ml of 6 N HCI was heated at reflux for four days. After cooling the solution was brought to pH 3 with concentrated NH₄0H, and the resulting solid collected and recrystallized from boiling H₂0 to give 11.5 g of the product as a white solid, mp 246-248°.

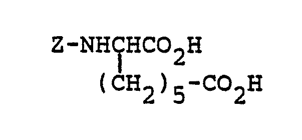

A solution of 40.2 g (0.21 mole) of 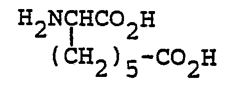 in 470 ml of 1.8 M NaOH was cooled in ice and treated dropwise over twenty minutes with 46.8 ml (0.32 mole) of benzyl chloroformate. After four hours at 0°, the solution was allowed to stir at room temperature overnight. The solution was washed with Et₂0 and then brought to pH 2. This solution was extracted with Et₂O and the Et₂O dried and concentrated under reduced pressure. There was obtained 14.48 g of the product as a white solid, mp 112-115°.

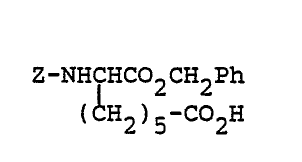

A solution of 16.45 g (0.Q51 mole) of 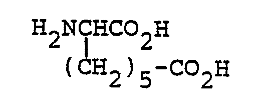 in 10 ml DMF was treated with 5.14 g (0.051 mole) of Et₃N followed by 10.4 g (0.061 mole) of benzyl bromide. The solution was stirred at room temperature overnight, then diluted with H₂0 and extracted with EtOAc. The EtOAc was washed with 5% NaHC0₃, the NaHC0₃ acidified to pH 2, and extracted with EtOAc. The EtOAc was dried and the solvent removed under reduced pressure to give 8.53 g of the product, mp 75-75°.

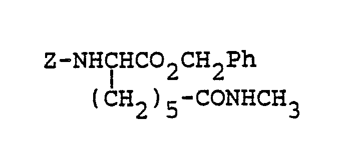

A solution of 2.5 g (6.0 mmole) of 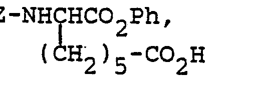 0.41 g (6.0 mmole) of methylamine, hydrochloride, and 0.82 g (6.0 mmole) of HOBT in 30 ml DMF was cooled in ice and 0.61 g (6.0 mmole) Et₃N added followed by 1.24 g (6.0 mmole) of DCC in 10 ml DMF and the solution stirred at room temperature overnight. The mixture was filtered and the solvent removed under high vacuum. The residue was taken up in EtOAc, filtered, and washed with 1 N HCI, saturated NaHCO₃, and saturated NaCI. Drying and removal of the solvent under reduced pressure gave the crude product which was chromatographed on silica gel, eluting with CHCl₃/MeOH (97/3). There was obtained 2.04 g of the product as a white foam.

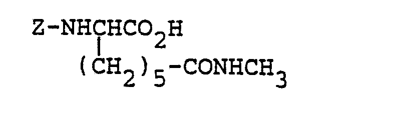

A solution of 1.54 g (3.6 mmole) of 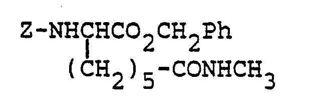 in 30 ml MeOH was treated with 288 mg (7.2 mmole) of NaOH in 5 ml H₂0 and the solution stirred at room temperature for one and one-half days. The solvent was removed under reduced pressure and the residue taken up in CH₂Cl₂ and washed with 1 N HCI, then saturated NaCI. Drying and removal of the solvent under reduced pressure gave the crude product which was chromatographed on silica gel, eluting with EtOAc, then MeOH. There was obtained 0.89 g of the product as an oil which crystallized on standing.

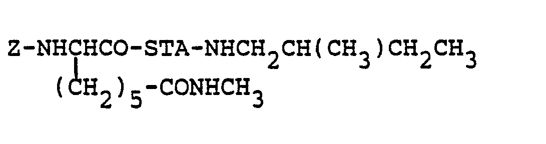

A solution of 0.71 g (2.2 mmole) of 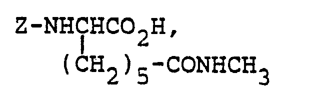 0.57 g (2.2 mmole) of STA-NHCH₂CH(CH₃)CH₂CH₃ and 0.3 g (2.2 mmole) of HOBT in 30 ml DMF was cooled in ice and 0.45 g (2.2 mmole) of DCC added, and the solution allowed to stir at room temperature overnight. The mixture was filtered and the solvent removed under high vacuum. The residue was taken up in EtOAc and washed with 1 N HCI, saturated NaHC0₃, and saturated NaCI. Drying and removal of the solvent under reduced pressure gave 0.69 g of the product as a white foam.

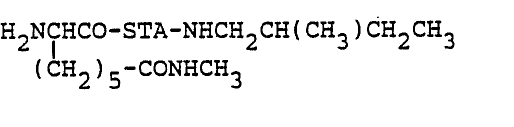

A solution of 0.54 g of 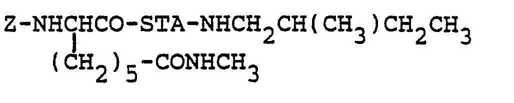 in 250 ml MeOH was treated with 0.5 g of 20% Pd/C and stirred under hydrogen for two hours. The mixture was filtered and the solvent removed under reduced pressure giving 0.43 g of the product as a colorless gum, sufficiently pure for use in the following reaction.

### INTERMEDIATES FOR EXAMPLES 48 AND 49

### BOC-NHC(CH₂CH₂CH₂CH ₌ CH₂)(C0₂Et)₂

To a solution of 14.5 g (0.053 mole) of a-BOC-aminomalonic acid, diethyl ester in 100 ml EtOH was added 25.6 g (0.079 mole) of a 21% solution of NaOEt in EtOH, followed by 9.37 ml (0.079 mole) of 5-bromo-1-pentene. The solution was refluxed under a nitrogen atmosphere for twenty hours, and was then concentrated under reduced pressure. The residue was diluted with Et₂O, washed with H₂O and saturated NaHC0₃, dried, and concentrated under reduced pressure to give 14.7 g of the product as an orange oil. The structure was confirmed by NMR spectroscopy.

### BOC-NHCH(CH₂CH₂CH₂CH = CH₂)C0₂H

To a solution of 12.8 g (0.037 mole) of BOC-NHC(CH₂CH₂CH₂CH=CH₂)(CO₂Et)₂ in 100 ml of THF was added 112 ml (0.112 mole) of 1 N NaOH and 30 ml EtOH. The solution was refluxed for fifteen hours, cooled, and concentrated under reduced pressure. The residue was diluted with EtOAc, washed with H₂0, dried, and concentrated under reduced pressure to an oil. This was dissolved in 200 ml xylene and heated at reflux for two hours. The organic solution was washed with saturated NaHC0₃, acidified to pH 3 with solid citric acid, and extracted with EtOAc. After drying, removal of the solvent under reduced pressure gave 6.85 g of the product as a white solid. The structure was confirmed by NMR spectroscopy.

### BOC-NHCH(CH₂CH₂CH₂CH = CH₂)CO-STA-LEU-NHCH₂Ph

To a solution of 2.46 g (10.1 mmole) of BOC-NHCH(CH₂CH₂CH₂CH=CH₂)C0₂H and 3.82 g (10.1 mmole) of STA-LEU-NHCH₂Ph in 50 ml of CH₃CN was added 1.43 g (10.6 mmole) of HOBT followed by 2.19 g (10.6 mmole) of DCC. The mixture was stirred for two days at room temperature and filtered. The filtrate was concentrated and diluted with EtOAc. The organic layer was washed with saturated NaHC0₃, H₂0, and 10% citric acid solution. Drying and removal of the solvent under reduced pressure gave 5.7 g of the product. The structure was confirmed by NMR spectroscopy.

### H₂NCH(CH₂CH₂CH₂CH = CH₂)CO-STA-LEU-NHCH₂Ph

To a solution of 5.5 g (9.61 mmole) of BOC-NHCH(CH₂CH₂CH₂CH=CH₂)CO-STA-LEU-NHCH₂Ph in 200 ml of CH₂C1₂ was added 200 ml TFA, and the solution stirred at room temperature for one hour. The solvent was removed under reduced pressure and the residue taken up in EtOAc and washed with saturated NaHC0₃. After drying and removal of the solvent under reduced pressure there was obtained 4.9 g of the product. The structure was confirmed by NMR spectroscopy.

### INTERMEDIATES FOR EXAMPLES 52-54

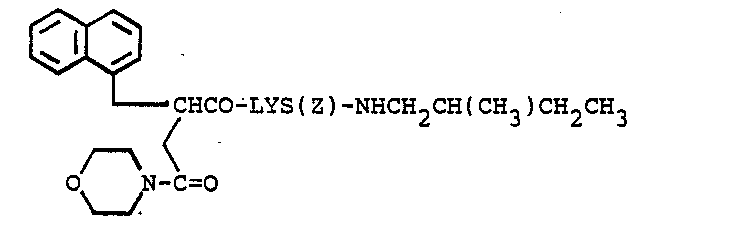

Using the procedure described for preparing intermediates for Example 2, but substituting 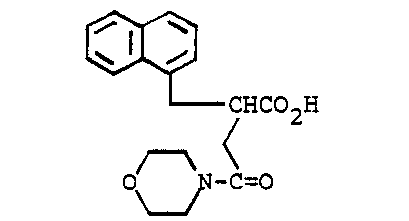 gives the title compound. 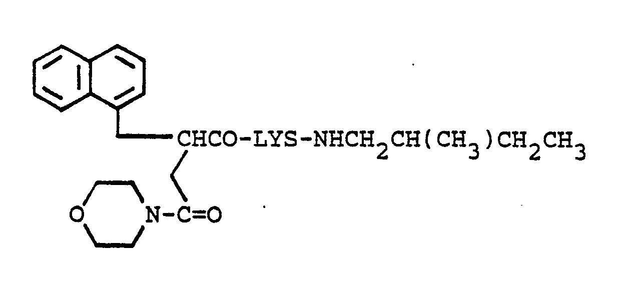 Treatment of 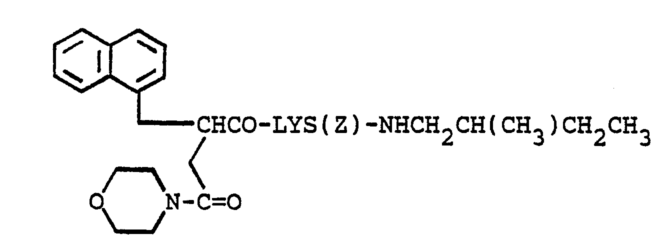 with hydrogen in the presence of Pd/C in MeOH gives the title compound.

### INTERMEDIATES FOR EXAMPLE 55

### BOC-AMINOMALONIC ACID, ISOPROPYL BENZYL ESTER

Following the procedure for intermediates for Example 45 but substituting ISOPROPYL, BENZYL MALONATE for ETHYL BENZYL MALONATE gave 12.2 g of the title compound as an oil. The structure was confirmed by NMR and mass spectroscopy.

### AMINOMALONIC ACID, ISOPROPYL BENZYL ESTER• HCI

Treatment of BOC-AMINOMALONIC ACID, ISOPROPYL BENZYL ESTER with HCI gas in CH₂Cl₂ according to the procedure described for the intermediates for Example 45 gave 9.37 g of the title compound as a white solid. The structure was confirmed by NMR and mass spectroscopy.

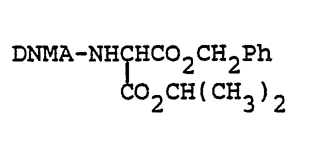

Following the procedure described in the intermediate for Example 45 but substituting AMINOMALONIC ACID, ISOPROPYL BENZYL ESTER . HCI for AMINOMALONIC ACID, ETHYL BENZYL ESTER . HCI gave 5.83 g of the title compound. The structure was confirmed by NMR and mass spectroscopy.

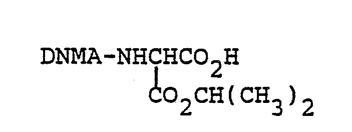

Treatment of 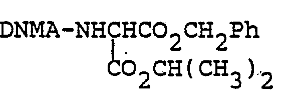 with hydrogen in the presence of 20% Pd/C according to the procedure described in the intermediates for Example 45 gave 4.69 g of the title compound as a white foam. The structure was confirmed by NMR and mass spectroscopy.

### COMMON INTERMEDIATES

### BOC-STA-LEU-NHCH₂Ph

1.80 g LEU-NHCH₂Ph.HCI [Japan 83/59952], 2.27 g BOC-STA (US Pat. 4,397,786) and 1.04 g 1-hydroxybenzotriazole-hydrate were dissolved in 125 ml dichloromethane and cooled to 0°. 1.07 ml of Et₃N was then added. A cold solution of 1.59 g dicyclohexylcarbodiimide in 20 ml dichloromethane was added, followed by the addition of 50 ml cold dimethylformamide. The mixture was stirred at 0° for two hours, followed by 12° overnight. The mixture was then filtered, stripped to a paste, and resuspended in ethyl acetate. The suspension was filtered, the filtrate washed with 1 N citric acid, saturated sodium chloride solution, saturated sodium bicarbonate solution, saturated sodium chloride solution, and dried over anhydrous magnesium sulfate. The suspension was filtered and stripped to a white foam 3.77 g, which was crystallized from ethyl ether and hexane giving a white solid, 3.41 g (92% yield), mp 89-92°. Spectral and elemental analysis confirmed the structure. 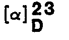 = -34.2° (C, 1.06, MeOH).

### STA-LEU-NHCH₂Ph•HCl

2.77 g BOC-STA-LEU-NHCH₂Ph was dissolved in 100 ml dichloromethane, which was then saturated with anhydrous hydrogen chloride gas. After stirring at 25° for one hour, the solvent was removed in vacuo, and the residue resuspended in dichloromethane, giving a crystalline solid. The suspension was diluted with ethyl ether, filtered, and the solid dried in vacuo, 2.24 g, 93% yield. Spectral and elemental analyses confirmed the structure. 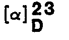 = -19.1 °(C, 1.06, MeOH).

### BOC-STA-NHCH₂CH(CH₃)CH₂CH₃

BOC-STA (27.53 g, 0.1 mole, US Patent 4,397,786) and HOBT*H₂0 (14.2 g, 0.105 mole) were dissolved in 40 ml DMF. 300 ml CH₂Cl₂ was added, and the mixture was cooled to 0°. A solution of DCC (21.66 g, 0.105 mole) in 50 ml CH₂Cl₂ was added, followed by S-2-methyibutylamine (12 ml, 0.1 mole). After stirring at 0° for two hours, the mixture was allowed to warm to 25° over 1.5 hours. The mixture was filtered, and the solvent was removed in vacuo. The residue was dissolved in EtOAc, which was washed with 1 N citric acid, brine, saturated NaHCO₃ solution, and brine. The organic phase was dried over MgS0₄, filtered, and stripped to a gum, 36.90 g. The gum was dissolved in Et₂0 and treated with charcoal to remove colored impurities. The suspension was filtered and stripped to a gum, 35.2 g, which was suitable for use in the following procedure without further purification.

### STA-NHCH₂CH(CH₃)CH₂CH₃•HCl

BOC-STA-NHCH₂CH(CH₃)CH₂CH₃ (34.4 g, 0.1 mole) was dissolved in 250 ml CH₂CI₂, and the solution was purged occasionally with anhydrous HCI gas over three hours. A solid precipitated from solution which was filtered, washed with CH₂CI₂, and dried at 40° in vacuo to a hygroscopic solid, 21 g. The solid was triturated with a mixture of CH₂Cl₂/Et₂O, filtered, and dried at 40° in vacuo to a white solid, 19.34 g. Spectral analysis confirmed the structure.

### STA-NHCH₂CH(CH₃)CH₂CH₃

A solution of 38.0 g (0.11 mole) of BOC-STA-NHCH₂CH(CH₃)CH₂CH₃ in 250 ml CH₂CI₂ was treated with HCI gas every one-half hour over a three hour period. The solvent was removed under reduced pressure and the residue taken up in 30 ml H₂O and 110 ml of 1 N HCI. The solution was washed twice with Et₂0, the pH brought to 13 with 2 N NaOH, and the solution extracted twice with Et₂0. The Et₂0 was washed with saturated NaCl, dried, and the solvent removed under reduced pressure giving 22.3 g of the product as an oil which solidified on standing. The material was sufficiently pure for use in subsequent reactions.

## Claims

1. A peptide of the formula 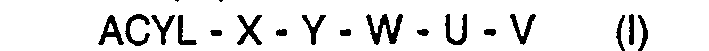 or a pharmaceutically acceptable acid addition salt thereof, wherein ACYL is BOC, IVA, NVA, DNMA, Z, or 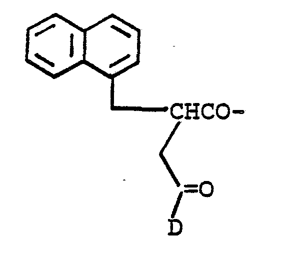 wherein D is 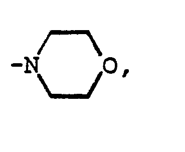 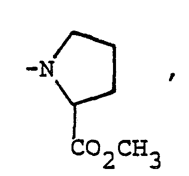 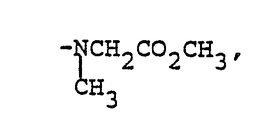 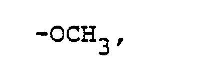 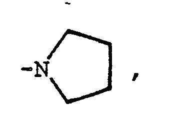 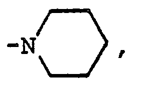 or 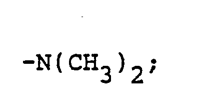 X is absent, PHE, HOMOPHE, NAPHTHYLALA, CYCLOHEXYLALA, TYR, or TRP, with the proviso that when ACYL is DNMA or 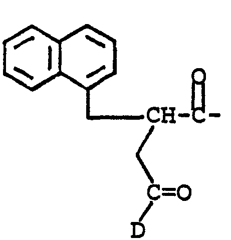 wherein D is as above, X is absent; Y is 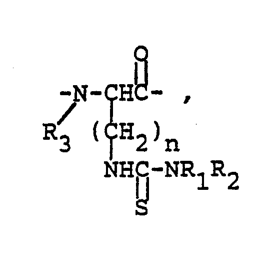 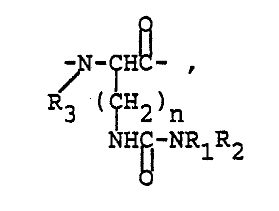 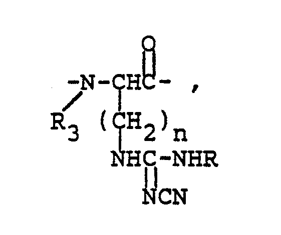 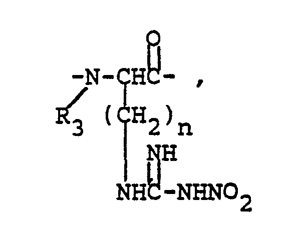 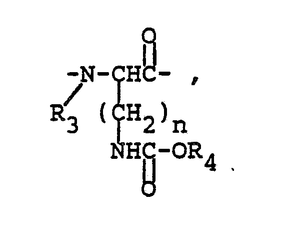 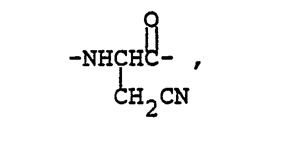 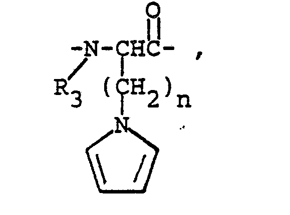 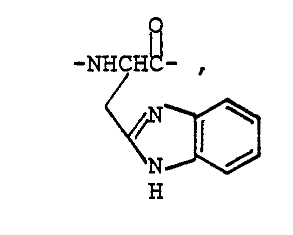 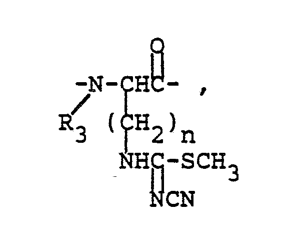 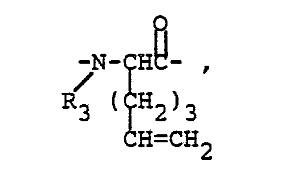 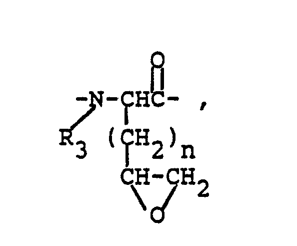 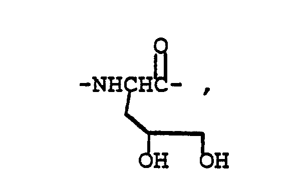 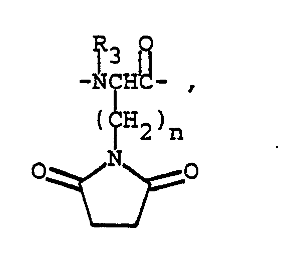 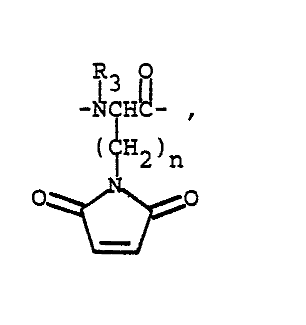 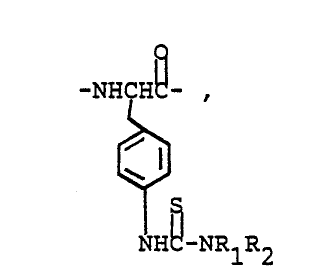 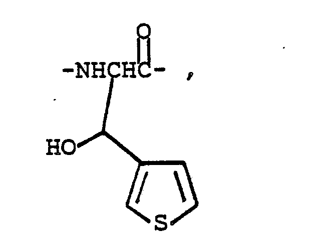 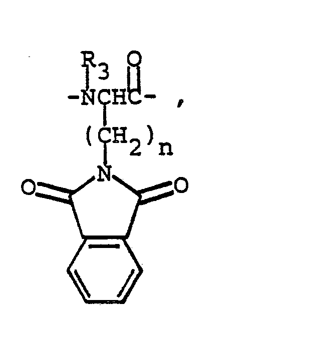 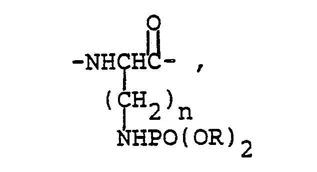 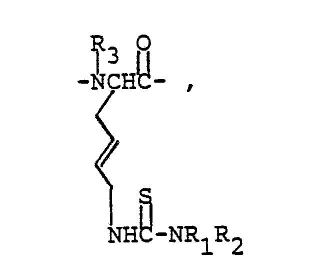 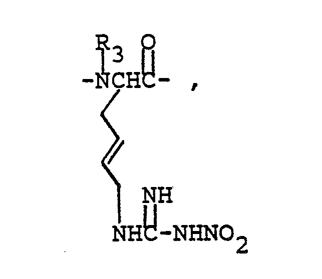 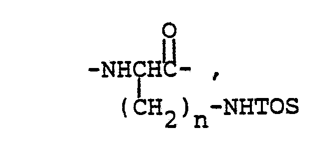 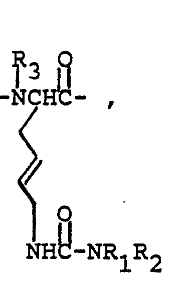 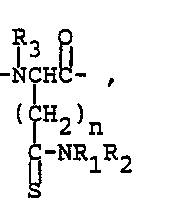 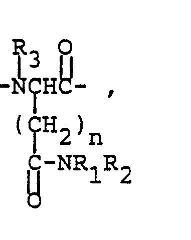 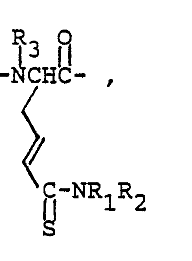 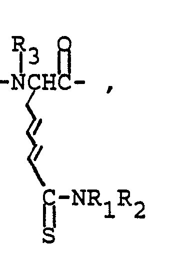 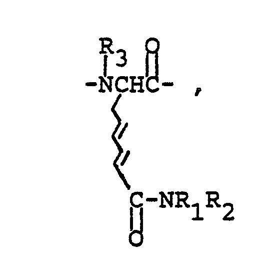 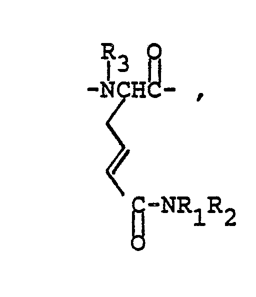 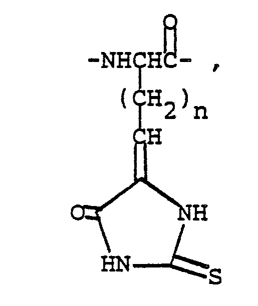 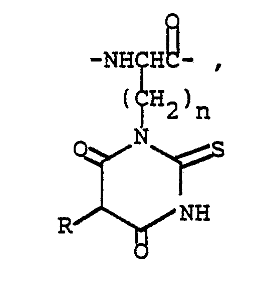 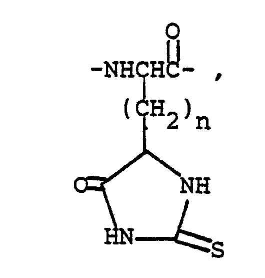 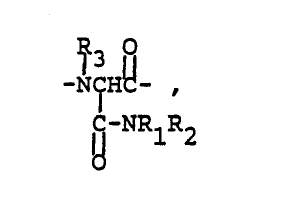 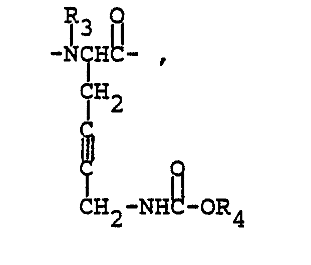 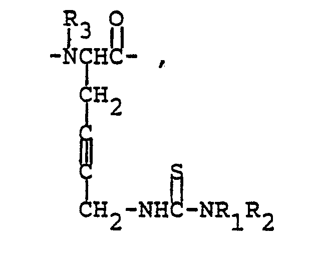 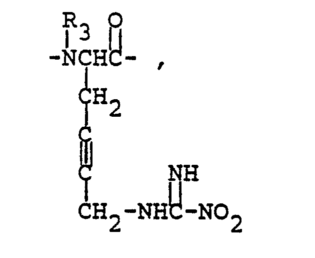 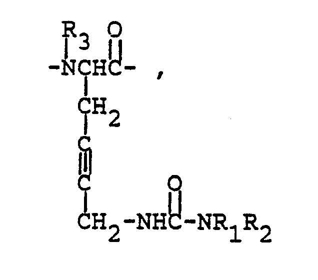 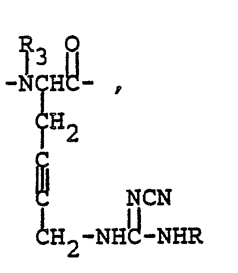 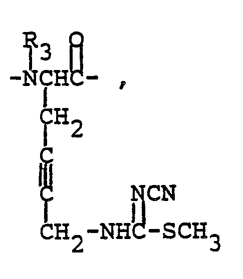 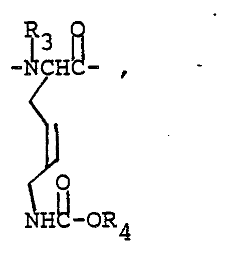 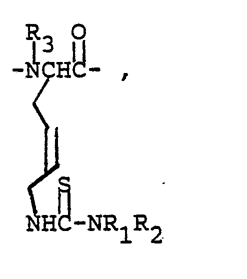 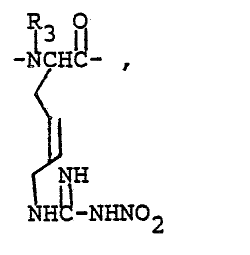 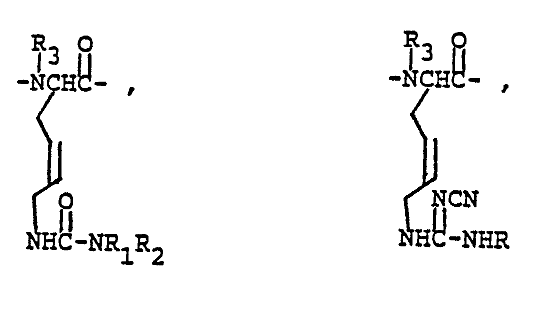 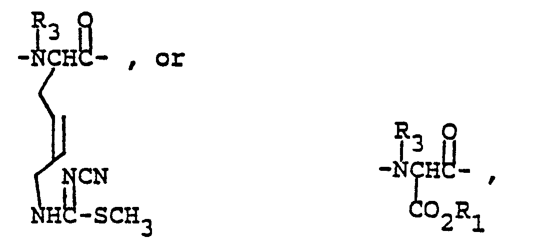 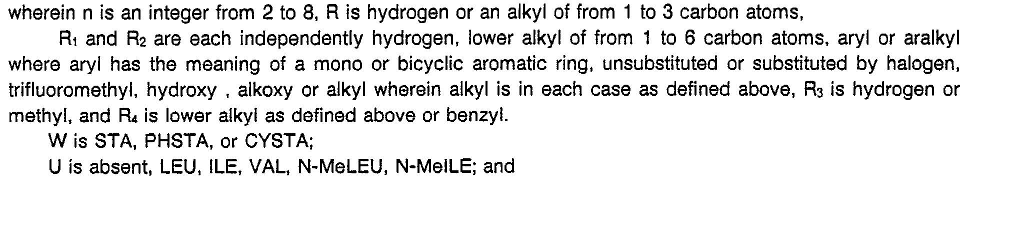 V is

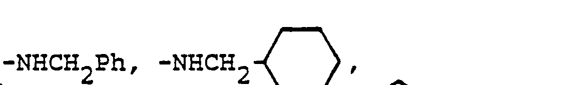

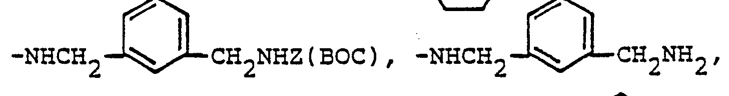

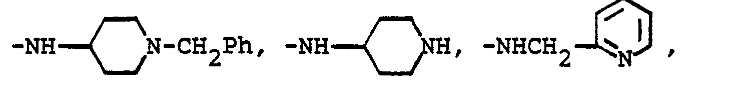

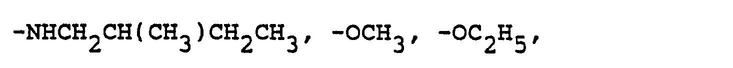

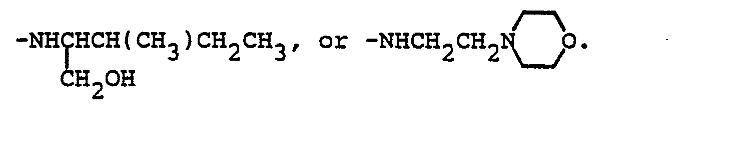

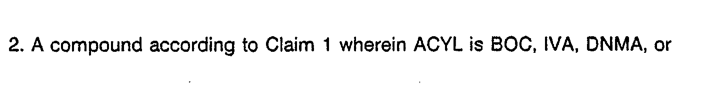

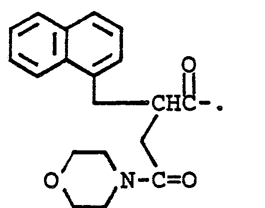

3. A compound according to Claim 1 wherein V is 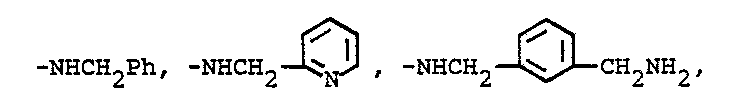 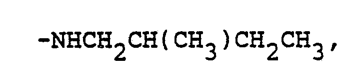 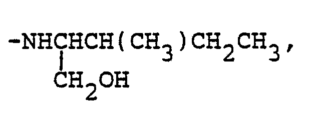 or

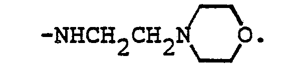

4. A peptide according to Claim 1 wherein the peptide is a member selected from the group consisting of: 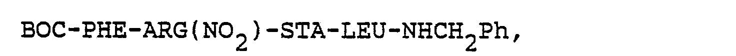 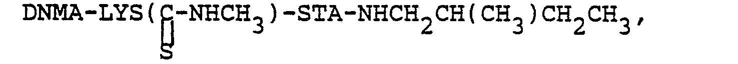 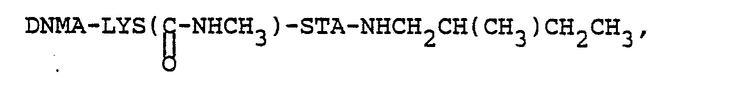 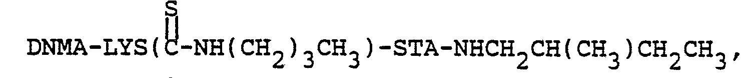 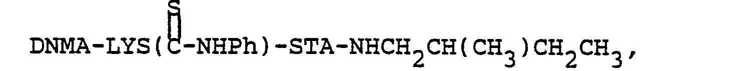 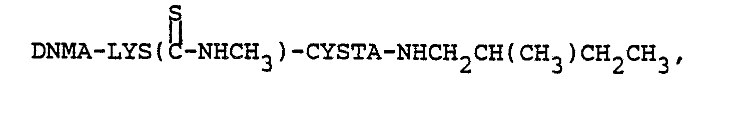 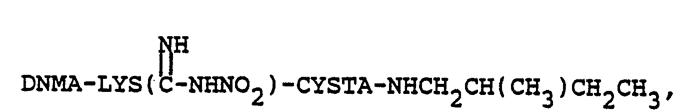 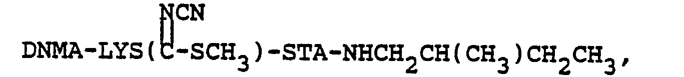 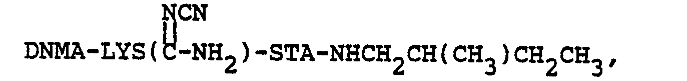 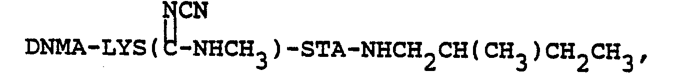 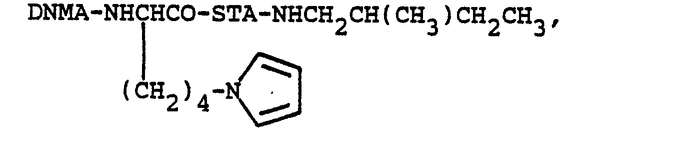 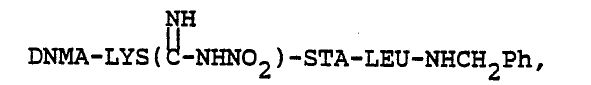 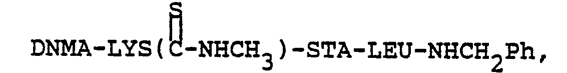 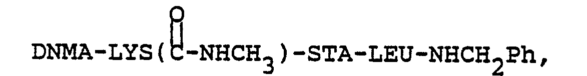 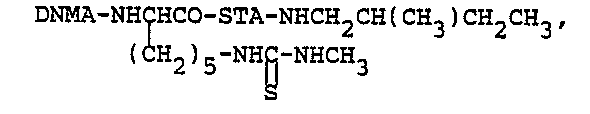 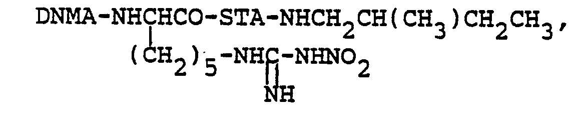 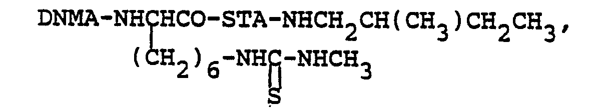 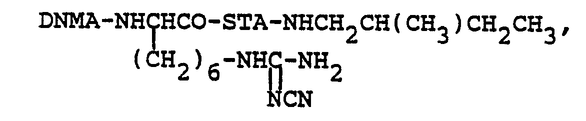 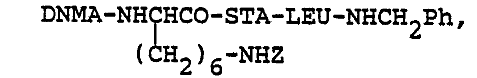 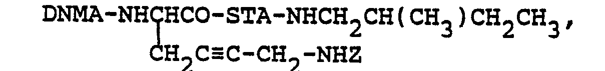 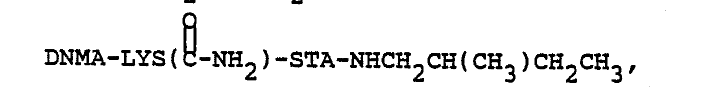 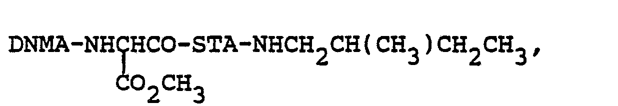 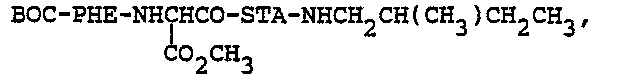 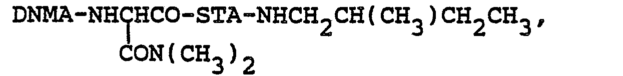 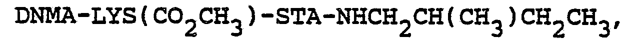 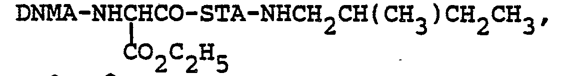 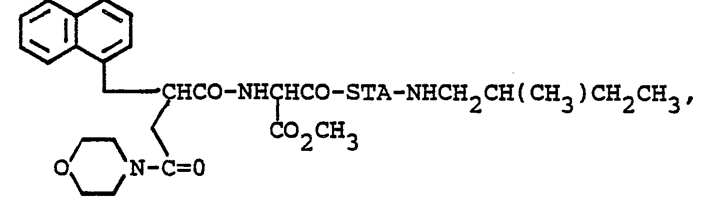 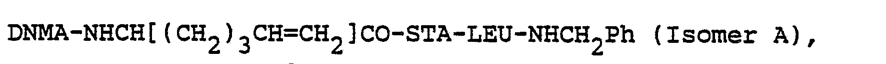 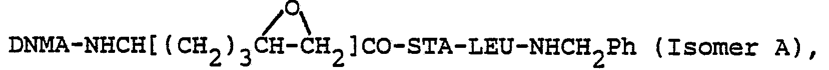 or

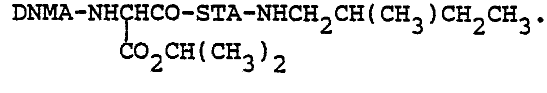

5. A process for the preparation of compounds according to Claim 1 which comprises:
(a) reacting BOC-protected statine with a primary amine to form the corresponding BOC-protected compound,
(b) reacting the BOC-protected compound with a strong acid removing the protecting group and forming the corresponding compound with a free amino terminus,
(c) reacting this compound with BOC-protected LYS(Z) to form the corresponding protected compound,
(d) reacting this protected compound with a strong acid to form the corresponding compound with a free amino terminus,
(e) reacting this compound with di-(1-naphthylmethyl)acetic acid forming a DNMA-terminated peptide,
(f) removing the (Z) of step (c) above by catalytic hydrogenation, and
(g) reacting the resulting peptide with methyl isocyanate or methyl thioisocyanate forming a desired compound.

6. A pharmaceutical composition comprising a renin-inhibitory or a hyperaldosteronism-inhibitory effective amount or an amount effective for treating congestive heart failure of a compound as claimed in Claim 1 together with a pharmaceutically acceptable carrier.

7. A method of use of compound according to Claim 1 for the preparation of a pharmaceutical for treating renin-associated hypertension or hyperaldosteronism or congestive heart failure.

8. Use of a peptide according to Claim 1 for the manufacture of a diagnostic for determining the presence of renin-associated hypertension in a patient by administering said peptide to said patient, at a hypotensive dosage level and as a single dose followed by monitoring of said patient's blood pressure.

1. A process for the preparation of peptides of the formula ACYL-X-Y-W-U-V (I) or a pharmaceutically acceptable acid addition salt thereof, wherein . ACYL is BOC, IVA, NVA, DNMA, Z, or 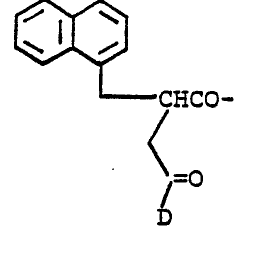 wherein D is 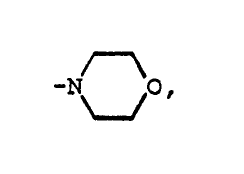 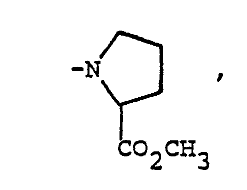 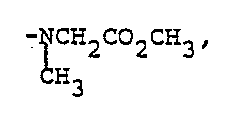 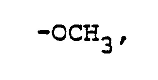 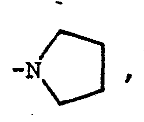 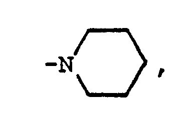 or 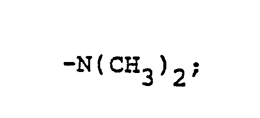 X is absent, PHE, HOMOPHE, NAPHTHYLALA, CYCLOHEXYLALA, TYR, or TRP, with the proviso that when ACYL is DNMA or 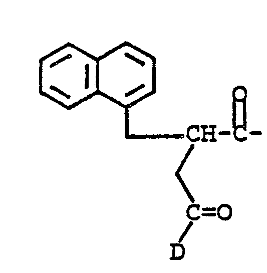 wherein D is as above, X is absent; Y is 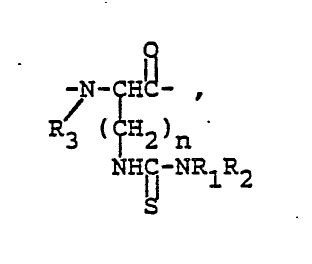 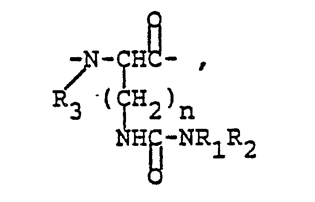 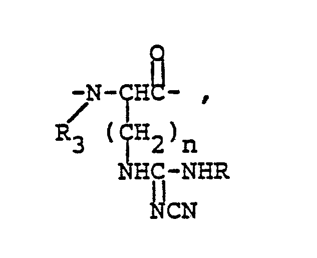 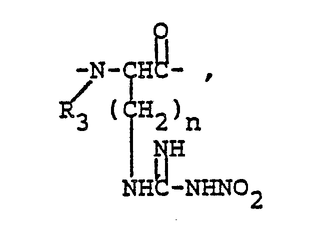 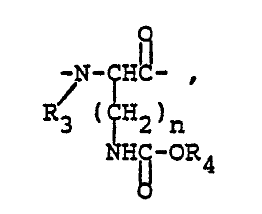 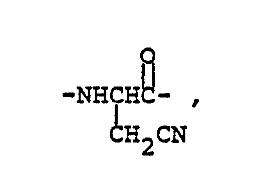 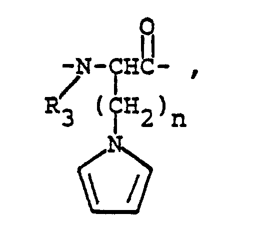 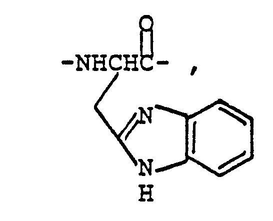 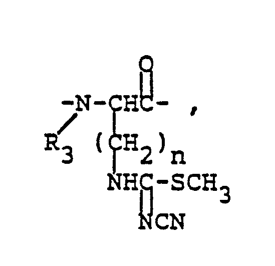 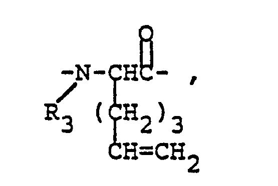 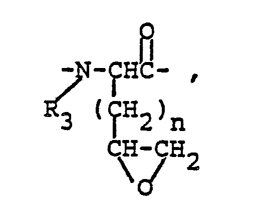 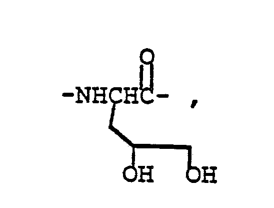 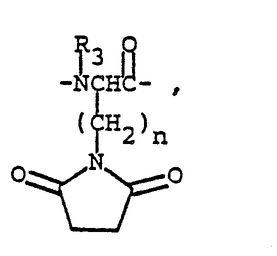 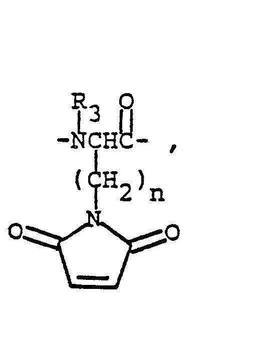 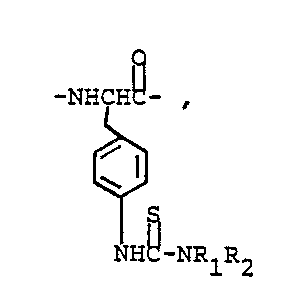 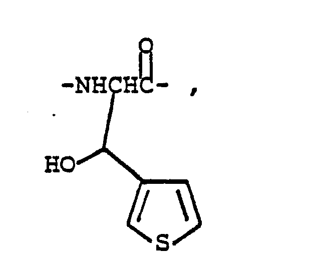 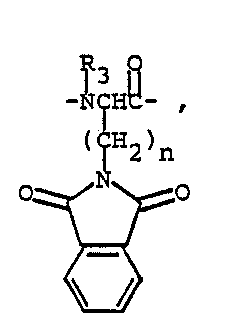 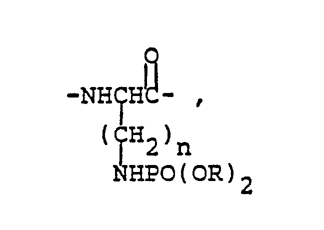 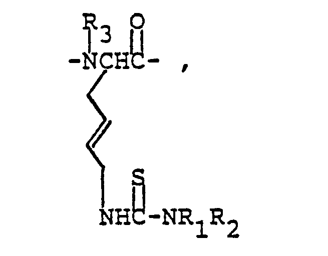 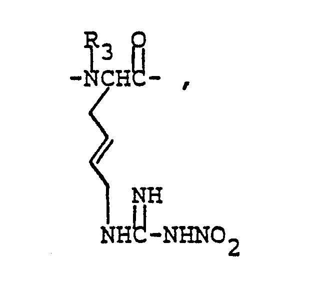 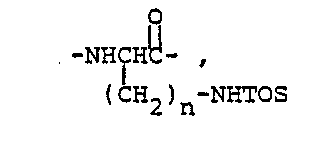 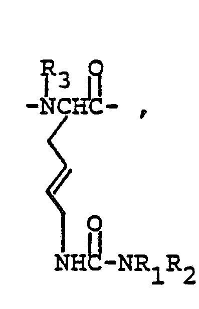 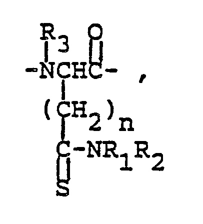 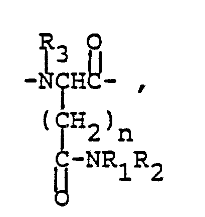 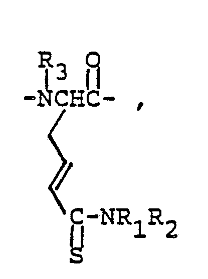 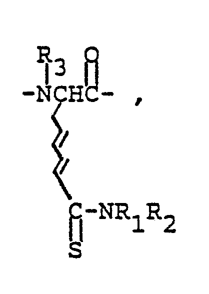 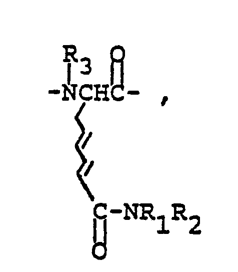 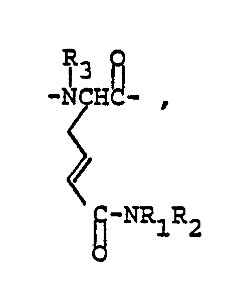 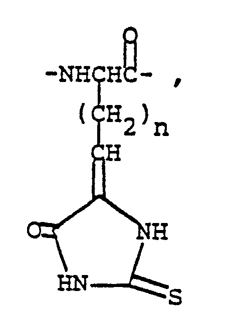 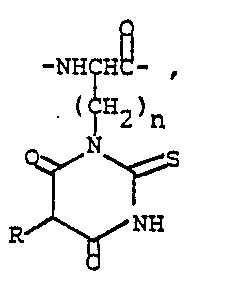 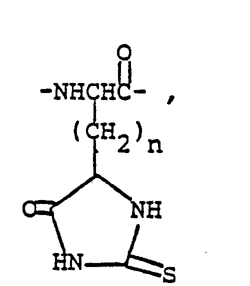 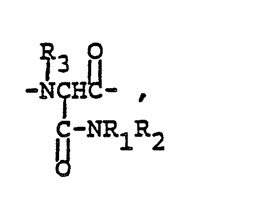 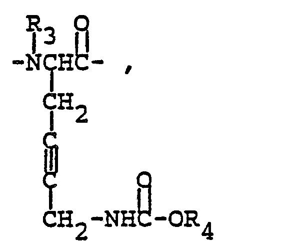 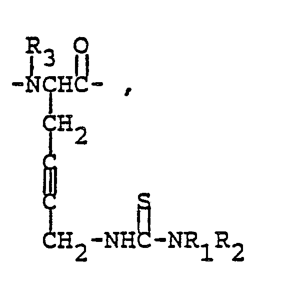 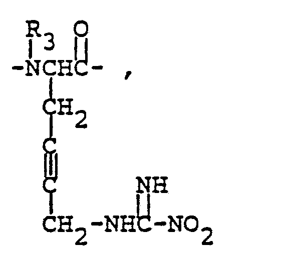 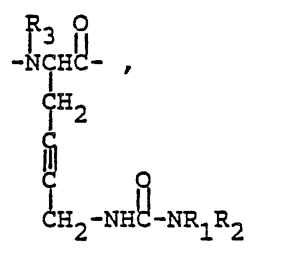 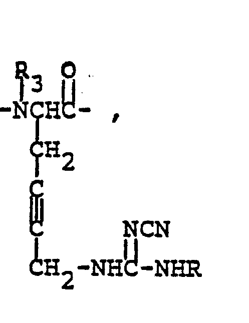 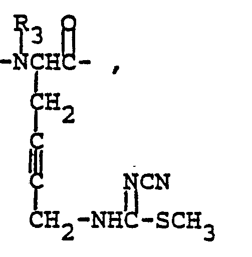 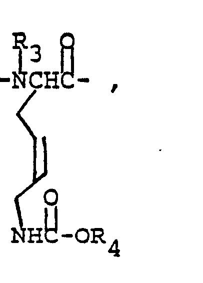 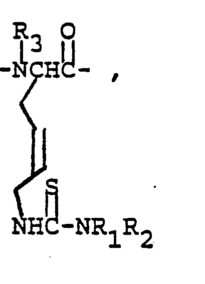 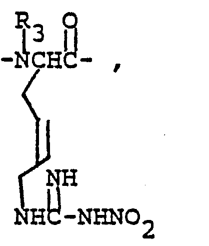 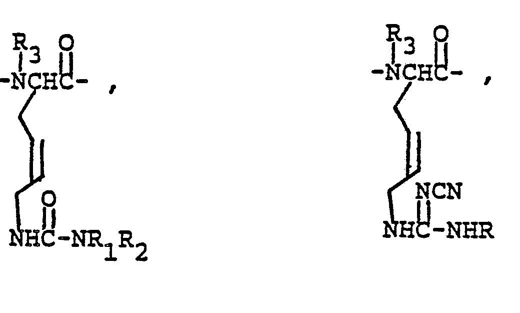 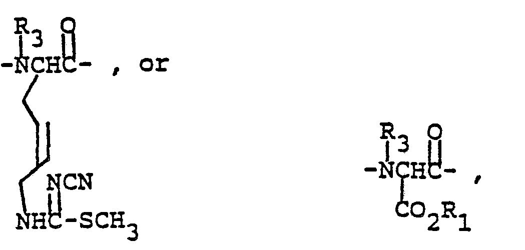 wherein n is an integer from 2 to 8, R is hydrogen or an alkyl of from 1 to 3 carbon atoms,
R₁ and R₂ are each independently hydrogen, lower alkyl of from 1 to 6 carbon atoms, aryl or aralky where aryl has the meaning of a mono or bicyclic aromatic ring, unsubstituted or substituted by halogen trifluoromethyl, hydroxy , alkoxy or alkyl wherein alkyl is in each case as defined above, R₃ is hydrogen o methyl, and R₄ is lower alkyl as defined above or benzyl.
W is STA, PHSTA, or CYSTA;
U is absent, LEU, ILE, VAL, N-MeLEU, N-MelLE; and V is 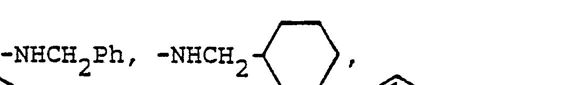 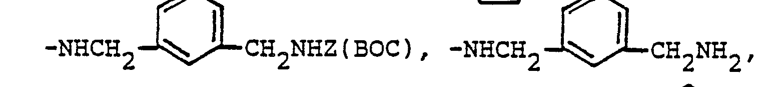 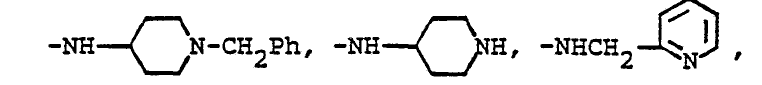 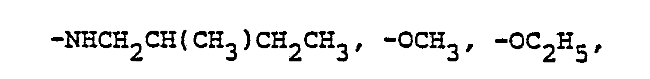 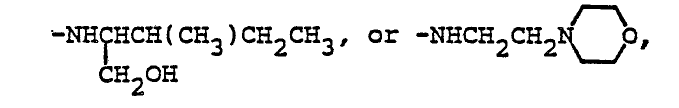 which comprises:
(a) reacting BOC-protected statine with a primary amine to form the corresponding BOC-protected compound,
(b) reacting the BOC-protected compound with a strong acid removing the protecting group and forming the corresponding compound with a free amino terminus,
(c) reacting this compound with BOC-protected LYS(Z) to form the corresponding protected compound,
(d) reacting this protected compound with a strong acid to form the corresponding compound with a free amino terminus,
(e) reacting this compound with di-(1-naphthylmethyl)acetic acid forming a DNMA-terminated peptide,
. (f) removing the (Z) of step (c) above by catalytic hydrogenation, and
(g) reacting the resulting peptide with methyl isocyanate or methyl thioisocyanate forming a desired compound.

2. A process according to Claim 1 for a compound wherein ACYL is BOC, IVA, DNMA, or

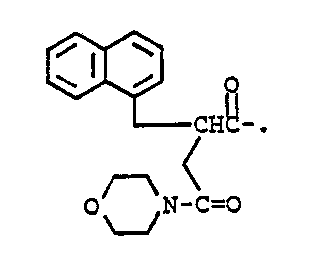

3. A process according to Claim 1 for the preparation of a compound wherein V is 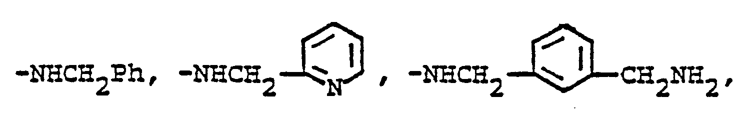 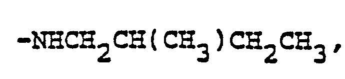 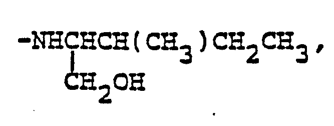 or

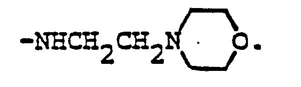

4. A process according to Claim 1 for the preparation of a compound selected from the group consisting of: 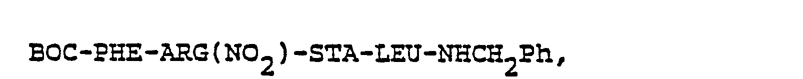 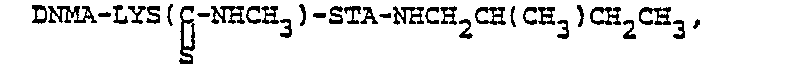 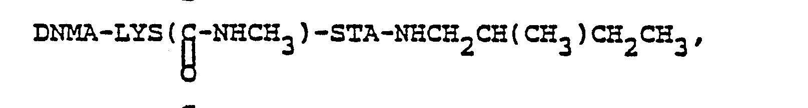 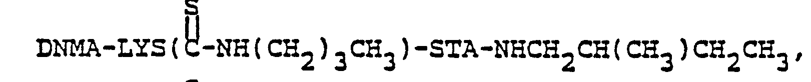 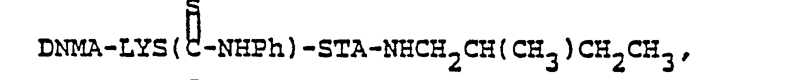 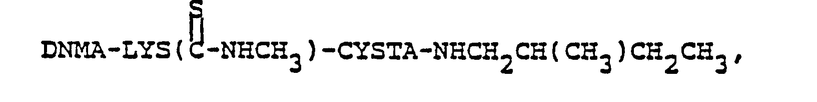 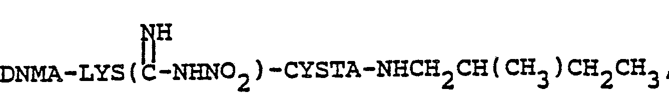 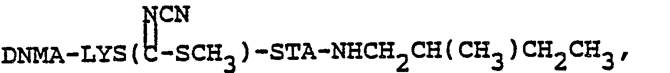 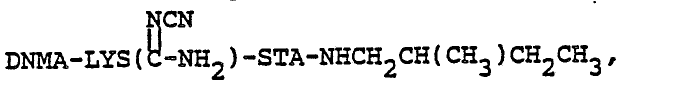 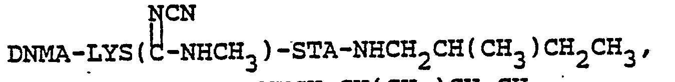 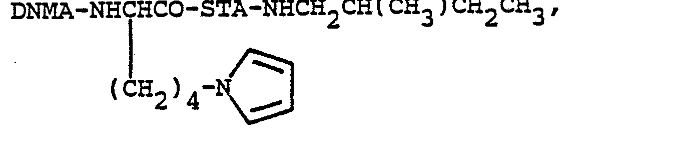 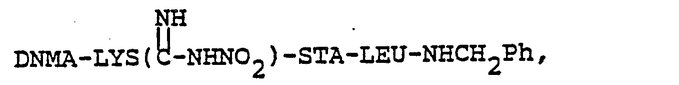 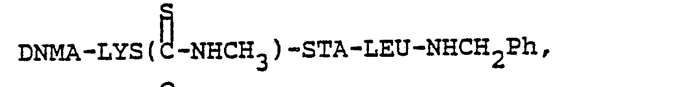 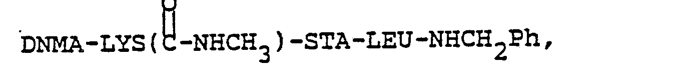 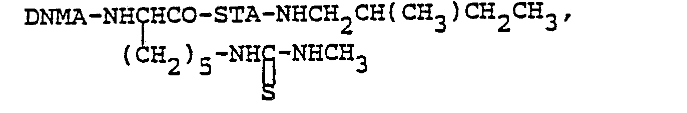 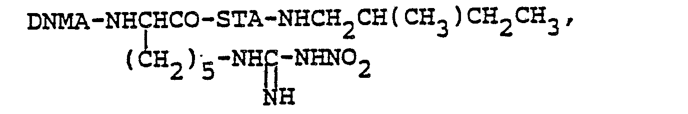 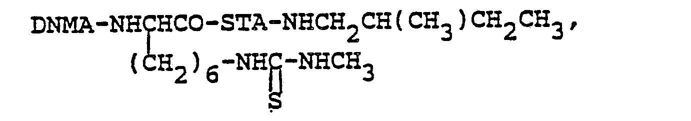 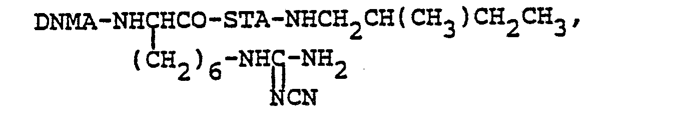 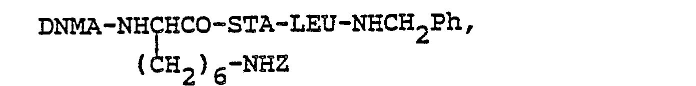 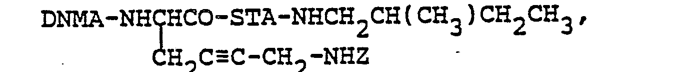 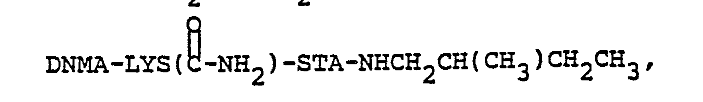 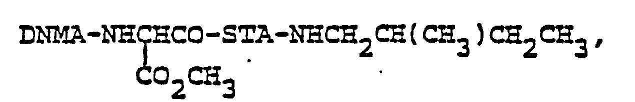 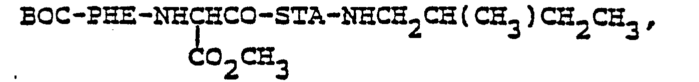 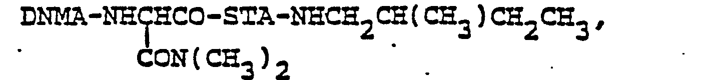 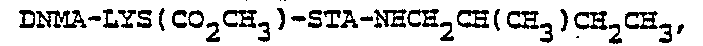 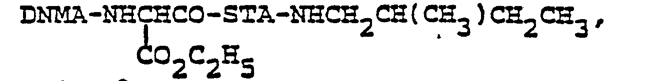 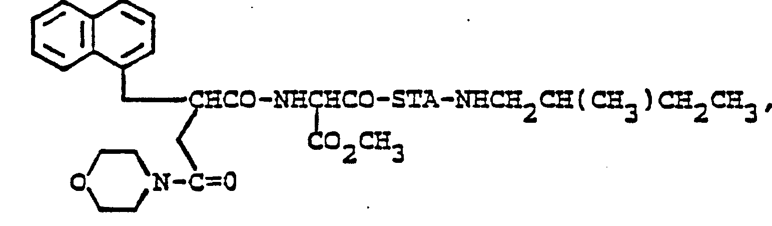 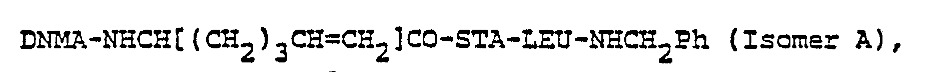 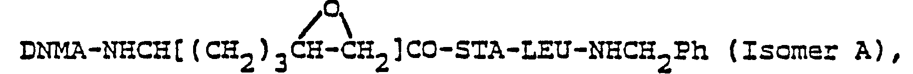 or

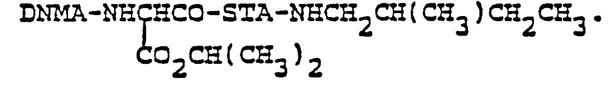

5. A method of use of a compound according to claim 1 for the preparation of a pharmaceutical for treating renin-associated hypertension or hyperaldosteronism or congestive heart failure.

6. Use of a peptide according to Claim 1 for the manufacture of a diagnostic for determining the presence of renin-associated hypertension in a patient by administering said peptide to said patient, at a hypotensive dosage level and as a single dose followed by monitoring of said patient's blood pressure.
